# EUROPEAN PATENT APPLICATION

(11) **EP 4 727 319 A1**
(43) Date of publication of application: **15.04.2026**
(21) Application number: 25200152.4
(22) Date of filing: 04.09.2025
(51) Int. Cl.: H10K 85/60, H10K 50/15, H10K 50/17

(54) **ORGANIC COMPOUND, LIGHT-EMITTING DEVICE COMPRISING THE ORGANIC COMPOUND, ELECTRONIC APPARATUS COMPRISING THE LIGHT-EMITTING DEVICE, AND ELECTRONIC EQUIPMENT COMPRISING THE ELECTRONIC APPARATUS**

(30) Priority: 25.09.2024 KR 20240130208
(71) Applicant: Samsung Display Co., Ltd., Yongin-si, Gyeonggi-do 17113 (KR)
(72) Inventor: Lee, Hyungwoo, 17113 Yongin-si (KR); Kim, Gyeongheon, 17113 Yongin-si (KR); Kim, Hoilim, 17113 Yongin-si (KR); Yu, Hayoung, 17113 Yongin-si (KR); Lee, Junyoung, 17113 Yongin-si (KR)
(74) Representative: Crow, Martin

(57) **Abstract**

A light-emitting device comprises a first electrode (110), a second electrode (150) opposite to the first electrode, and an interlayer between the first electrode and the second electrode and comprising an emission layer, wherein the interlayer comprises an organic compound comprising a first moiety represented by Formula 1 and a second moiety represented by Formula 2: 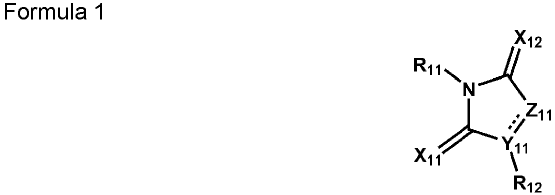 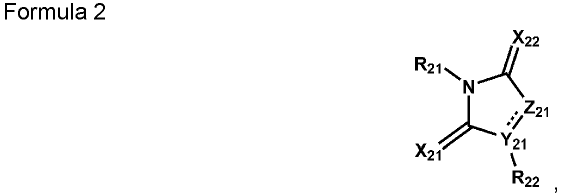 wherein, in Formulae 1 and 2,
a moiety represented by 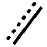 is a single bond or a double bond,
X₁₁, X₁₂, X₂₁, and X₂₂ are each independently O or S,
Y₁₁ is C, C(R₁₃), or N,
Z₁₁ is C(R₁₄), N, N(R₁₄), O, or S,
Y₂₁ is C, C(R₂₃), or N,
Z₂₁ is C(R₂₄), N, N(R₂₄), O, or S,
R₁₁ or R₁₂ is a linking site to the second moiety, and
R₂₁ or R₂₂ is a linking site to the first moiety.

## Description

### CROSS-REFERENCE TO RELATED APPLICATION

The present application claims priority to and the benefit of Korean Patent Application No. 10-2024-0130208, filed on September 25, 2024, in the Korean Intellectual Property Office, the entire disclosure of which is incorporated herein by reference.

### BACKGROUND

### 1. Field

One or more embodiments of the present disclosure relate to an organic compound, a light-emitting device comprising the organic compound, an electronic apparatus comprising the light-emitting device, and electronic equipment comprising the electronic apparatus.

### 2. Description of the Related Art

Self-emissive devices (for example, organic light-emitting devices, and/or the like) among light-emitting devices have relatively wide viewing angles, high contrast ratios, short response times, and excellent or suitable characteristics in terms of luminance, driving voltage, and response speed, compared to other light-emitting devices that are not self-emissive.

A light-emitting device may comprise a first electrode, a hole transport region, an emission layer, an electron transport region, and a second electrode that are sequentially arranged. Holes injected from the first electrode may move toward the emission layer through the hole transport region. Electrons injected from the second electrode may move toward the emission layer through the electron transport region. These carriers, namely the holes and electrons, recombine in the emission layer to produce excitons. As the excitons transition and decay from an excited state to a ground state, light may be generated.

### SUMMARY

One or more aspects of embodiments of the present disclosure are directed toward an organic compound having excellent or suitable highest occupied molecular orbital (HOMO) energy level, lowest unoccupied molecular orbital (LUMO) energy level, hole mobility, electron mobility, and glass transition temperature, a light-emitting device having excellent or suitable driving voltage, current efficiency, and lifespan by including the organic compound, an electronic apparatus having excellent or suitable display quality by including the light-emitting device, and electronic equipment having high quality by including the electronic apparatus. For example, the disclosure includes a light-emitting device with excellent or suitable driving voltage, current efficiency, and lifespan by including the organic compound, an electronic apparatus with excellent or suitable display quality by including the light-emitting device, and electronic equipment with high quality by including the electronic apparatus.

Additional aspects will be set forth in part in the description which follows and, in part, will be apparent from the description, or may be learned by practice of the presented embodiments of the disclosure.

According to one or more embodiments of the present disclosure, a light-emitting device comprises a first electrode, a second electrode opposite to (e.g., facing) the first electrode, and an interlayer between (e.g., arranged between) the first electrode and the second electrode and comprising an emission layer, wherein the interlayer may comprise an organic compound comprising a first moiety represented by Formula 1 and a second moiety represented by Formula 2: 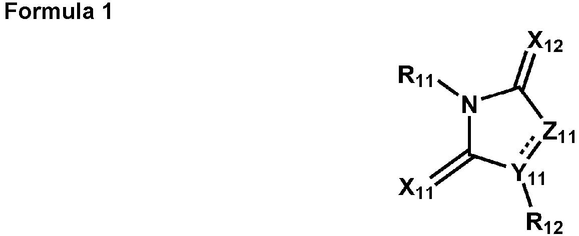 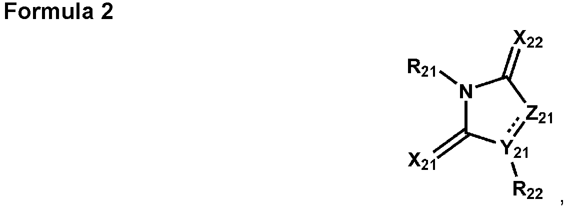
wherein, in Formulae 1 and 2,
a moiety represented by 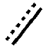 may be a single bond or a double bond,
X₁₁, X₁₂, X₂₁, and X₂₂ may each independently be O or S,
Y₁₁ may be C, C(R₁₃), or N,
Z₁₁ may be C(R₁₄), N, N(R₁₄), O, or S,
Y₂₁ may be C, C(R₂₃), or N,
Z₂₁ may be C(R₂₄), N, N(R₂₄), O, or S,
R₁₁ to R₁₄ and R₂₁ to R₂₄ may each independently be hydrogen, deuterium, -F, -Cl, - Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂),
R₁₁ or R₁₂ may be a linking site to the second moiety,
R₂₁ or R₂₂ may be a linking site to the first moiety,
R₁₀ₐ may be:
   deuterium, -F, -Cl, -Br, -I, a hydroxyl group, a cyano group, or a nitro group;
   a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), -C(=O)(Q₁₁), -S(=O)2(Q11), - P(=O)(Q₁₁)(Q₁₂), or any combination thereof;
   a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, - Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂), or any combination thereof; or
   -Si(Q₃₁)(Q₃₂)(Q₃₃), -N(Q₃₁)(Q₃₂), -B(Q₃₁)(Q₃₂), -C(=O)(Q₃₁), -S(=O)2(Q31), or - P(=O)(Q₃₁)(Q₃₂), and
   Q₁ to Q₃, Q₁₁ to Q₁₃, Q₂₁ to Q₂₃, and Q₃₁ to Q₃₃ may each independently be:
   hydrogen, deuterium, -F, -Cl, -Br, -I, a hydroxyl group, a cyano group, or a nitro group; or
   a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

According to one or more embodiments of the present disclosure, an electronic apparatus comprises the light-emitting device and a thin-film transistor electrically connected to the light-emitting device.

According to one or more embodiments of the present disclosure, electronic equipment comprises the electronic apparatus, wherein the electronic equipment may be at least one selected from among a flat panel display, a curved display, a computer monitor, a medical monitor, a television, a billboard, an indoor light, an outdoor light, a signal light, a head-up display, a fully transparent display, a partially transparent display, a flexible display, a rollable display, a foldable display, a stretchable display, a laser printer, a telephone, a mobile phone, a tablet, a phablet, a personal digital assistant (PDA), a wearable device, a laptop computer, a digital camera, a camcorder, a viewfinder, a micro display, a 3-dimension (3D) display, a virtual reality display, an augmented reality display, a vehicle, a video wall comprising multiple displays tiled together, a theater screen, a stadium screen, a phototherapy device, and a signboard.

According to one or more embodiments of the present disclosure, provided is the organic compound comprising the first moiety represented by Formula 1 and the second moiety represented by Formula 2.

### BRIEF DESCRIPTION OF THE DRAWINGS

The accompanying drawings are included to provide a further understanding of the present disclosure and are incorporated in and constitute a part of the present disclosure. The drawings illustrate embodiments of the present disclosure and, together with the description, serve to explain principles of the present disclosure. The above and other aspects, features, and advantages of certain embodiments of the disclosure will be more apparent from the following description taken in conjunction with the accompanying drawings, in which:
FIG. 1 is a schematic view of a light-emitting device according to one or more embodiments of the present disclosure;
FIG. 2 is a schematic view of an electronic apparatus according to one or more embodiments of the present disclosure;
FIG. 3 is a schematic view of an electronic apparatus according to one or more embodiments of the present disclosure;
FIG. 4 is a schematic perspective view of electronic equipment comprising a light-emitting device according to one or more embodiments of the present disclosure;
FIG. 5 is a schematic view of an exterior of a vehicle as electronic equipment comprising a light-emitting device according to one or more embodiments of the present disclosure; and
FIGS. 6A - 6C are each a schematic view of an interior of the vehicle of FIG. 5 according to one or more embodiments of the present disclosure.

### DETAILED DESCRIPTION

Reference will now be made in more detail to one or more embodiments, examples of which are illustrated in the accompanying drawings, wherein like reference numerals refer to like elements throughout the disclosure, and duplicative descriptions thereof may not be provided for conciseness. In this regard, the presented embodiments may have different forms and should not be construed as being limited to the descriptions set forth herein. Accordingly, embodiments of the present disclosure are merely described, by referring to the drawings, to explain aspects of the present disclosure. As used herein, the term "and/or" or "or" may include any and all combinations of one or more of the associated listed items. Throughout the disclosure, the expressions such as "at least one of," "one of," and "selected from," when preceding a list of elements, modify the entire list of elements and do not modify the individual elements of the list. For example, "at least one of a, b, or c", "at least one selected from among a, b, and c", "at least one selected from among a to c", and/or the like, may indicates only a, only b, only c, both (e.g., simultaneously) a and b, both (e.g., simultaneously) a and c, both (e.g., simultaneously) b and c, all of a, b, and c, or variations thereof.

In the present disclosure, the expression "bonded to" indicates that two atoms are directly bonded to each other via a covalent bond, a coordinate bond, and/or the like, without any other atoms between the two atoms.

The expression "linked to" indicates not only that two atoms are "bonded" to each other, but also that single or multiple other atoms may be present between the two atoms. For example, if (e.g., when) a first atom is bonded to a second atom and the second atom is bonded to a third atom, the first atom is linked to the third atom.

For example, if (e.g., when) atoms A₁ to A₃ are in a relationship of "A₁-A₂-A₃," atom A₂ is bonded to atom A₁ and is linked to atom A₁, atom A₂ is bonded to atom A₃ and is linked to atom A₃, and atom A₁ is not bonded to atom A₃ but is linked to atom A₃.

According to one or more embodiments of the present disclosure, a light-emitting device may comprise: a first electrode; a second electrode opposite to (e.g., facing) the first electrode; and an interlayer between (e.g., arranged between) the first electrode and the second electrode and comprising an emission layer, wherein the interlayer may comprise an organic compound comprising a first moiety represented by Formula 1 and a second moiety represented by Formula 2: 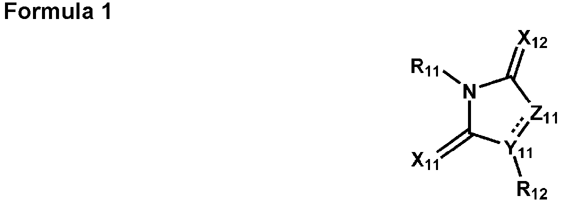 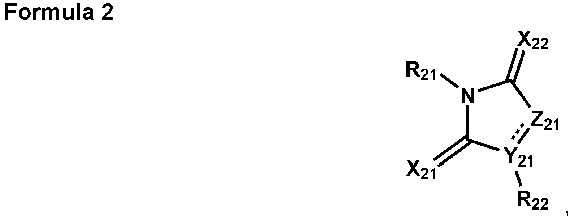
wherein, in Formulae 1 and 2,
a moiety represented by 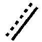 may be a single bond or a double bond, in other words, 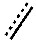 may represent a single bond or a double bond,
X₁₁, X₁₂, X₂₁, and X₂₂ may each independently be O or S,
Y₁₁ may be C, C(R₁₃), or N,
Z₁₁ may be C(R₁₄), N, N(R₁₄), O, or S,
Y₂₁ may be C, C(R₂₃), or N,
Z₂₁ may be C(R₂₄), N, N(R₂₄), O, or S,
R₁₁ to R₁₄ and R₂₁ to R₂₄ may each independently be hydrogen, deuterium, -F, -Cl, - Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂),
R₁₁ or R₁₂ may be a linking site to the second moiety,
R₂₁ or R₂₂ may be a linking site to the first moiety,
R₁₀ₐ may be:
   deuterium, -F, -Cl, -Br, -I, a hydroxyl group, a cyano group, or a nitro group;
   a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), - P(=O)(Q₁₁)(Q₁₂), or any combination thereof;
   a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, - Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂), or any combination thereof; or
   -Si(Q₃₁)(Q₃₂)(Q₃₃), -N(Q₃₁)(Q₃₂), -B(Q₃₁)(Q₃₂), -C(=O)(Q₃₁), -S(=O)2(Q31), or - P(=O)(Q₃₁)(Q₃₂), and
   Q₁ to Q₃, Q₁₁ to Q₁₃, Q₂₁ to Q₂₃, and Q₃₁ to Q₃₃ may each independently be:
   hydrogen, deuterium, -F, -Cl, -Br, -I, a hydroxyl group, a cyano group, or a nitro group; or
   a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

The term "interlayer" as used herein refers to a single layer and/or all of multiple layers arranged between the first electrode and the second electrode of the light-emitting device.

In one or more embodiments, the interlayer may further comprise a hole transport region between (e.g., arranged between) the first electrode and the emission layer. The hole transport region may comprise a hole injection layer, a hole transport layer, an emission auxiliary layer, an electron-blocking layer, or any combination thereof. For example, in one or more embodiments, the hole transport region may comprise a hole injection layer and a hole transport layer that are sequentially stacked from the first electrode in the stated order.

In one or more embodiments, the hole transport region may comprise at least one of the organic compound. For example, in one or more embodiments, the hole injection layer that is in contact with the first electrode may comprise at least one of the organic compound. The hole transport region may further comprise a hole transport material that is different from the organic compound.

In one or more embodiments, the emission layer may comprise a phosphorescent dopant containing a transition metal. The transition metal may be platinum. The emission layer may be to emit blue light.

In one or more embodiments, the light-emitting device may further comprise a capping layer arranged outside (e.g., on) the first electrode and/or outside (e.g., on) the second electrode. For example, in one or more embodiments, the light-emitting device may further comprise a first capping layer arranged outside (e.g., on) the first electrode. For example, in one or more embodiments, the light-emitting device may comprise a first capping layer, a first electrode, a hole transport region, an emission layer, and a second electrode that are sequentially arranged in the stated order. In one or more embodiments, the light-emitting device may further comprise a second capping layer arranged outside (e.g., on) the second electrode. For example, in one or more embodiments, the light-emitting device may comprise a first electrode, a hole transport region, an interlayer, a second electrode, and a second capping layer that are sequentially arranged in the stated order. In one or more embodiments, the light-emitting device may further comprise a first capping layer arranged outside (e.g., on) the first electrode and a second capping layer arranged outside (e.g., on) the second electrode. For example, in one or more embodiments, the light-emitting device may comprise a first capping layer, a first electrode, a hole transport region, an interlayer, a second electrode, and a second capping layer that are sequentially arranged in the stated order.

Because the light-emitting device comprises at least one of the organic compound, the light-emitting device may have excellent or suitable driving voltage, current efficiency, and/or lifespan.

According to one or more embodiments of the present disclosure, an electronic apparatus may comprise: the light-emitting device; and a thin-film transistor electrically connected to the light-emitting device. Because the electronic apparatus comprises the light-emitting device, the electronic apparatus may have excellent or suitable display quality.

According to one or more embodiments of the present, an electronic equipment may comprise the electronic apparatus, wherein the electronic equipment may be at least one of a flat panel display, a curved display, a computer monitor, a medical monitor, a television, a billboard, an indoor light, an outdoor light, a signal light, a head-up display, a fully transparent display, a partially transparent display, a flexible display, a rollable display, a foldable display, a stretchable display, a laser printer, a telephone, a mobile phone, a tablet, a phablet, a personal digital assistant (PDA), a wearable device, a laptop computer, a digital camera, a camcorder, a viewfinder, a micro display, a 3D display, a virtual reality display, an augmented reality display, a vehicle, a video wall comprising multiple displays tiled together, a theater screen, a stadium screen, a phototherapy device, or a signboard.

According to one or more embodiments of the present disclosure, there is provided an organic compound comprising a first moiety represented by Formula 1 and a second moiety represented by Formula 2: 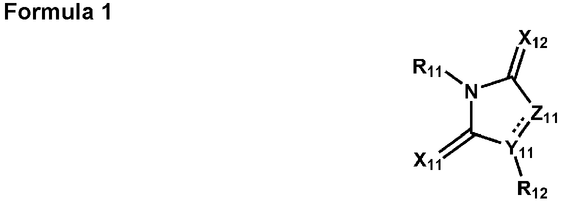 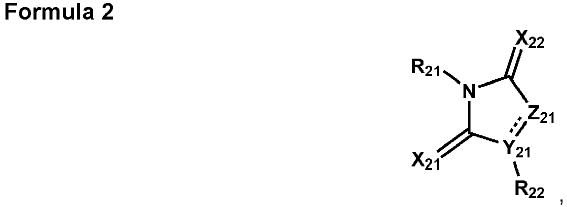
wherein, in Formulae 1 and 2,
a moiety represented by 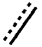 may be a single bond or a double bond, in other words, 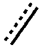 may represent a single bond or a double bond,
X₁₁, X₁₂, X₂₁, and X₂₂ may each independently be O or S,
Y₁₁ may be C, C(R₁₃), or N,
Z₁₁ may be C(R₁₄), N, N(R₁₄), O, or S,
Y₂₁ may be C, C(R₂₃), or N,
Z₂₁ may be C(R₂₄), N, N(R₂₄), O, or S,
R₁₁ to R₁₄ and R₂₁ to R₂₄ may each independently be hydrogen, deuterium, -F, -Cl, - Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂),
R₁₁ or R₁₂ may be a linking site to the second moiety,
R₂₁ or R₂₂ may be a linking site to the first moiety, and
R₁₀ₐ may be as described herein.

The organic compound may comprise (e.g., consist of) the first moiety and the second moiety.

The first moiety and the second moiety may not be condensed with each other. For example, the first moiety and the second moiety may be bonded or linked to each other, and may not be condensed with each other.

In one or more embodiments, R₁₁ in Formula 1 may be a linking site to R₂₁ in Formula 2. In one or more embodiments, R₁₁ in Formula 1 may be a bonding site to R₂₁ in Formula 2. In one or more embodiments, R₁₁ in Formula 1 may be a linking site to R₂₂ in Formula 2. In one or more embodiments, R₁₁ in Formula 1 may be a bonding site to R₂₂ in Formula 2. In one or more embodiments, R₁₂ in Formula 1 may be a linking site to R₂₁ in Formula 2. In one or more embodiments, R₁₂ in Formula 1 may be a bonding site to R₂₁ in Formula 2. In one or more embodiments, R₁₂ in Formula 1 may be a linking site to R₂₂ in Formula 2. In one or more embodiments, R₁₂ in Formula 1 may be a bonding site to R₂₂ in Formula 2.

In one or more embodiments, the first moiety may not comprise (e.g., may exclude) a (e.g., any) condensed ring. The first moiety may comprise a single ring containing at least one nitrogen atom as a ring-forming atom. The first moiety may comprise a 5-membered ring having a structure of Y₁₁-C-N-C-Z₁₁ (wherein Y₁₁ and Z₁₁ are bonded to each other via a single bond or a double bond) sequentially linked to each other. For example, the first moiety may be clearly different from a ring having a ring-forming structure of Y₁₁-C-C-C-Z₁₁ or a ring-forming structure of Y₁₁-C-N=C-Z₁₁.

In one or more embodiments, the second moiety may not comprise (e.g., may exclude) a (e.g., any) condensed ring. The second moiety may comprise a single ring containing at least one nitrogen atom as a ring-forming atom. The second moiety may comprise a 5-membered ring having a structure of Y₂₁-C-N-C-Z₂₁ (wherein Y₂₁ and Z₂₁ are bonded to each other via a single bond or a double bond) sequentially linked to each other. For example, the second moiety may be clearly different from a ring having a ring-forming structure of Y₂₁-C-C-C-Z₂₁ or a ring-forming structure of Y₂₁-C-N=C-Z₂₁.

In one or more embodiments, each of the first moiety and the second moiety may be independently represented by any one selected from among Formulae 11 to 18: 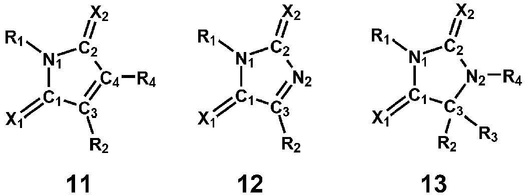 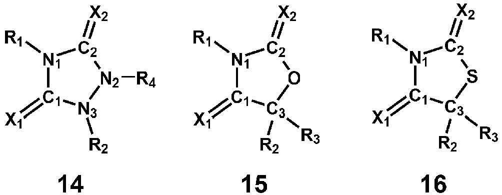 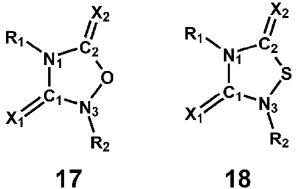
wherein, in Formulae 11 to 18,
N₁ to N₃ may each be nitrogen,
C₁ to C₄ may each be carbon,
X₁ and X₂ may each independently be O or S,
R₁ may be the same as described with respect to R₁₁ or R₂₁ in Formulae 1 and 2,
R₂ may be as described with respect to R₁₂ or R₂₂ in Formulae 1 and 2,
R₃ may be as described with respect to R₁₃ or R₂₃ in Formulae 1 and 2, and
R₄ may be as described with respect to R₁₄ or R₂₄ in Formulae 1 and 2.

Referring to Formulae 11 to 18, in one or more embodiments, the organic compound may comprise a 5-membered single ring containing N₁ as a ring-forming atom. For example, R₂ and R₄ may not be linked to each other to form a condensed ring with the 5-membered single ring, and R₃ and R₄ may not be linked to each other to form a condensed ring with the 5-membered single ring. N₁ may be bonded to R₁ via a single bond. N₁ may be bonded to C₁ via a single bond. N₁ may be bonded to C₂ via a single bond. C₁ may be bonded to X₁ via a double bond. C₂ may be bonded to X₂ via a double bond. For example, the organic compound may be clearly different from a compound that does not contain nitrogen as a ring-forming atom, may be clearly different from a compound in which R₁ is absent so that N₁ is bonded to C₁ or C₂ via a double bond, and may be clearly different from a compound in which R₃ and R₄ are linked to each other to form a ring so that the ring forms a condensed ring with a 5-membered single ring containing N₁.

When the moiety represented by 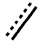 in Formula 1 is a single bond, Y₁₁ may be C(R₁₃) or N, and Z₁₁ may be N(R₁₄), O, or S. When the moiety represented by 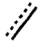 in Formula 1 is a double bond, Y₁₁ may be C, and Z₁₁ may be C(R₁₄) or N.

When the moiety represented by 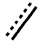 in Formula 2 is a single bond, Y₂₁ may be C(R₂₃) or N, and Z₂₁ may be N(R₂₄), O, or S. When the moiety represented by 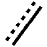 in Formula 2 is a double bond, Y₂₁ may be C, and Z₂₁ may be C(R₂₄) or N.

In one or more embodiments, R₁₁ to R₁₄ and R₂₁ to R₂₄ in Formulae 1 and 2 may each independently be hydrogen, deuterium, -F, -Cl, -Br, -I, a cyano group, a C₁-C₆₀ alkyl group unsubstituted or substituted with at least one R₁₀ₐ, or a C₁-C₆₀ alkoxy group unsubstituted or substituted with at least one R₁₀ₐ, wherein R₁₁ or R₁₂ may be a linking site to the second moiety, and R₂₁ or R₂₂ may be a linking site to the first moiety.

In one or more embodiments, at least one of the first moiety or the second moiety may comprise at least one of deuterium, -F, or a cyano group. For example, in one or more embodiments, at least one selected from among R₁₁ to R₁₄ may be a cyano group. In one or more embodiments, at least one selected from among R₂₁ to R₂₄ may be a cyano group. In one or more embodiments, at least one selected from among R₁₁ to R₁₄ may be a cyano group, and at least one selected from among R₂₁ to R₂₄ may be a cyano group. In one or more embodiments, each of R₁₁ to R₁₄ and R₂₁ to R₂₄ may be a cyano group.

In one or more embodiments, the first moiety and the second moiety may each independently be represented by any one selected from among Formulae M1 to M63: 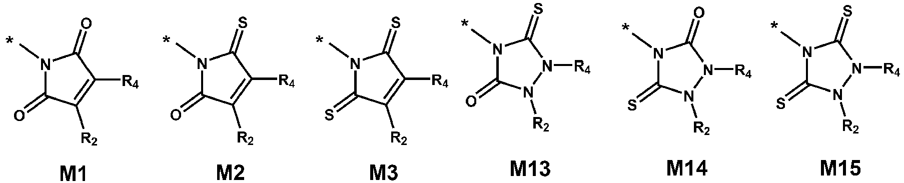 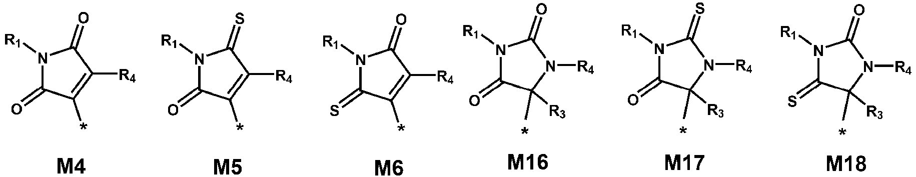 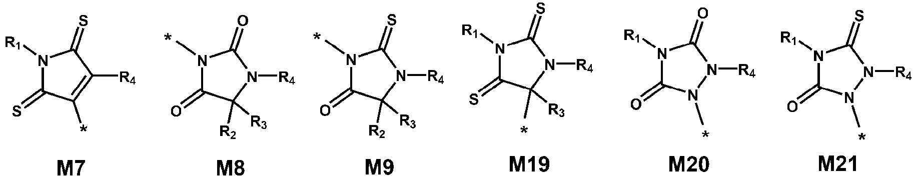 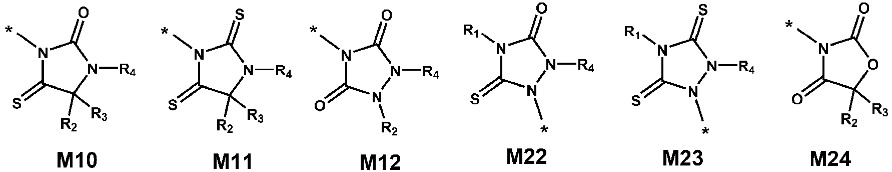 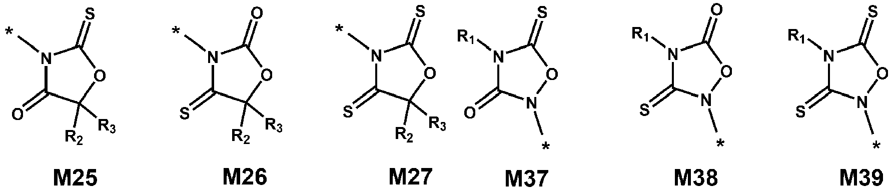 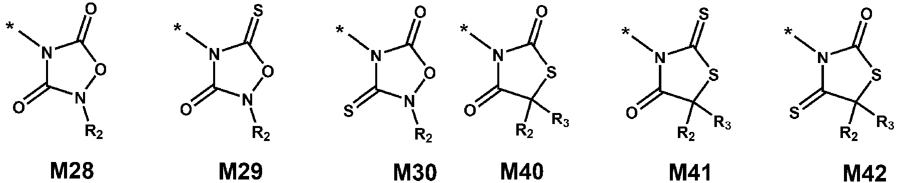 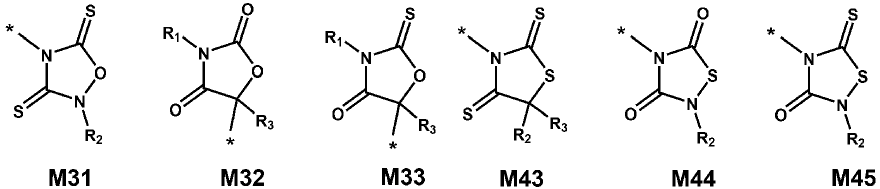 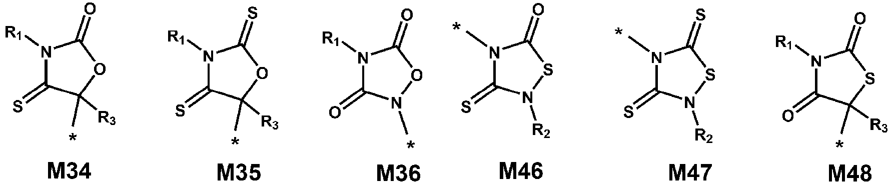 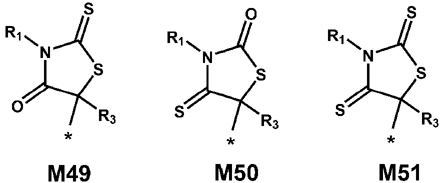 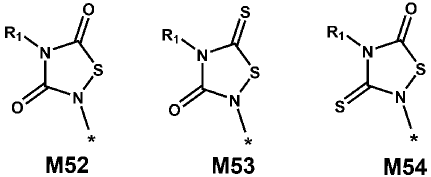 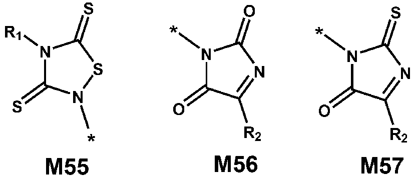 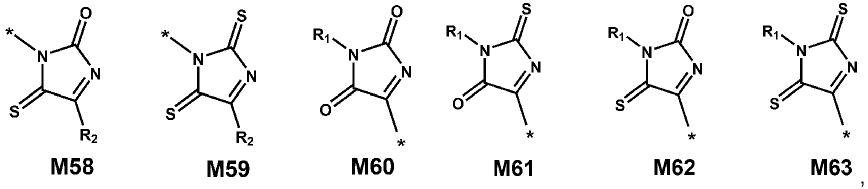
wherein, in Formulae M1 to M63,
R₁ may be the same as described with respect to R₁₁ and R₂₁,
R₂ may be the same as described with respect to R₁₂ and R₂₂,
R₃ may be the same as described with respect to R₁₃ and R₂₃,
R₄ may be the same as described with respect to R₁₄ and R₂₄, and
* may indicate a bonding site to a neighboring atom.

In one or more embodiments, the first moiety and the second moiety may be identical to each other. For example, in one or more embodiments, R₁₁ in Formula 1 may be a linking site to R₂₁ in Formula 2, X₁₁ and X₂₁ may be identical to each other, X₁₂ and X₂₂ may be identical to each other, Y₁₁ and Y₂₁ may be identical to each other, and Z₁₁ and Z₂₁ may be identical to each other. In one or more embodiments, R₁₂ in Formula 1 may be a linking site to R₂₂ in Formula 2, X₁₁ and X₂₁ may be identical to each other, X₁₂ and X₂₂ may be identical to each other, Y₁₁ and Y₂₁ may be identical to each other, and Z₁₁ and Z₂₁ may be identical to each other.

In one or more embodiments, the organic compound may further comprise a third moiety for linking the first moiety and the second moiety to each other. The third moiety may be bonded to each of the first moiety and the second moiety. The organic compound may comprise (e.g., consist of) the first moiety, the second moiety, and the third moiety. The first moiety and the second moiety may have a symmetrical structure with respect to the third moiety.

In one or more embodiments, the third moiety may be *-(L₁)ₙ₁-*', *-(L₁)ₙ₂=(L₂)ₙ₃-*', or *-C(R₁)=(L₁)ₙ₄=C(R₂)-*'.

In the third moiety, L₁ and L₂ may each independently be a C₃-C₆₀ carbocyclic group unsubstituted or substituted with at least one R_{10b} or a C₁-C₆₀ heterocyclic group unsubstituted or substituted with at least one R_{10b},
n1 to n4 may each independently be an integer from 0 to 5,
* may indicate a bonding site to the first moiety,
*' may indicate a bonding site to the second moiety,
R₁ and R₂ may each independently be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂),
R₁₀ₐ and Q₁ to Q₃ may each be the same as described herein, and
R_{10b} may be the same as described with respect to R₁₀ₐ.

When n1 is 0, *-(L₁)ₙ₁-*' may indicate a single bond. When n1 is 2 or greater, a plurality of L₁(s) may be identical to or different from each other. When n2 is 0, *-(L₁)ₙ₂=(L₂)ₙ₃-*' may indicate *=(L₂)ₙ₃-*'. When n2 is 2 or greater, a plurality of L₁(s) may be identical to or different from each other. When n3 is 0, *-(L₁)ₙ₂=(L₂)ₙ₃-*' may indicate *-(L₁)ₙ₂=*'. When n3 is 2 or greater, a plurality of L₂(s) may be identical to or different from each other. When n2 is 0 and n3 is 0, *-(L₁)ₙ₂=(L₂)ₙ₃-*' may indicate a double bond. When n4 is 0, *-C(R₁)=(L₁)ₙ₄=C(R₂)-*' may indicate *-C(R₁)=C(R₂)-*'. When n4 is 2 or greater, a plurality of L₁(s) may be identical to or different from each other.

In one or more embodiments, the sum of n2 and n3 may be 1 or greater. For example, in one or more embodiments, the third moiety may not be a double bond.

In one or more embodiments, n1 may be 0, 1, 2, or 3, n2 may be 1 or 2, n3 may be 1 or 2, and n4 may be 1 or 2.

In one or more embodiments, L₁ and L₂ may each independently be an unsubstituted C₃-C₆₀ carbocyclic group or an unsubstituted C₁-C₆₀ heterocyclic group. In one or more embodiments, L₁ and L₂ may each independently be a C₃-C₆₀ carbocyclic group substituted with one R_{10b} or a C₁-C₆₀ heterocyclic group substituted with one R_{10b}. In one or more embodiments, L₁ and L₂ may each independently be a C₃-C₆₀ carbocyclic group substituted with two R_{10b} or a C₁-C₆₀ heterocyclic group substituted with two R_{10b}. In one or more embodiments, L₁ and L₂ may each independently be a C₃-C₆₀ carbocyclic group substituted with three R_{10b} or a C₁-C₆₀ heterocyclic group substituted with three R_{10b}. In one or more embodiments, L₁ and L₂ may each independently be a C₃-C₆₀ carbocyclic group substituted with four R_{10b} or a C₁-C₆₀ heterocyclic group substituted with four R_{10b}.

L₁ and L₂ may each independently be a single ring or a condensed ring. In one or more embodiments, L₁ and L₂ may each independently be i) a 5-membered carbocyclic group, ii) a 5-membered heterocyclic group, iii) a 6-membered carbocyclic group, iv) a 6-membered heterocyclic group, v) a 7-membered carbocyclic group, vi) a 7-membered heterocyclic group, vii) an 8-membered carbocyclic group, viii) an 8-membered heterocyclic group, or ix) a polycyclic group that is a condensed ring of any two or more thereof.

In one or more embodiments, L₁ and L₂ may each independently be a C₆-C₆₀ arylene group unsubstituted or substituted with at least one R_{10b} or a C₁-C₆₀ heteroarylene group unsubstituted or substituted with at least one R_{10b}.

In one or more embodiments, L₁ and L₂ may each independently be a cyclopentene group, a cyclopentadiene group, a cyclohexene group, a cyclohexadiene group, a cyclooctatetraene group, a benzene group, a naphthalene group, an anthracene group, a phenanthrene group, a perylene group, a phenalene group, a pyrene group, a tetracene group, a triphenylene group, a pyridine group, a pyrimidine group, a triazine group, a pyrrole group, an imidazole group, a thiazole group, an oxazole group, a furan group, a thiophene group, a carbazole group, a dibenzofuran group, a dibenzothiophene group, a pyridoquinolizine group, a dihydropyridine group, a dihydropyrazine group, or a condensed cyclic group formed by any combination thereof.

In one or more embodiments, the third moiety may be *-L₁-*', where L₁ may be an unsubstituted C₃-C₆₀ carbocyclic group or an unsubstituted C₁-C₆₀ heterocyclic group. In one or more embodiments, the third moiety may be *-L₁-*', where L₁ may be a C₃-C₆₀ carbocyclic group substituted with one R_{10b} or a C₁-C₆₀ heterocyclic group substituted with one R_{10b}.

In one or more embodiments, R_{10b} may comprise a group represented by Formula 3: 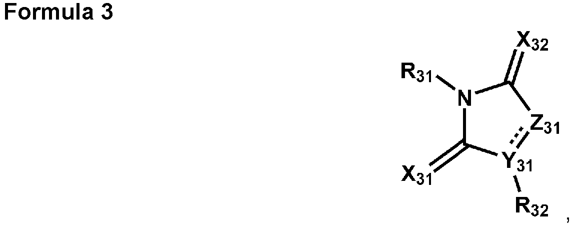
wherein, in Formula 3,
a moiety represented by 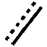 may be a single bond or a double bond,
X₃₁ and X₃₂ may each independently be O or S,
Y₃₁ may be C, C(R₃₃), or N,
Z₃₁ may be C(R₃₄), N, N(R₃₄), O, or S,
R₃₁ to R₃₄ may each independently be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂),
R₃₁ or R₃₂ may be a bonding site to a neighboring atom, and
R₁₀ₐ and Q₁ to Q₃ may each be the same as described herein.

In one or more embodiments, Formula 3 may be as described herein for Formulae 1 and 2. In one or more embodiments, Formula 3 may be any one selected from Formulae M1 to M63.

In one or more embodiments, the first moiety, the second moiety, and the group represented by Formula 3 may be identical to each other.

For example, in one or more embodiments, L₁ may be a phenylene group substituted with at least one R_{10b}, R_{10b} may be a benzene group substituted with a C₁-C₆₀ heterocyclic group, and the C₁-C₆₀ heterocyclic group may be a group represented by Formula 3. For another example, in one or more embodiments, L₁ may be a phenylene group substituted with at least one R_{10b}, R_{10b} may be a group represented by Formula 3.

In one or more embodiments, R₁ and R₂ in the third moiety may each independently be a group represented by Formula 4: 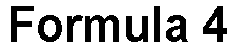 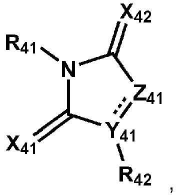
wherein, in Formula 4,
a moiety represented by 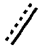 may be a single bond or a double bond,
X₄₁ and X₄₂ may each independently be O or S,
Y₄₁ may be C, C(R₄₃), or N,
Z₄₁ may be C(R₄₄), N, N(R₄₄), O, or S,
R₄₁ to R₄₄ may each independently be hydrogen, deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, -Si(Q₃₁)(Q₃₂)(Q₃₃), -N(Q₃₁)(Q₃₂), -B(Q₃₁)(Q₃₂), -C(=O)(Q₃₁), -S(=O)2(Q31), or - P(=O)(Q₃₁)(Q₃₂),
R₄₁ or R₄₂ may be a bonding site to a neighboring atom, and
Q₃₁ to Q₃₃ may each be the same as described herein.

In one or more embodiments, Formula 4 may be as described herein for Formulae 1 and 2. In one or more embodiments, Formula 4 may be any one selected from among Formulae M1 to M63.

In one or more embodiments, the first moiety, the second moiety, the group represented by Formula 3, and the group represented by Formula 4 may be identical to each other. In one or more embodiments, the first moiety, the second moiety, and the group represented by Formula 3 may be identical to each other. In one or more embodiments, the first moiety, the second moiety, and the group represented by Formula 4 may be identical to each other.

In one or more embodiments, the organic compound may not comprise (e.g., may exclude any) fluorine (F). The environment may be polluted during the preparation or use of compounds comprising fluorine. In this regard, because the organic compound does not comprise fluorine, environmental pollution may be prevented or reduced during the preparation or use of the organic compound. The organic compound may not comprise (e.g., may exclude any) fluorine and may have excellent or suitable highest occupied molecular orbital (HOMO) energy level, lowest unoccupied molecular orbital (LUMO) energy level, hole mobility, electron mobility, and glass transition temperature. Thus, the organic compound may be suitable for use as a hole transport material.

In one or more embodiments, the organic compound may be one of (e.g., selected from among) Compounds 1 to 39:

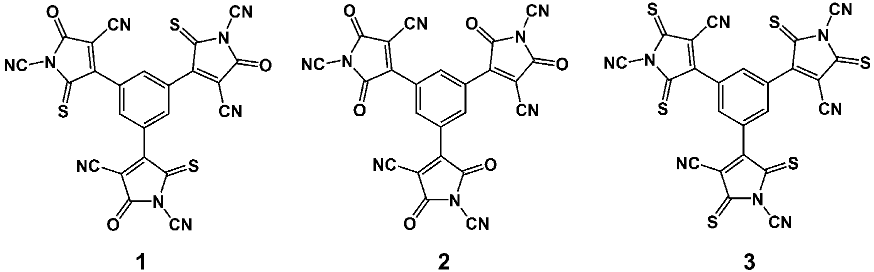

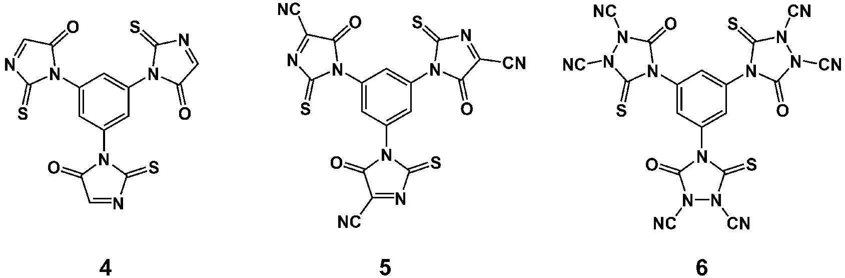

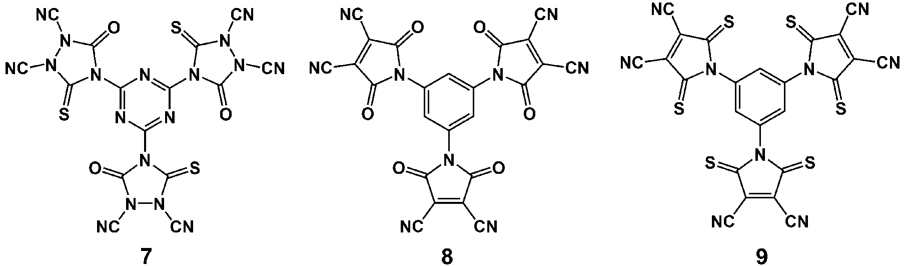

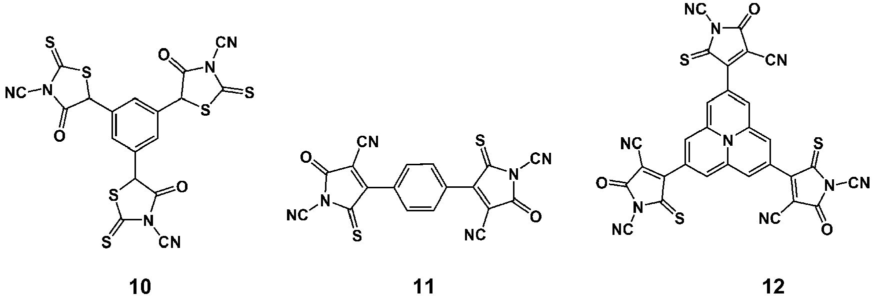

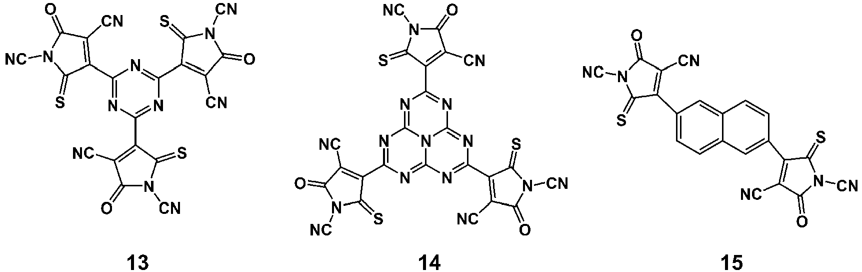

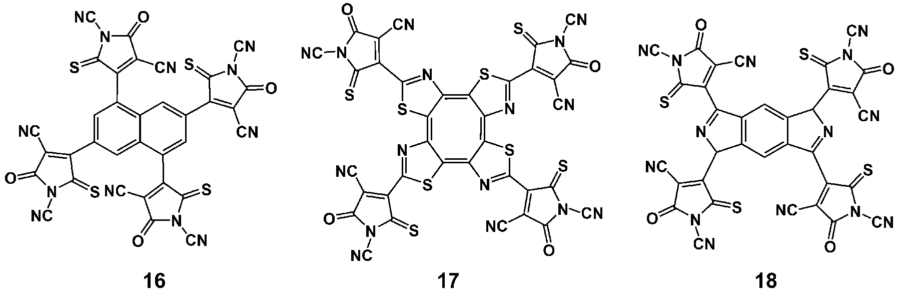

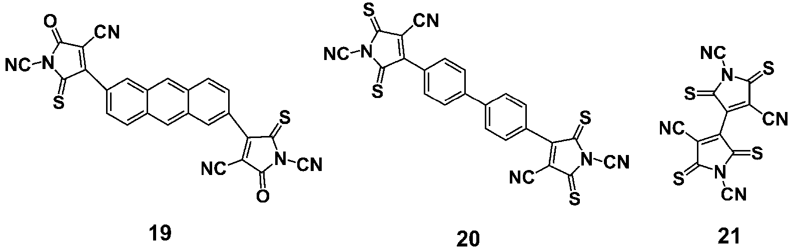

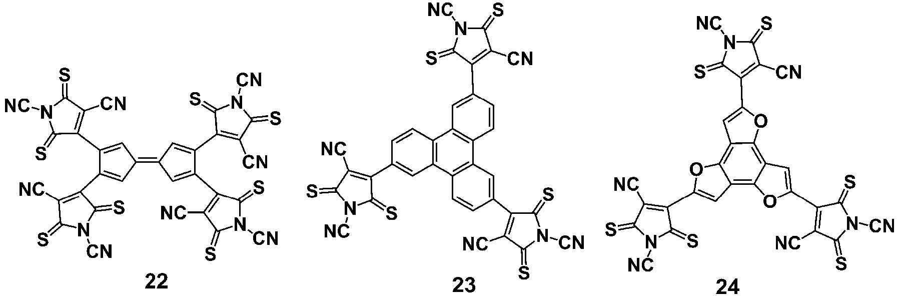

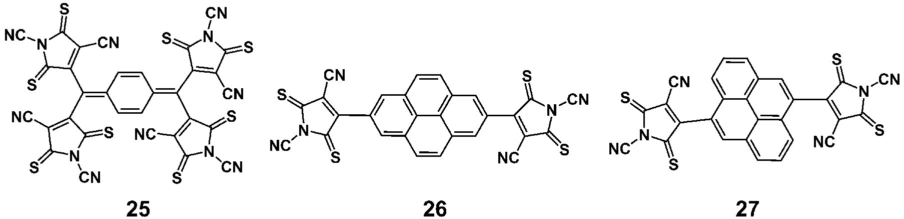

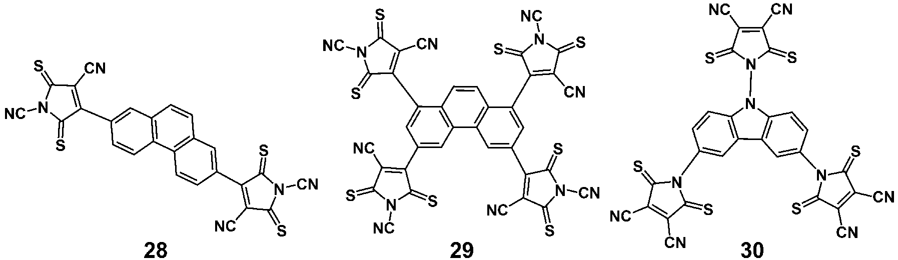

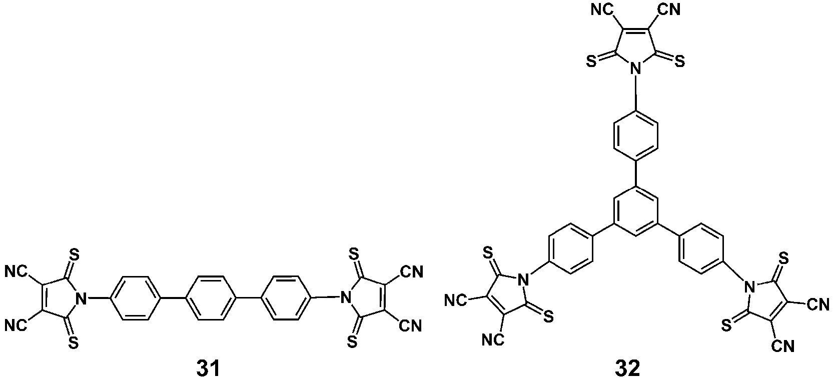

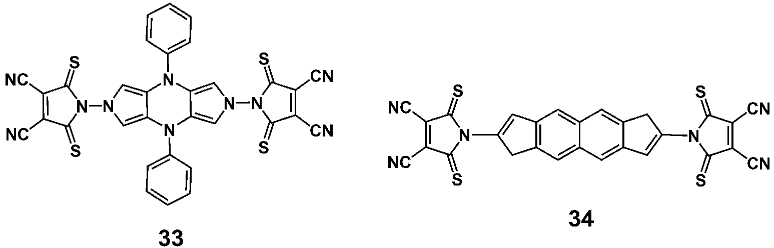

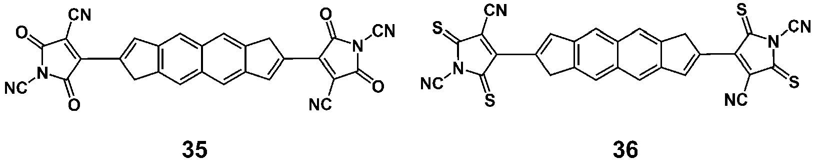

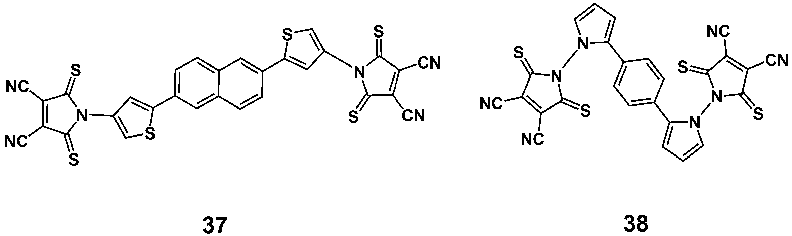

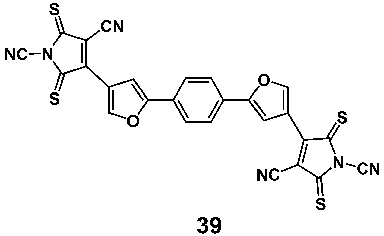

The organic compound may comprise two or more 5-membered rings each containing, as a ring-forming atom, a nitrogen atom bonded to three different atoms, wherein each of the 5-membered rings may contain, as ring-forming atoms, two carbon atoms bonded to the nitrogen atom, each of the two carbon atoms may be bonded to an oxygen atom or a sulfur atom via a double bond, and each of the 5-membered rings may be a single ring that is not condensed with another ring. As a result, the organic compound may have a HOMO energy level and a LUMO energy level that are suitable for use as a hole transport material, may have high hole mobility, and may concurrently (e.g., simultaneously) have a high glass transition temperature. For example, the organic compound may have excellent or suitable hole-transporting characteristics and thermal stability and morphological stability. Accordingly, a light-emitting device comprising the organic compound may have a low driving voltage, high current efficiency, and a long lifespan.

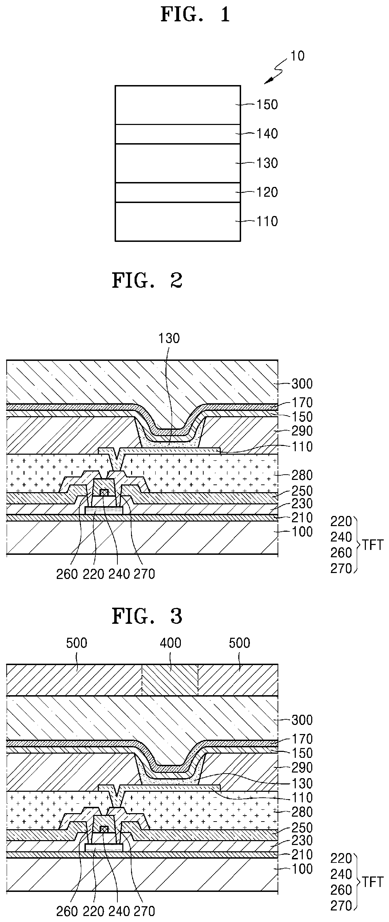

### Description of FIG. 1

FIG. 1 is a schematic cross-sectional view of a light-emitting device 10 according to one or more embodiments of the present disclosure. The light-emitting device 10 may comprise a first electrode 110, an interlayer, and a second electrode 150. The interlayer may comprise a hole transport region 120, an emission layer 130, and an electron transport region 140.

Hereinafter, the structure of the light-emitting device 10 according to one or more embodiments and a method of manufacturing the light-emitting device 10 will be described in more detail with reference to FIG. 1.

### First electrode 110

In FIG. 1, in one or more embodiments, a substrate may be additionally provided and arranged under the first electrode 110 and/or on the second electrode 150. As the substrate, a glass substrate or a plastic substrate may be used. In one or more embodiments, the substrate may be a flexible substrate. For example, the substrate may comprise plastics with excellent or suitable heat resistance and durability, such as polyimide, polyethylene terephthalate (PET), polycarbonate, polyethylene naphthalate, polyarylate (PAR), polyetherimide, or any combination thereof.

The first electrode 110 may be formed by depositing or sputtering a material for forming the first electrode 110 on the substrate. When the first electrode 110 is an anode, a high-work function material that facilitates injection of holes may be used as a material for forming the first electrode 110.

The first electrode 110 may be a reflective electrode, a transflective electrode, or a transmissive electrode. In one or more embodiments, if (e.g., when) the first electrode 110 is a transmissive electrode, a material for forming the first electrode 110 may comprise indium tin oxide (ITO), indium zinc oxide (IZO), tin oxide (SnO₂), zinc oxide (ZnO), or any combination thereof. In one or more embodiments, if (e.g., when) the first electrode 110 is a transflective electrode or a reflective electrode, a material for forming the first electrode 110 may comprise magnesium (Mg), silver (Ag), aluminum (Al), aluminum-lithium (Al-Li), calcium (Ca), magnesium-indium (Mg-In), magnesium-silver (Mg-Ag), or any combination thereof.

The first electrode 110 may have a single-layer structure comprising (e.g., consisting of) a single layer or a multi-layer structure comprising multiple layers. For example, in one or more embodiments, the first electrode 110 may have a three-layer structure of ITO/Ag/ITO.

### Interlayer

The interlayer may be arranged on the first electrode 110. The interlayer may comprise the hole transport region 120, the emission layer 130, and the electron transport region 140.

The interlayer may comprise one or more suitable organic materials, a metal-containing compound such as an organometallic compound, an inorganic material such as quantum dots, and/or the like. For example, in one or more embodiments, the interlayer may comprise the organic compound of one or more embodiments of the present disclosure.

In one or more embodiments, the interlayer may comprise i) two or more emitting units sequentially stacked between the first electrode 110 and the second electrode 150, and ii) a charge generation layer between adjacent emitting units among the two or more emitting units. When the interlayer comprises the two or more emitting units and the charge generation layer as described herein, the light-emitting device 10 may be a tandem light-emitting device.

### Hole transport region 120

The hole transport region 120 may have i) a single-layer structure comprising (e.g., consisting of) a single layer comprising (e.g., consisting of) a single material, ii) a single-layer structure comprising (e.g., consisting of) a single layer comprising multiple materials that are different from each other, or iii) a multi-layer structure comprising multiple layers comprising multiple materials that are different from each other.

The hole transport region 120 may comprise a hole injection layer, a hole transport layer, an emission auxiliary layer, an electron-blocking layer, or any combination thereof.

In one or more embodiments, the hole transport region 120 may have a multi-layer structure comprising a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron-blocking layer structure, wherein constituent layers of each structure are stacked sequentially from the first electrode 110 in the stated order.

In one or more embodiments, the hole transport region 120 may comprise the organic compound of the present disclosure. For example, in one or more embodiments, the hole transport region 120 may comprise at least one selected from among Compounds 1 to 39 of the present disclosure. In one or more embodiments, the hole transport region 120 may comprise a hole injection layer, and the hole injection layer may comprise the organic compound of the present disclosure, for example, the hole injection layer may comprise at least one selected from among Compounds 1 to 39 of the present disclosure.

In one or more embodiments, the hole transport region 120 may comprise a compound represented by Formula 201, a compound represented by Formula 202, or any combination thereof: 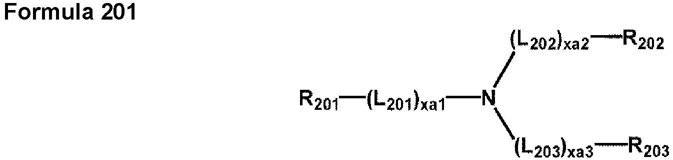 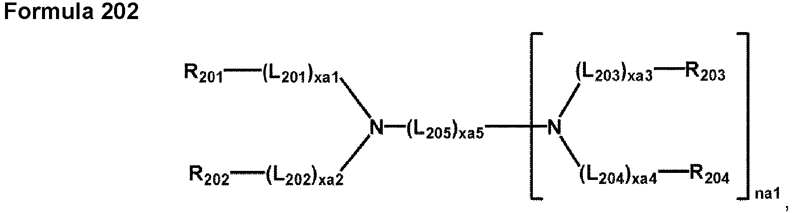
wherein, in Formulae 201 and 202,
L₂₀₁ to L₂₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
L₂₀₅ may be *-O-*', *-S-*', *-N(Q₂₀₁)-*', a C₁-C₂₀ alkylene group unsubstituted or substituted with at least one R₁₀ₐ, a C₂-C₂₀ alkenylene group unsubstituted or substituted with at least one R₁₀ₐ, a C₃-C₆₀ carbocyclic group unsubstituted or substituted with at least one R₁₀ₐ, or a C₁-C₆₀ heterocyclic group unsubstituted or substituted with at least one R₁₀ₐ,
xa1 to xa4 may each independently be an integer from 0 to 5,
xa5 may be an integer from 1 to 10,
R₂₀₁ to R₂₀₄ and Q₂₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
R₂₀₁ and R₂₀₂ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ, to form a C₈-C₆₀ polycyclic group (for example, a carbazole group, and/or the like) unsubstituted or substituted with at least one R₁₀ₐ (for example, see Compound HT16, and/or the like),
R₂₀₃ and R₂₀₄ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ, to form a C₈-C₆₀ polycyclic group unsubstituted or substituted with at least one R₁₀ₐ, and
na1 may be an integer from 1 to 4.

In one or more embodiments, each of Formulae 201 and 202 may comprise at least one selected from among groups represented by Formulae CY201 to CY217: 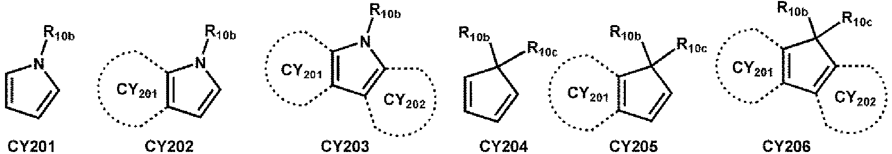 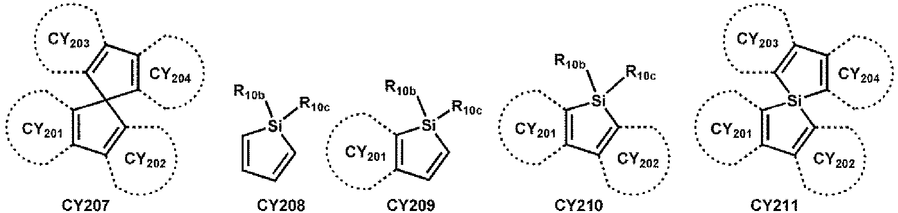 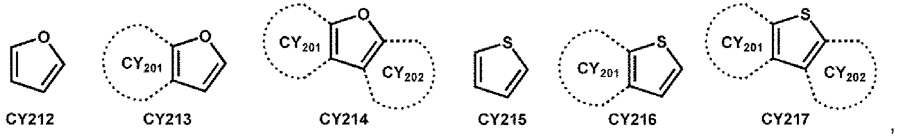 wherein, in Formulae CY201 to CY217, R_{10b} and R_{10c} may each be the same as described with respect to R₁₀ₐ, ring CY₂₀₁ to ring CY₂₀₄ may each independently be a C₃-C₂₀ carbocyclic group or a C₁-C₂₀ heterocyclic group, and at least one hydrogen in Formulae CY201 to CY217 may be unsubstituted or substituted with R₁₀ₐ.

In one or more embodiments, ring CY₂₀₁ to ring CY₂₀₄ in Formulae CY201 to CY217 may each independently be a benzene group, a naphthalene group, a phenanthrene group, or an anthracene group.

In one or more embodiments, each of Formulae 201 and 202 may comprise at least one selected from among the groups represented by Formulae CY201 to CY203.

In one or more embodiments, Formula 201 may comprise at least one selected from among the groups represented by Formulae CY201 to CY203 and at least one selected from among the groups represented by Formulae CY204 to CY217.

In one or more embodiments, in Formula 201, xa1 may be 1, R₂₀₁ may be a group represented by one selected from among Formulae CY201 to CY203, xa2 may be 0, and R₂₀₂ may be a group represented by one selected from among Formulae CY204 to CY207.

In one or more embodiments, each of Formulae 201 and 202 may not comprise (e.g., may exclude) any of the groups represented by Formulae CY201 to CY203.

In one or more embodiments, each of Formulae 201 and 202 may not comprise (e.g., may exclude) any of the groups represented by Formulae CY201 to CY203 and may comprise at least one selected from among the groups represented by Formulae CY204 to CY217.

In one or more embodiments, each of Formulae 201 and 202 may not comprise (e.g., may exclude) any of the groups represented by Formulae CY201 to CY217.

In one or more embodiments, the hole transport region 120 may comprise: one of (e.g., comprise at least one or be any one selected from among) Compounds HT1 to HT46; 4,4',4"-[tris(3-methylphenyl)phenylamino] triphenylamine (m-MTDATA); 4,4',4"-tris(N,N-diphenylamino)triphenylamine (TDATA); 4,4',4"-tris[N-(2-naphthyl)-N-phenylamino]-triphenylamine (2-TNATA); N,N'-di(naphthalen-1-yl)-N,N'-diphenyl-benzidine (NPB(NPD)); β-NPB; N,N'-bis(3-methylphenyl)-N,N'-diphenyl-[1,1'-biphenyl]-4,4'-diamine (TPD); spiro-TPD; spiro-NPB; methylated NPB; 4,4'-cyclohexylidene bis[N,N-bis(4-methylphenyl)benzenamine] (TAPC); 4,4'-bis[N,N'-(3-tolyl)amino]-3,3'-dimethylbiphenyl (HMTPD); 4,4',4"-tris(N-carbazolyl)triphenylamine (TCTA); polyaniline/dodecylbenzenesulfonic acid (PANI/DBSA); poly(3,4-ethylenedioxythiophene)/poly(4-styrenesulfonate) (PEDOT/PSS); polyaniline/camphor sulfonic acid (PANI/CSA); polyaniline/poly(4-styrenesulfonate) (PANI/PSS); or any combination thereof:

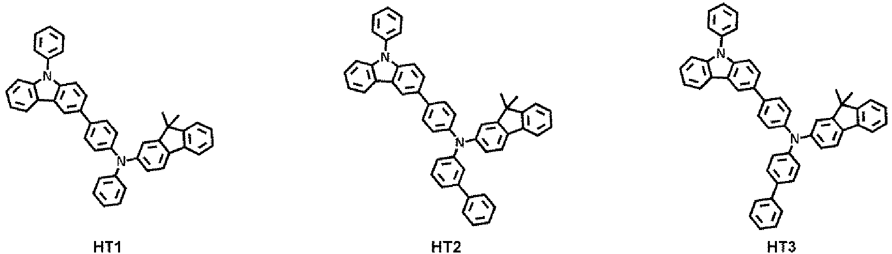

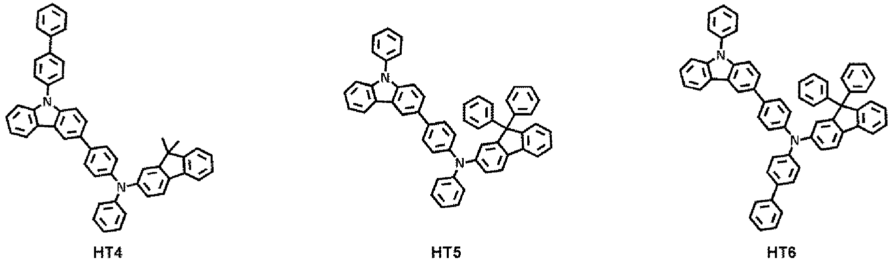

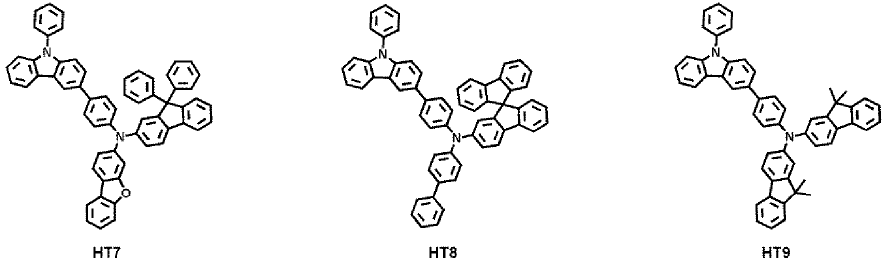

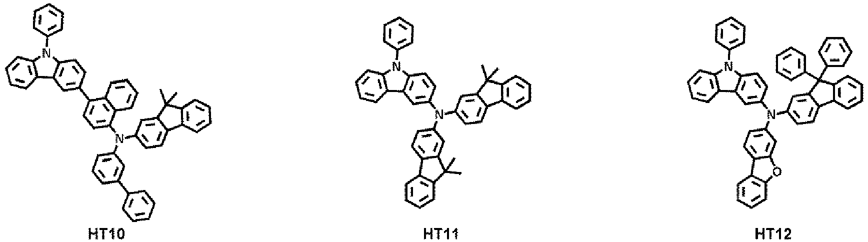

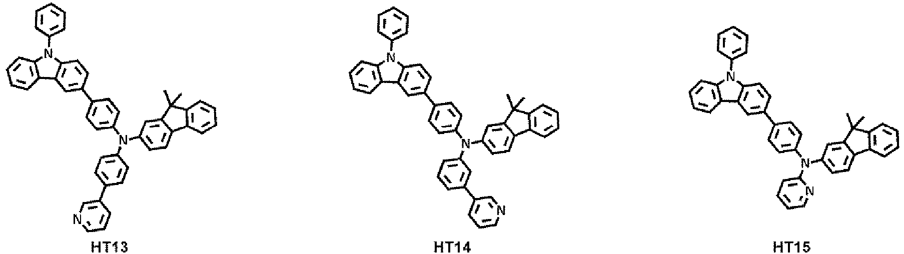

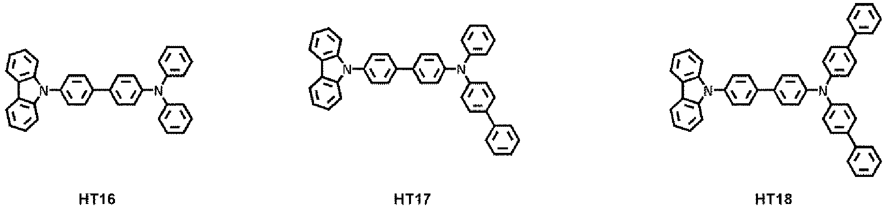

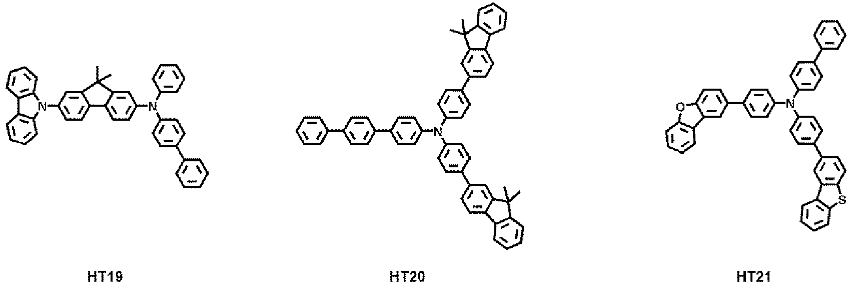

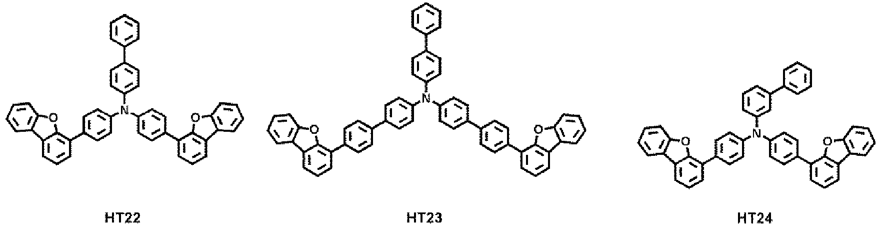

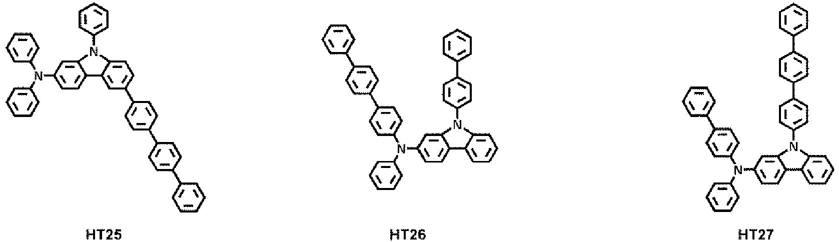

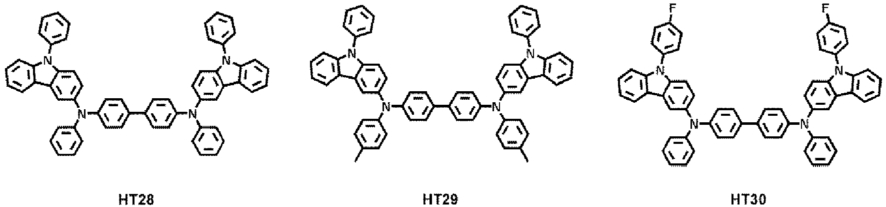

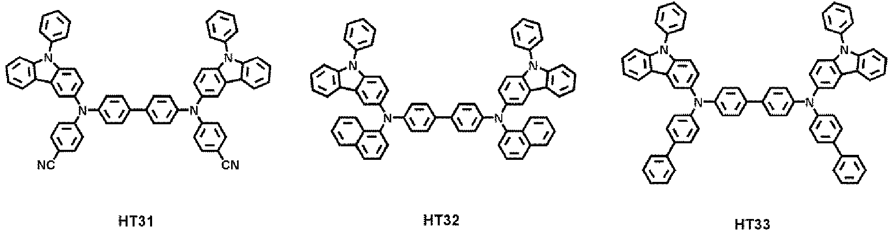

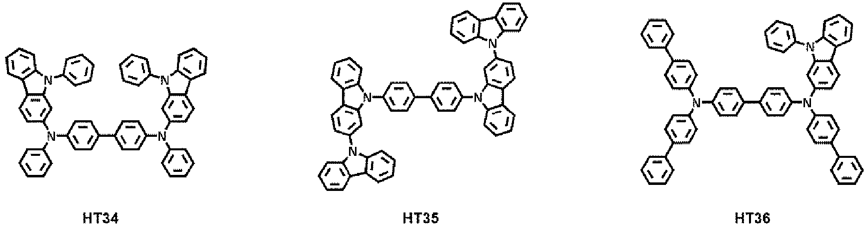

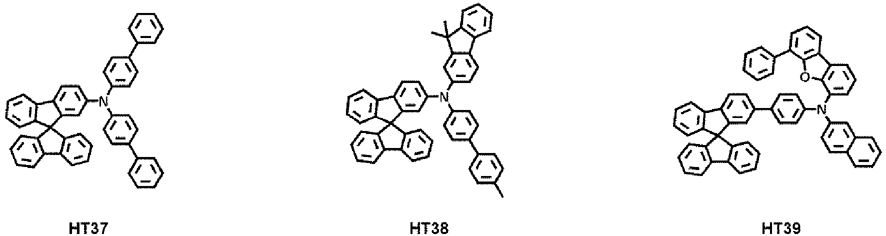

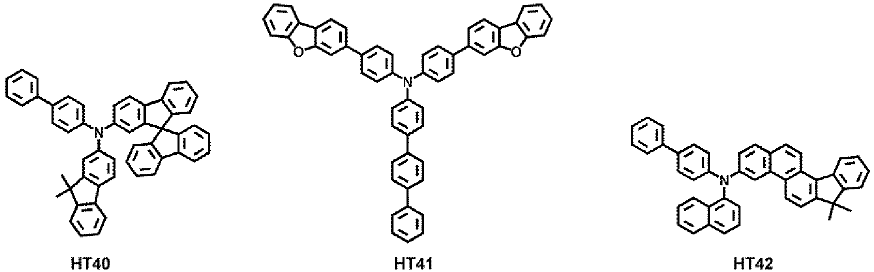

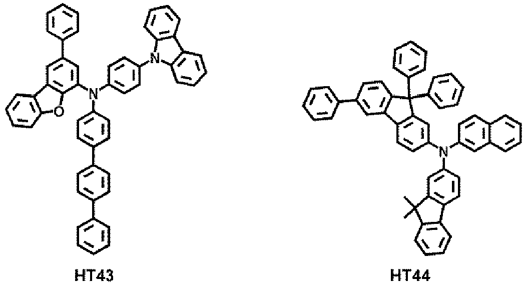

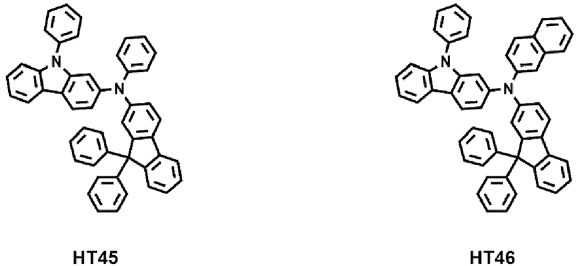

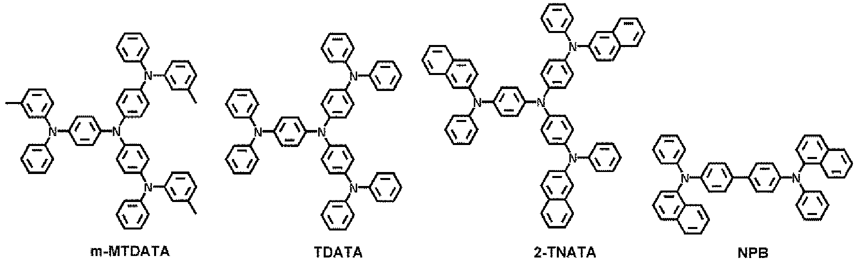

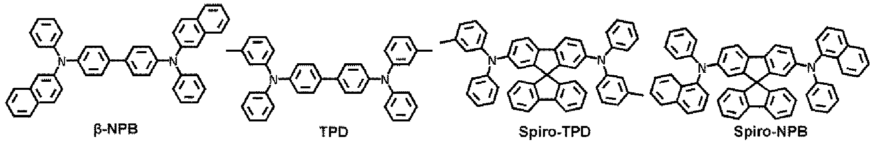

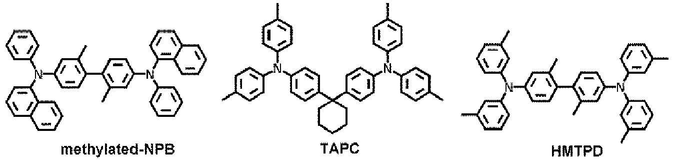

A thickness of the hole transport region 120 may be in a range of about 50 Angstroms (Å) to about 10,000 Å, for example, about 100 Å to about 4,000 Å. When the hole transport region 120 comprises a hole injection layer, a hole transport layer, or any combination thereof, a thickness of the hole injection layer may be in a range of about 100 Å to about 9,000 Å, for example, about 100 Å to about 1,000 Å, and a thickness of the hole transport layer may be in a range of about 50 Å to about 2,000 Å, for example, about 100 Å to about 1,500 Å. When the thicknesses of the hole transport region, the hole injection layer, and the hole transport layer are within the respective ranges described above, satisfactory hole-transporting characteristics may be obtained without a substantial increase in driving voltage.

The emission auxiliary layer may serve to increase light-emission efficiency by compensating for an optical resonance distance according to the wavelength of light emitted by the emission layer 130. The electron blocking layer may be a layer that prevents electron leakage from the emission layer 130 to the hole transport region 120. Materials that may be included in the hole transport region 120 may be included in the emission auxiliary layer and the electron-blocking layer.

### p-dopant

In one or more embodiments, the hole transport region 120 may comprise, in addition to one or more of the materials described above, a charge-generation material for the improvement of conductive properties. The charge-generation material may be uniformly (e.g., substantially uniformly) or non-uniformly dispersed in the hole transport region 120 (for example, in the form of a single layer comprising (e.g., consisting of) a charge-generation material).

The charge-generation material may be, for example, a p-dopant.

In one or more embodiments, the p-dopant may have a LUMO energy level of about -3.5 eV or less.

In one or more embodiments, the p-dopant may comprise a quinone derivative, a cyano group-containing compound, a compound comprising an element EL1 and an element EL2, or any combination thereof.

Non-limiting examples of the quinone derivative may comprise tetracyanoquinodimethane (TCNQ), 2,3,5,6-tetrafluoro-7,7,8,8-tetracyanoquinodimethane (F4-TCNQ), and/or the like.

Non-limiting examples of the cyano group-containing compound may comprise dipyrazino[2,3-f: 2',3'-h] quinoxaline-2,3,6,7,10,11-hexacarbonitrile (HAT-CN), a compound represented by Formula 221, and/or the like: 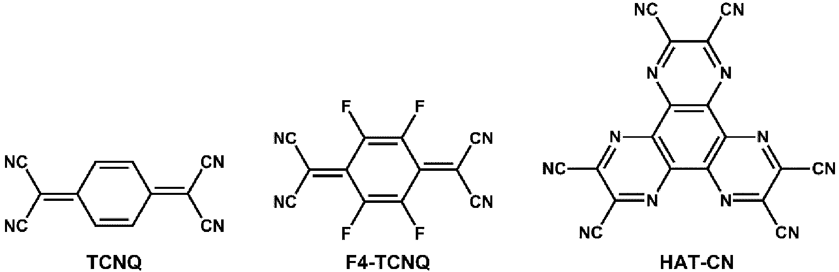 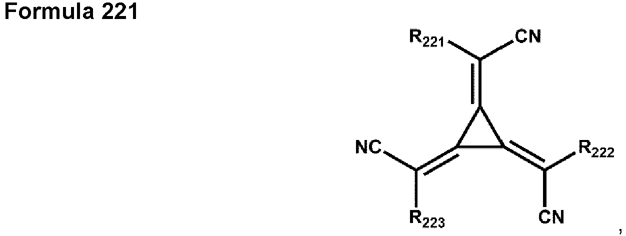
wherein, in Formula 221,
R₂₂₁ to R₂₂₃ may each independently be a C₃-C₆₀ carbocyclic group unsubstituted or substituted with at least one R₁₀ₐ or a C₁-C₆₀ heterocyclic group unsubstituted or substituted with at least one R₁₀₄, and
at least one selected from among R₂₂₁ to R₂₂₃ may each independently be a C₃-C₆₀ carbocyclic group or a C₁-C₆₀ heterocyclic group, each substituted with: a cyano group; -F; -Cl; -Br; -I; a C₁-C₂₀ alkyl group substituted with a cyano group, -F, -Cl, -Br, -I, or any combination thereof; or any combination thereof.

In the compound comprising the element EL1 and the element EL2, the element EL1 may be a metal, a metalloid, and/or a (e.g., any suitable) combination thereof, and the element EL2 may be a non-metal, a metalloid, and/or a (e.g., any suitable) combination thereof.

Non-limiting examples of the metal may comprise: an alkali metal (for example, lithium (Li), sodium (Na), potassium (K), rubidium (Rb), cesium (Cs), and/or the like); an alkaline earth metal (for example, beryllium (Be), magnesium (Mg), calcium (Ca), strontium (Sr), barium (Ba), and/or the like); a transition metal (for example, titanium (Ti), zirconium (Zr), hafnium (Hf), vanadium (V), niobium (Nb), tantalum (Ta), chromium (Cr), molybdenum (Mo), tungsten (W), manganese (Mn), technetium (Tc), rhenium (Re), iron (Fe), ruthenium (Ru), osmium (Os), cobalt (Co), rhodium (Rh), iridium (Ir), nickel (Ni), palladium (Pd), platinum (Pt), copper (Cu), silver (Ag), gold (Au), and/or the like); a post-transition metal (for example, zinc (Zn), indium (In), tin (Sn), and/or the like); a lanthanide metal (for example, lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), and/or the like); and/or the like.

Non-limiting examples of the metalloid may comprise silicon (Si), antimony (Sb), tellurium (Te), and/or the like.

Non-limiting examples of the non-metal may comprise oxygen (O), a halogen (for example, F, Cl, Br, I, and/or the like), and/or the like.

Non-limiting examples of the compound comprising the element EL1 and the element EL2 may comprise a metal oxide, a metal halide (for example, a metal fluoride, a metal chloride, a metal bromide, a metal iodide, and/or the like), a metalloid halide (for example, a metalloid fluoride, a metalloid chloride, a metalloid bromide, a metalloid iodide, and/or the like), a metal telluride, or any combination thereof.

Non-limiting examples of the metal oxide may comprise a tungsten oxide (for example, WO, W₂O₃, WO₂, WO₃, W₂O₅, and/or the like), a vanadium oxide (for example, VO, V₂O₃, VO₂, V₂O₅, and/or the like), a molybdenum oxide (for example, MoO, Mo₂O₃, MoO₂, MoO₃, Mo₂O₅, and/or the like), a rhenium oxide (for example, ReO₃, and/or the like), and/or the like.

Non-limiting examples of the metal halide may comprise an alkali metal halide, an alkaline earth metal halide, a transition metal halide, a post-transition metal halide, a lanthanide metal halide, and/or the like.

Non-limiting examples of the alkali metal halide may comprise LiF, NaF, KF, RbF, CsF, LiCl, NaCl, KCI, RbCl, CsCl, LiBr, NaBr, KBr, RbBr, CsBr, Lil, Nal, KI, Rbl, Csl, and/or the like.

Non-limiting examples of the alkaline earth metal halide may comprise BeF₂, MgF₂, CaF₂, SrF₂, BaF₂, BeCl₂, MgCl₂, CaCl₂, SrCl₂, BaCl₂, BeBr₂, MgBr₂, CaBr₂, SrBr₂, BaBr₂, Bel₂, MgI₂, CaI₂, SrI₂, BaI₂, and/or the like.

Non-limiting examples of the transition metal halide may comprise a titanium halide (for example, TiF₄, TiCl₄, TiBr₄, TiI₄, and/or the like), a zirconium halide (for example, ZrF₄, ZrCl₄, ZrBr₄, ZrI₄, and/or the like), a hafnium halide (for example, HfF₄, HfCl₄, HfBr₄, HfI₄, and/or the like), a vanadium halide (for example, VF₃, VCl₃, VBr₃, Vl₃, and/or the like), a niobium halide (for example, NbF₃, NbCl₃, NbBr₃, Nbl₃, and/or the like), a tantalum halide (for example, TaF₃, TaCl₃, TaBr₃, TaI₃, and/or the like), a chromium halide (for example, CrF₃, CrCl₃, CrBr₃, CrI₃, and/or the like), a molybdenum halide (for example, MoF₃, MoCl₃, MoBr₃, MoI₃, and/or the like), a tungsten halide (for example, WF₃, WCl₃, WBr₃, WI₃, and/or the like), a manganese halide (for example, MnF₂, MnCl₂, MnBr₂, MnI₂, and/or the like), a technetium halide (for example, TcF₂, TcCl₂, TcBr₂, TcI₂, and/or the like), a rhenium halide (for example, ReF₂, ReCl₂, ReBr₂, ReI₂, and/or the like), an iron(II) halide (for example, FeF₂, FeCl₂, FeBr₂, FeI₂, and/or the like), a ruthenium halide (for example, RuF₂, RuCl₂, RuBr₂, RuI₂, and/or the like), an osmium halide (for example, OsF₂, OsCl₂, OsBr₂, OsI₂, and/or the like), a cobalt halide (for example, CoF₂, CoCl₂, CoBr₂, CoI₂, and/or the like), a rhodium halide (for example, RhF₂, RhCl₂, RhBr₂, RhI₂, and/or the like), an iridium halide (for example, IrF₂, IrCl₂, IrBr₂, IrI₂, and/or the like), a nickel halide (for example, NiF₂, NiCl₂, NiBr₂, NiI₂, and/or the like), a palladium halide (for example, PdF₂, PdCl₂, PdBr₂, PdI₂, and/or the like), a platinum halide (for example, PtF₂, PtCl₂, PtBr₂, PtI₂, and/or the like), a copper(I) halide (for example, CuF, CuCl, CuBr, Cul, and/or the like), a silver halide (for example, AgF, AgCl, AgBr, Agl, and/or the like), a gold halide (for example, AuF, AuCl, AuBr, Aul, and/or the like), and/or the like.

Non-limiting examples of the post-transition metal halide may comprise a zinc halide (for example, ZnF₂, ZnCl₂, ZnBr₂, ZnI₂, and/or the like), an indium halide (for example, InI₃, and/or the like), a tin halide (for example, SnI₂, and/or the like), and/or the like.

Non-limiting examples of the lanthanide metal halide may comprise YbF, YbF₂, YbF₃, SmF₃, YbCl, YbCl₂, YbCl₃, SmCl₃, YbBr, YbBr₂, YbBr₃, SmBr₃, Ybl, YbI₂, YbI₃, SmI₃, and/or the like.

Non-limiting examples of the metalloid halide may comprise an antimony halide (for example, SbCl₅, and/or the like) and/or the like.

Non-limiting examples of the metal telluride may comprise an alkali metal telluride (for example, Li₂Te, Na₂Te, K₂Te, Rb₂Te, Cs₂Te, and/or the like), an alkaline earth metal telluride (for example, BeTe, MgTe, CaTe, SrTe, BaTe, and/or the like), a transition metal telluride (for example, TiTe₂, ZrTe₂, HfTe₂, V₂Te₃, Nb₂Te₃, Ta₂Te₃, Cr₂Te₃, Mo₂Te₃, W₂Te₃, MnTe, TcTe, ReTe, FeTe, RuTe, OsTe, CoTe, RhTe, IrTe, NiTe, PdTe, PtTe, Cu₂Te, CuTe, Ag₂Te, AgTe, Au₂Te, and/or the like), a post-transition metal telluride (for example, ZnTe, and/or the like), a lanthanide metal telluride (for example, LaTe, CeTe, PrTe, NdTe, PmTe, EuTe, GdTe, TbTe, DyTe, HoTe, ErTe, TmTe, YbTe, LuTe, and/or the like), and/or the like.

### Emission layer 130

When the light-emitting device 10 is a full-color light-emitting device, the emission layer 130 may be patterned into a red emission layer, a green emission layer, and/or a blue emission layer, according to a subpixel. In one or more embodiments, the emission layer 130 may have a stacked structure of two or more layers selected from among a red emission layer, a green emission layer, and a blue emission layer, in which the two or more layers contact each other or are separated from each other, to emit white light (e.g., combined white light). In one or more embodiments, the emission layer 130 may comprise two or more materials selected from among a red light-emitting material, a green light-emitting material, and a blue light-emitting material, in which the two or more materials are mixed with each other in a single layer, to emit white light (e.g., combined white light).

In one or more embodiments, the emission layer 130 may comprise a host and a dopant. The dopant may comprise a phosphorescent dopant, a fluorescent dopant, or any combination thereof.

An amount of the dopant in the emission layer 130 may be in a range of about 0.01 parts by weight to about 15 parts by weight based on 100 parts by weight of the host.

In one or more embodiments, the emission layer 130 may comprise a quantum dot.

In one or more embodiments, the emission layer 130 may comprise a delayed fluorescence material. The delayed fluorescence material may act as a host or a dopant in the emission layer 130.

A thickness of the emission layer 130 may be in a range of about 100 Å to about 1,000 Å, for example, about 200 Å to about 600 Å. When the thickness of the emission layer 130 is within this range, excellent or suitable luminescence characteristics may be obtained without a substantial increase in driving voltage.

### Host

In one or more embodiments, the host may comprise a compound represented by Formula 301:

**Formula 301** **[Ar₃₀₁]_{xb11}-[(L₃₀₁)_{xb1}-R₃₀₁]_{xb21},**

wherein, in Formula 301,
Ar₃₀₁ and L₃₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xb11 may be 1, 2, or 3,
xb1 may be an integer from 0 to 5,
R₃₀₁ may be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), -S(=O)₂(Q₃₀₁), or - P(=O)(Q₃₀₁)(Q₃₀₂),
xb21 may be an integer from 1 to 5, and
Q₃₀₁ to Q₃₀₃ may each be the same as described with respect to Q₁.

In one or more embodiments, if (e.g., when) xb11 in Formula 301 is 2 or greater, two or more of Ar₃₀₁(s) may be linked to each other via a single bond.

In one or more embodiments, the host may comprise a compound represented by Formula 301-1, a compound represented by Formula 301-2, or any combination thereof: 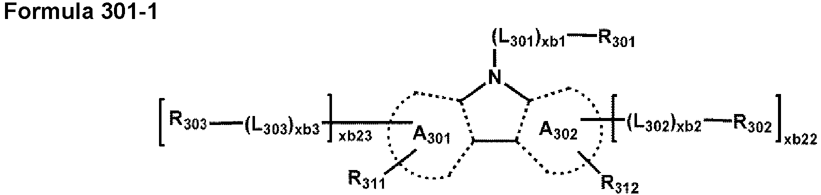 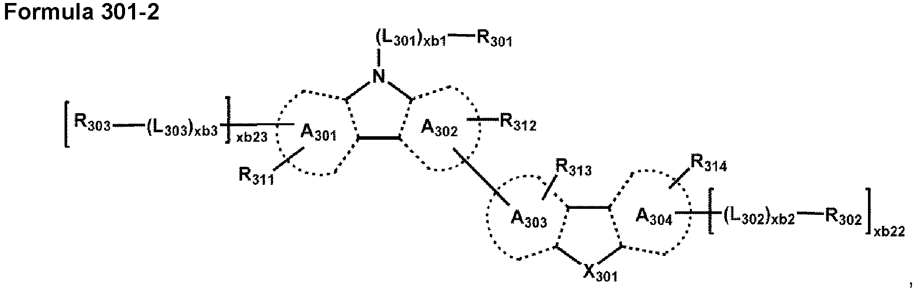
wherein, in Formulae 301-1 and 301-2,
ring A₃₀₁ to ring A₃₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
X₃₀₁ may be O, S, N[(L₃₀₄)_{xb4}-R₃₀₄], C(R₃₀₄)(R₃₀₅), or Si(R₃₀₄)(R₃₀₅),
xb22 and xb23 may each independently be 0, 1, or 2,
L₃₀₁, xb1, and R₃₀₁ may each be the same as described herein,
L₃₀₂ to L₃₀₄ may each independently be the same as described with respect to L₃₀₁,
xb2 to xb4 may each independently be the same as described with respect to xb1, and
R₃₀₂ to R₃₀₅ and R₃₁₁ to R₃₁₄ may each be the same as described with respect to R₃₀₁.

In one or more embodiments, the host may comprise an alkaline earth metal complex, a post-transition metal complex, or any combination thereof. For example, in one or more embodiments, the host may comprise a Be complex (for example, Compound H55), a Mg complex, a Zn complex, or any combination thereof.

In one or more embodiments, the host may comprise: one of (e.g., comprise at least one or be any one selected from among) Compounds H1 to H130; 9,10-di(2-naphthyl)anthracene (ADN); 2-methyl-9,10-bis(naphthalen-2-yl)anthracene (MADN); 9,10-di-(2-naphthyl)-2-t-butyl-anthracene (TBADN); 4,4'-bis(N-carbazolyl)-1,1'-biphenyl (CBP); 1,3-di(carbazol-9-yl)benzene (mCP); 1,3,5-tri(carbazol-9-yl)benzene (TCP); or any combination thereof:

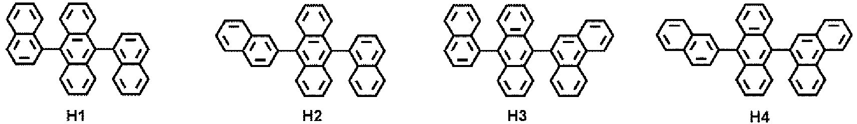

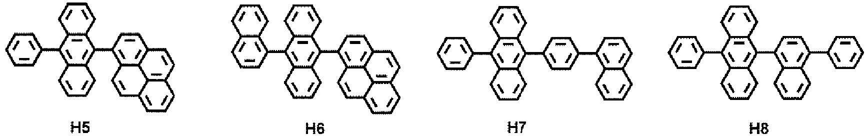

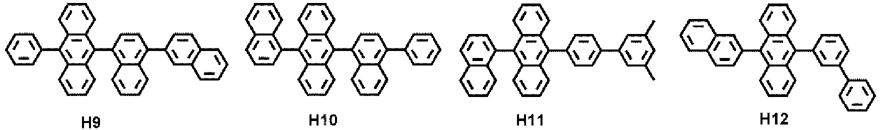

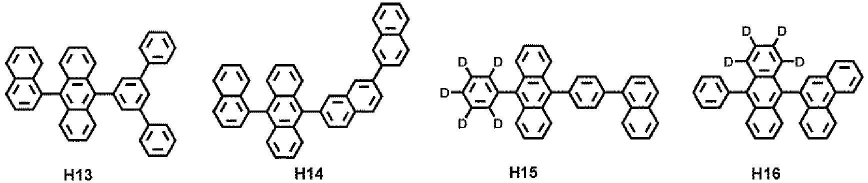

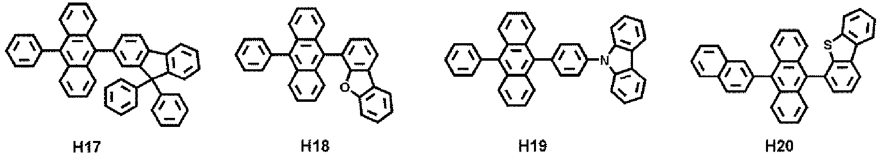

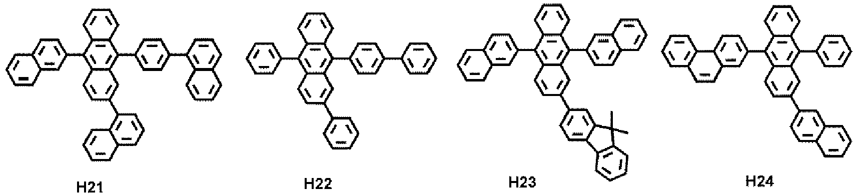

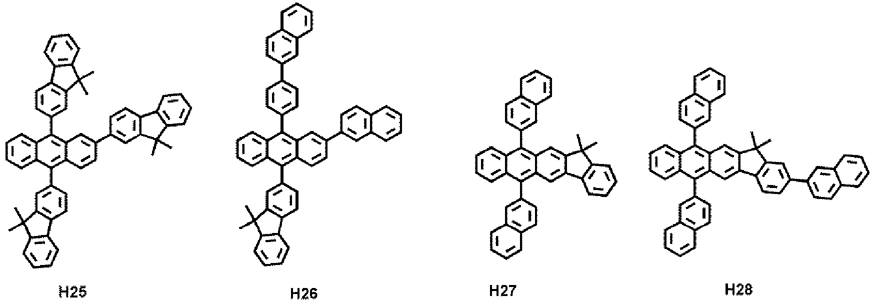

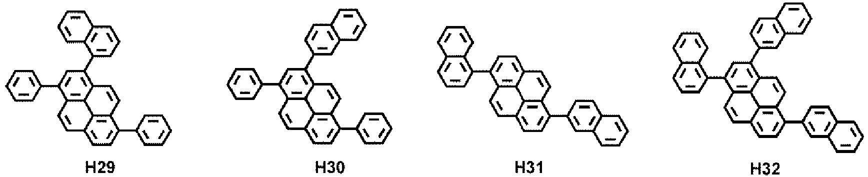

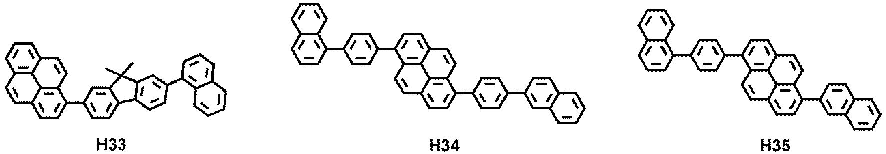

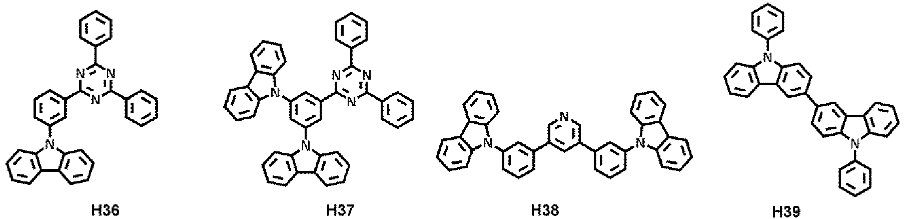

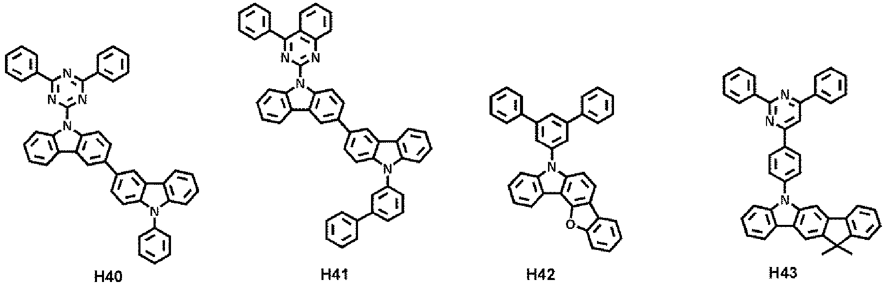

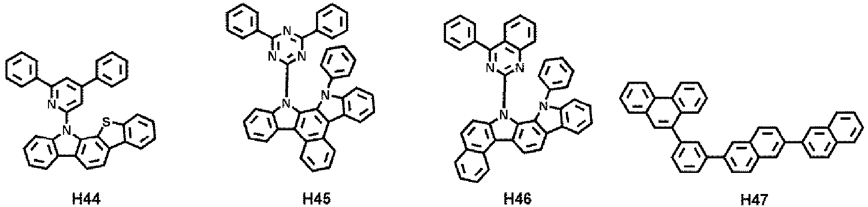

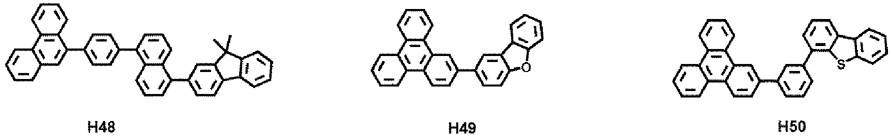

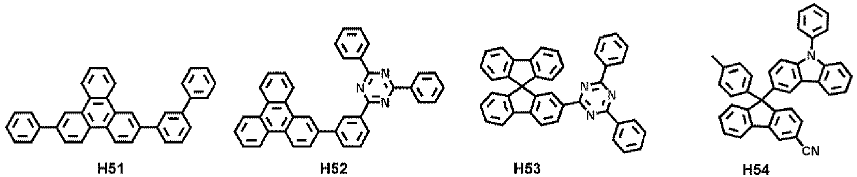

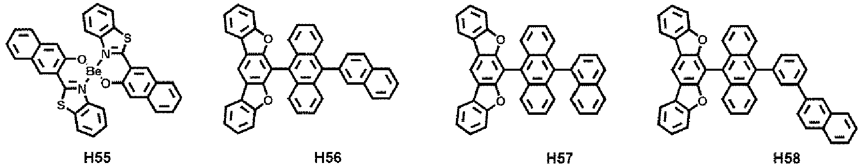

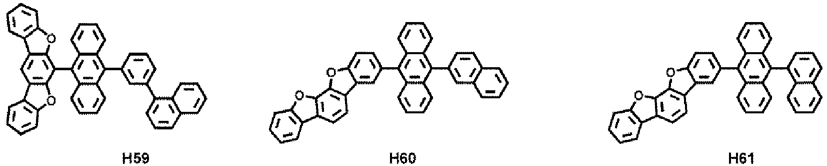

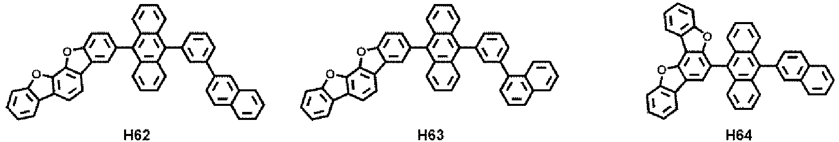

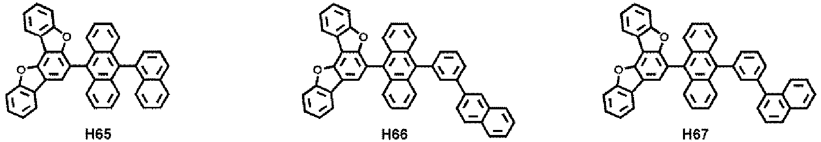

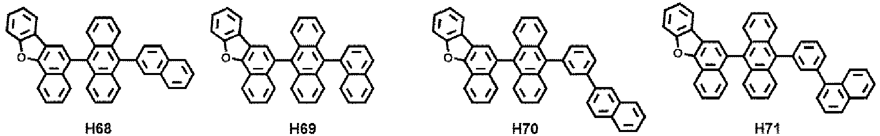

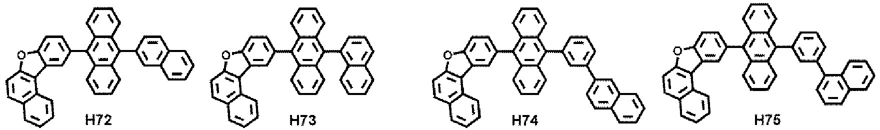

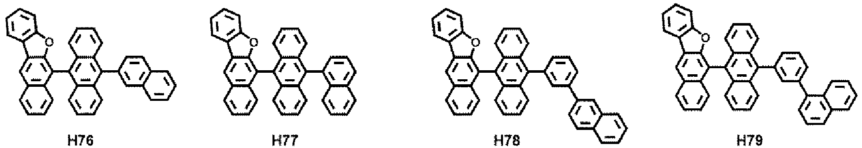

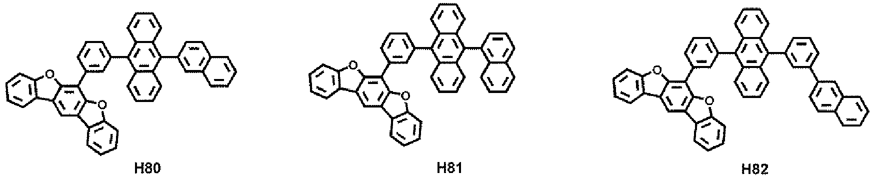

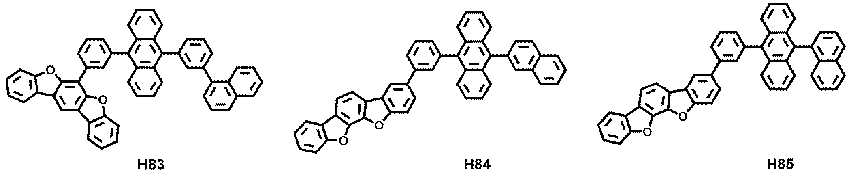

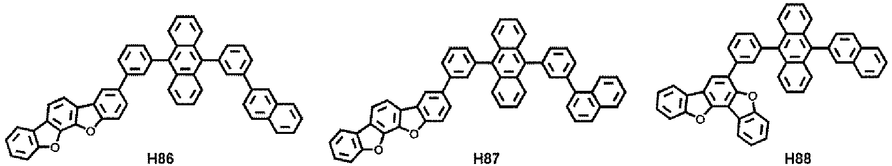

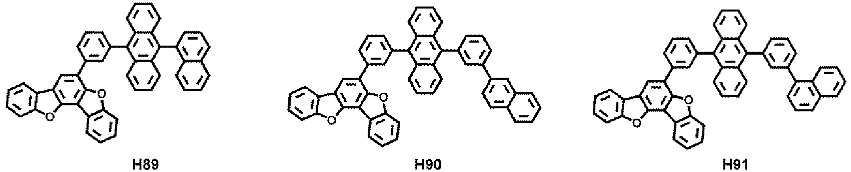

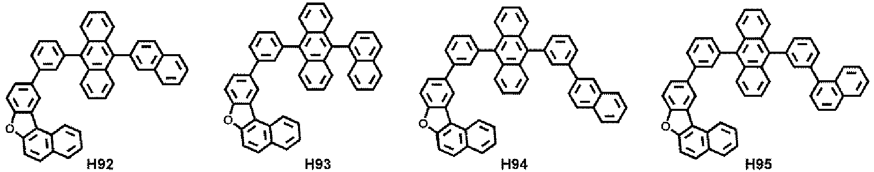

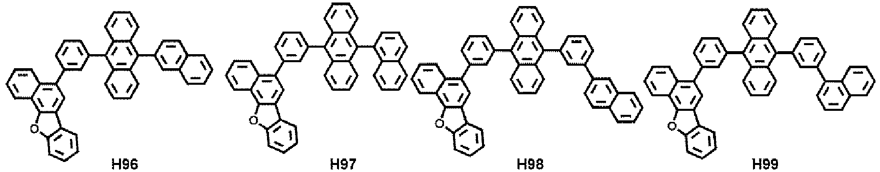

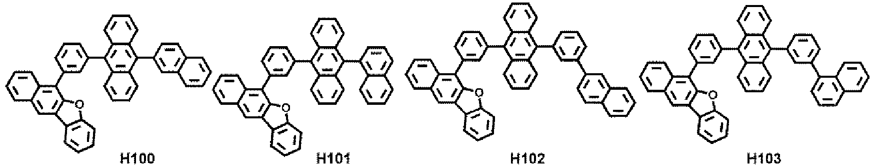

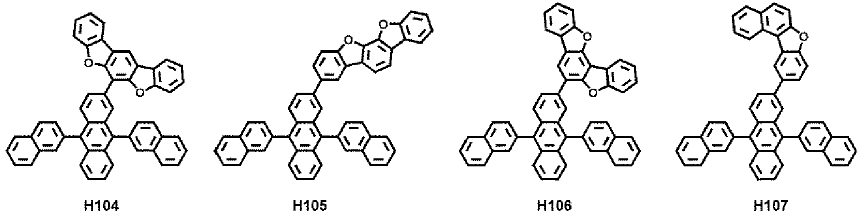

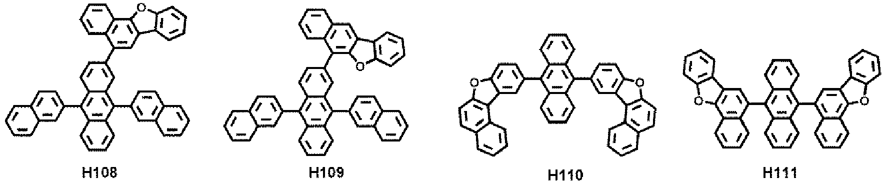

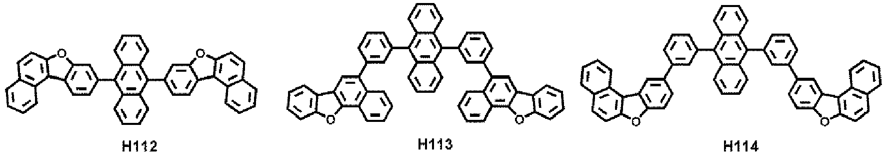

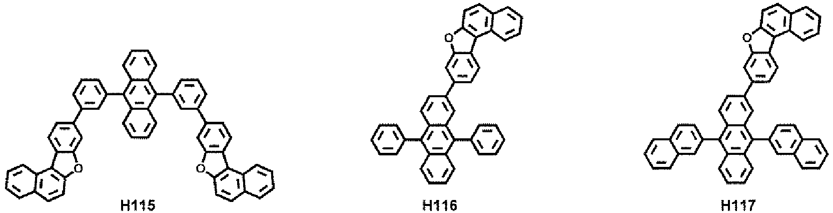

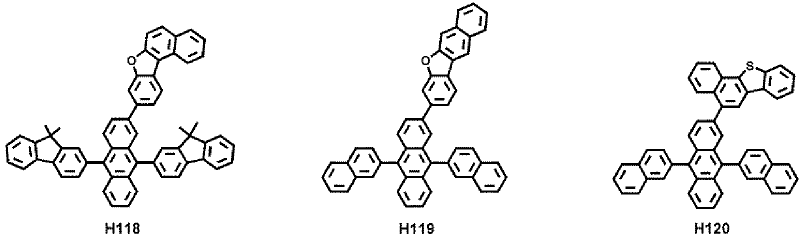

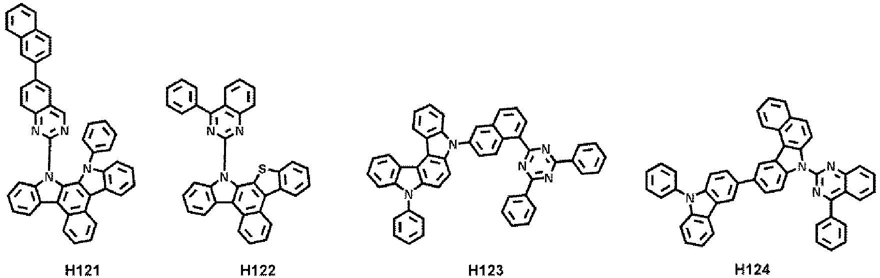

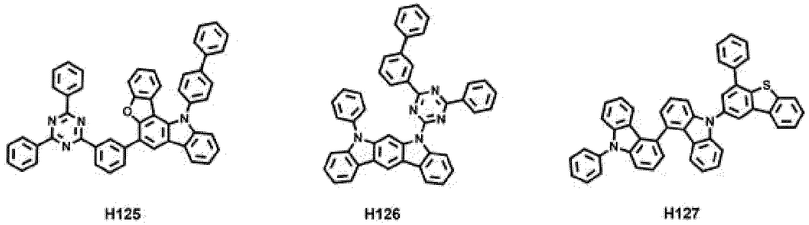

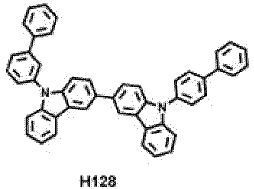

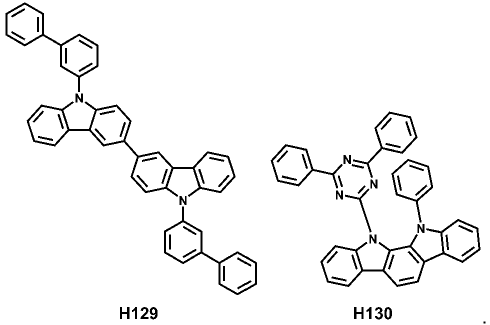

### Phosphorescent dopant

The phosphorescent dopant may comprise at least one transition metal as a central metal.

The phosphorescent dopant may comprise a monodentate ligand, a bidentate ligand, a tridentate ligand, a tetradentate ligand, a pentadentate ligand, a hexadentate ligand, or any combination thereof.

The phosphorescent dopant may be electrically neutral.

In one or more embodiments, the phosphorescent dopant may comprise an organometallic compound represented by Formula 401:

**Formula 401** **M(L₄₀₁)_{xc1}(L₄₀₂)_{xc2}**

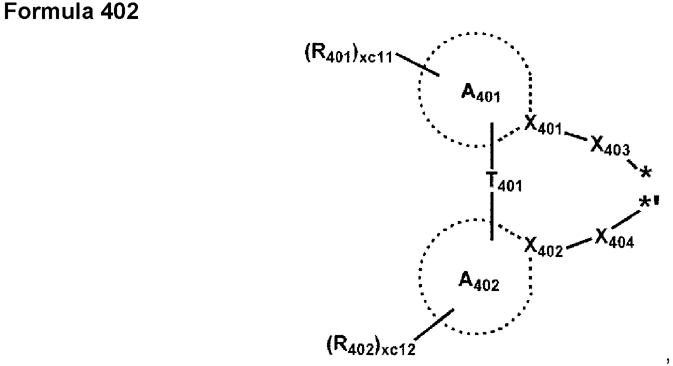

wherein, in Formulae 401 and 402,
M may be a transition metal (for example, iridium (Ir), platinum (Pt), palladium (Pd), osmium (Os), titanium (Ti), gold (Au), hafnium (Hf), europium (Eu), terbium (Tb), rhodium (Rh), rhenium (Re), or thulium (Tm)),
L₄₀₁ may be a ligand represented by Formula 402, and xc1 may be 1, 2, or 3, wherein, if (e.g., when) xc1 is 2 or greater, two or more of L₄₀₁(s) may be identical to or different from each other,
L₄₀₂ may be an organic ligand, and xc2 may be 0, 1, 2, 3, or 4, wherein, if (e.g., when) xc2 is 2 or greater, two or more of L₄₀₂(s) may be identical to or different from each other,
X₄₀₁ and X₄₀₂ may each independently be nitrogen or carbon,
ring A₄₀₁ and ring A₄₀₂ may each independently be a C₃-C₆₀ carbocyclic group or a C₁-C₆₀ heterocyclic group,
T₄₀₁ may be a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or *=C=*',
X₄₀₃ and X₄₀₄ may each independently be a chemical bond (for example, a covalent bond or a coordinate bond), O, S, N(Q₄₁₃), B(Q₄₁₃), P(Q₄₁₃), C(Q₄₁₃)(Q₄₁₄), or Si(Q₄₁₃)(Q₄₁₄),
Q₄₁₁ to Q₄₁₄ may each be the same as described with respect to Q₁,
R₄₀₁ and R₄₀₂ may each independently be hydrogen, deuterium, -F, -Cl, -Br, -I, a hydroxyl group, a cyano group, a nitro group, a C₁-C₂₀ alkyl group unsubstituted or substituted with at least one R₁₀ₐ, a C₁-C₂₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₄₀₁)(Q₄₀₂)(Q₄₀₃), - N(Q₄₀₁)(Q₄₀₂), -B(Q₄₀₁)(Q₄₀₂), -C(=O)(Q₄₀₁), -S(=O)₂(Q₄₀₁), or -P(=O)(Q₄₀₁)(Q₄₀₂),
Q₄₀₁ to Q₄₀₃ may each be the same as described with respect to Q₁,
xc11 and xc12 may each independently be an integer from 0 to 10, and
* and *' in Formula 402 may each indicate a bonding site to M in Formula 401.

In one or more embodiments, in Formula 402, i) X₄₀₁ may be nitrogen, and X₄₀₂ may be carbon, or ii) each of X₄₀₁ and X₄₀₂ may be nitrogen.

In one or more embodiments, if (e.g., when) xc1 in Formula 401 is 2 or greater, two rings A₄₀₁(s) among two or more of L₄₀₁(s) may optionally be linked to each other via T₄₀₂, which is a linking group, and/or two rings A₄₀₂(s) among two or more of L₄₀₁(s) may optionally be linked to each other via T₄₀₃, which is a linking group (see Compounds PD1 to PD4 and PD7). T₄₀₂ and T₄₀₃ may each be the same as described with respect to T₄₀₁.

L₄₀₂ in Formula 401 may be an organic ligand. For example, in one or more embodiments, L₄₀₂ may comprise a halogen, a diketone group (for example, an acetylacetonate group), a carboxylic acid group (for example, a picolinate group), -C(=O), an isonitrile group, a -CN group, a phosphorus-containing group (for example, a phosphine group, a phosphite group, and/or the like), or any combination thereof.

The phosphorescent dopant may comprise, for example, one of (e.g., comprise at least one or be any one selected from among) Compounds PD1 to PD39, Compound D1, or any combination thereof:

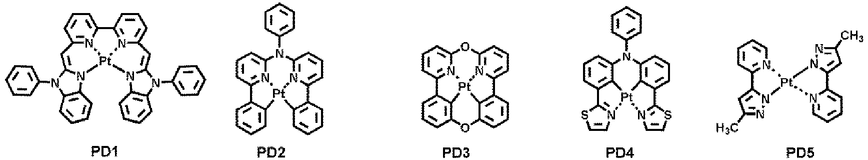

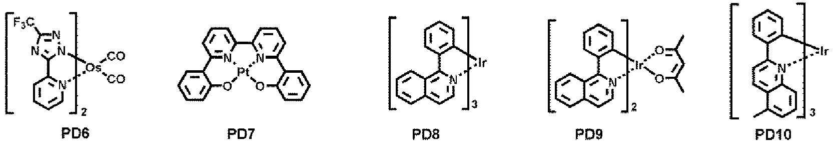

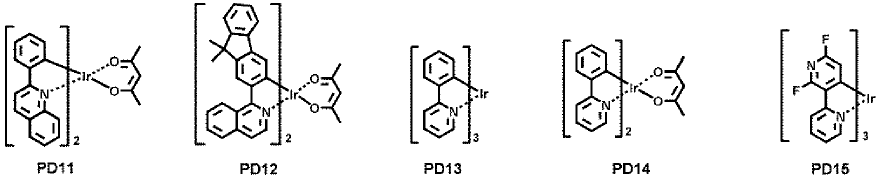

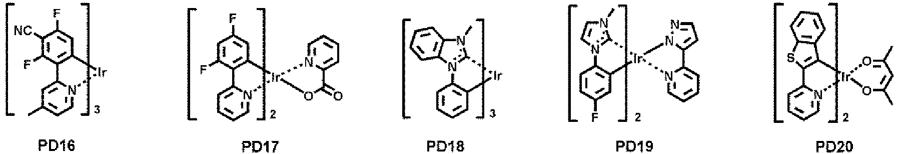

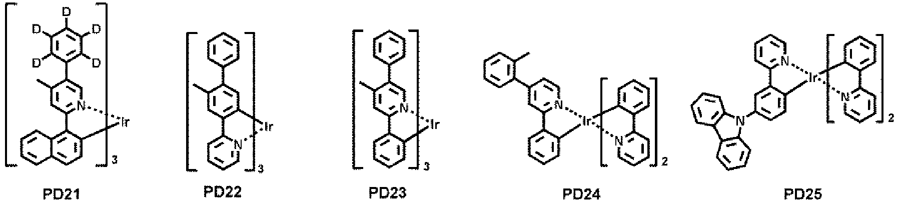

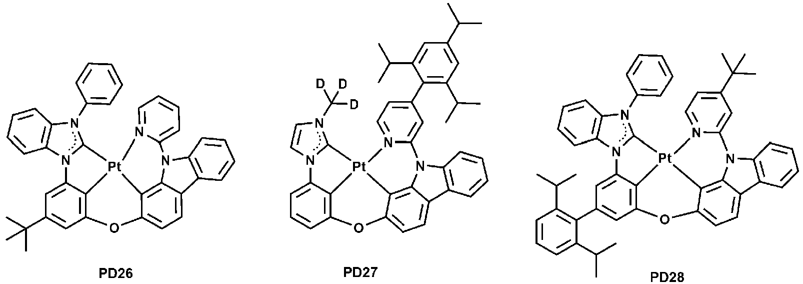

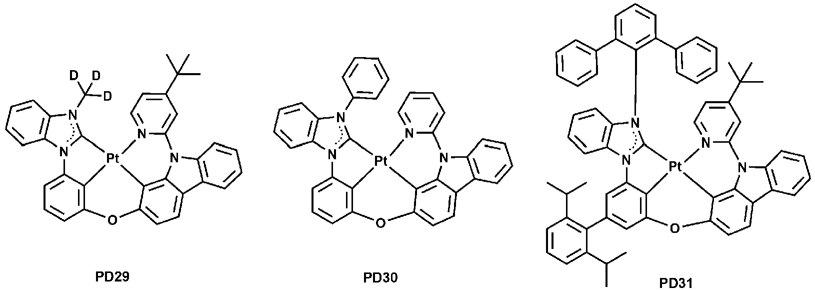

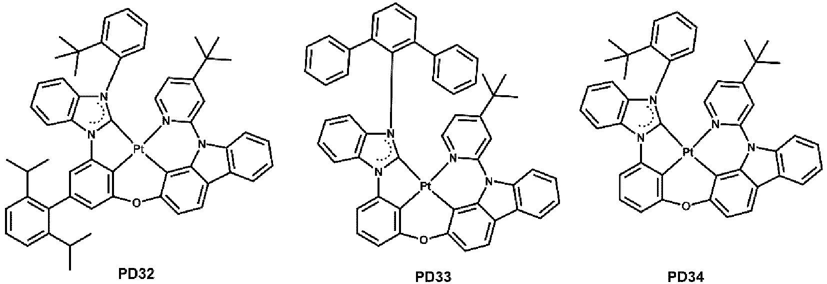

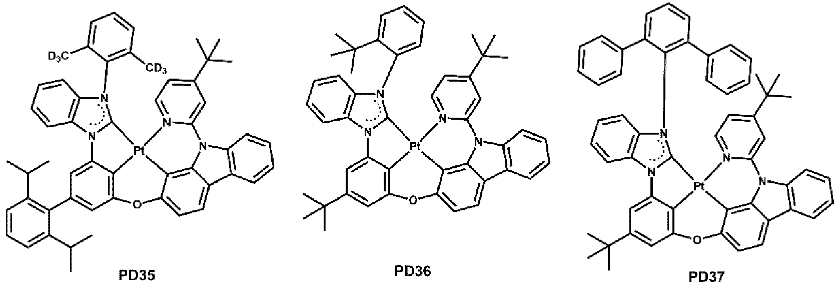

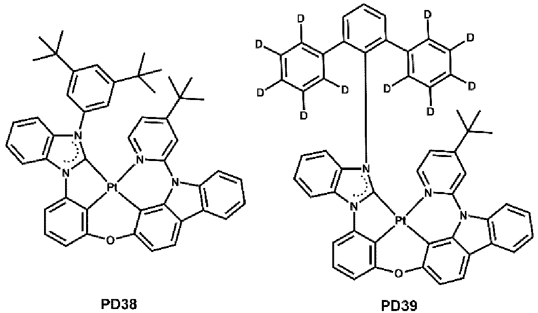

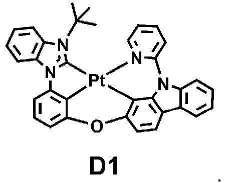

### Fluorescent dopant

The fluorescent dopant may comprise an amine group-containing compound, a styryl group-containing compound, or any combination thereof.

In one or more embodiments, the fluorescent dopant may comprise a compound represented by Formula 501: 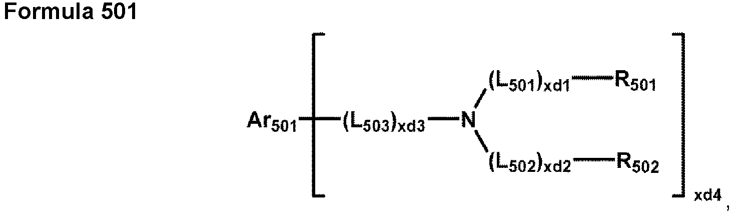
wherein, in Formula 501,
Ar₅₀₁, L₅₀₁ to L₅₀₃, R₅₀₁, and R₅₀₂ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xd1 to xd3 may each independently be 0, 1, 2, or 3, and
xd4 may be 1, 2, 3, 4, 5, or 6.

In one or more embodiments, Ar₅₀₁, in Formula 501 may be a condensed cyclic group (for example, an anthracene group, a chrysene group, a pyrene group, and/or the like) in which three or more monocyclic groups are condensed with each other.

In one or more embodiments, xd4 in Formula 501 may be 2.

In one or more embodiments, the fluorescent dopant may comprise: one of (e.g., comprise at least one or be any one selected from among) Compounds FD1 to FD37; 4,4'-bis(2,2-diphenylvinyl)-1,1'-biphenyl (DPVBi); 4,4'-bis[4-(N,N-diphenylamino)styryl]biphenyl (DPAVBi); or any combination thereof:

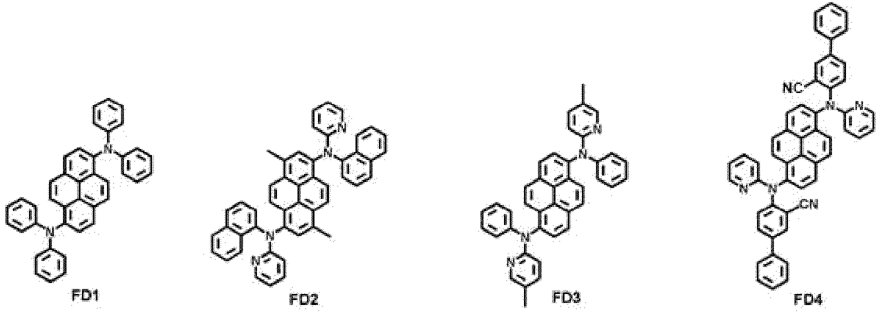

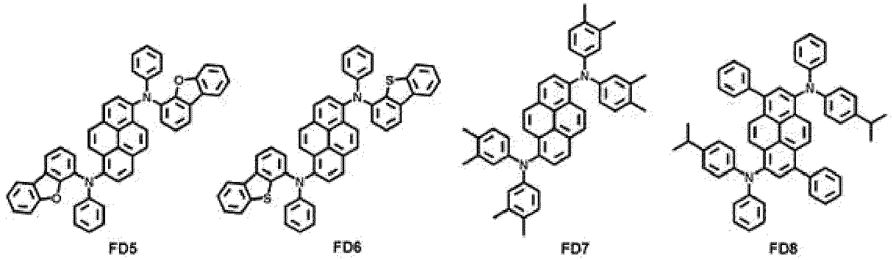

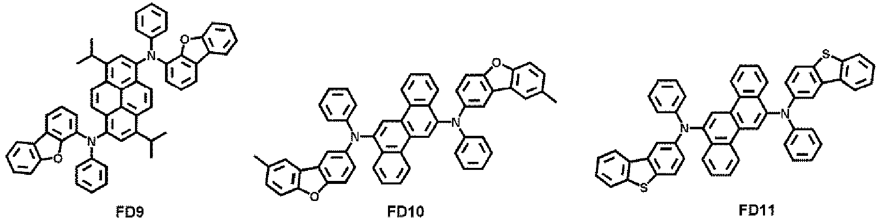

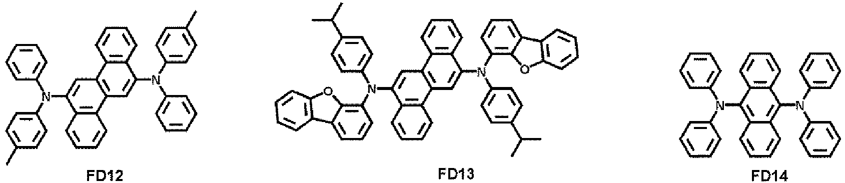

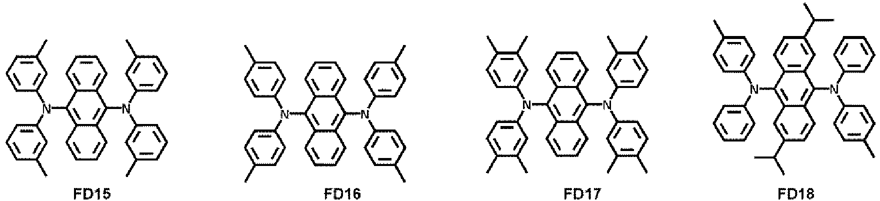

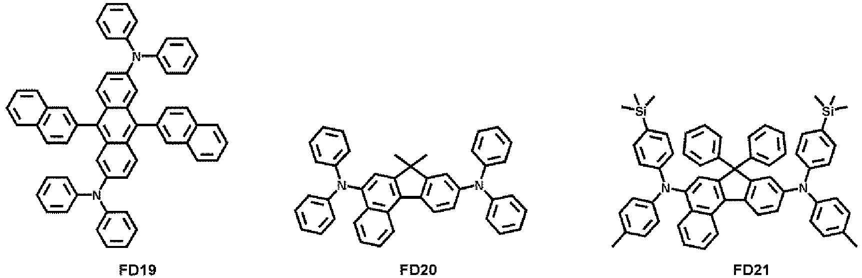

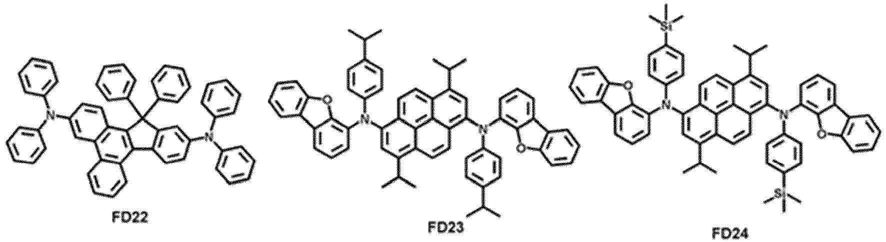

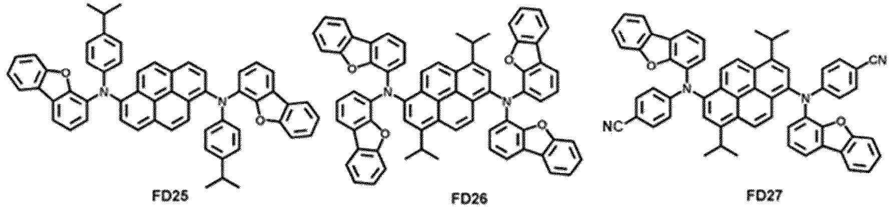

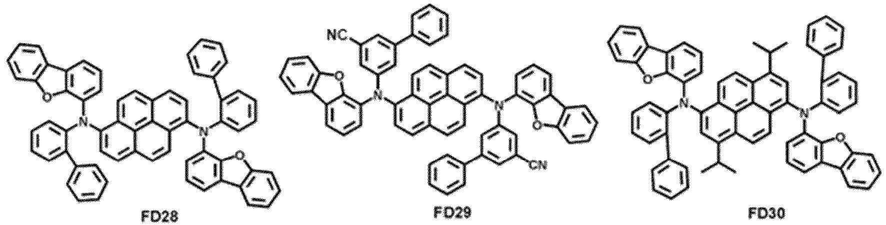

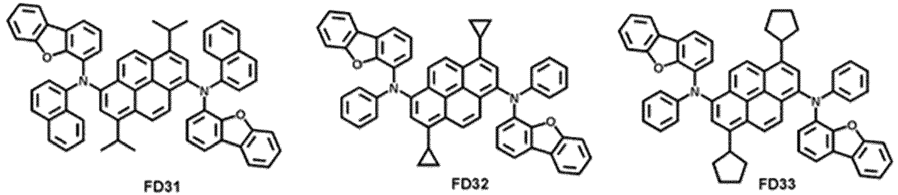

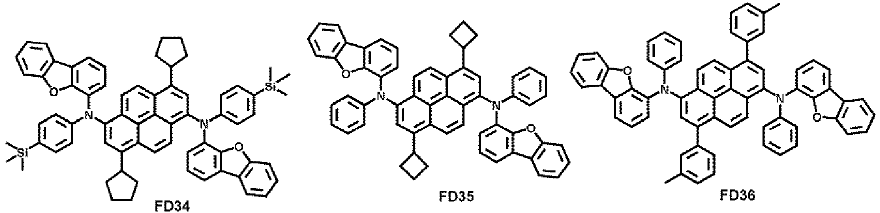

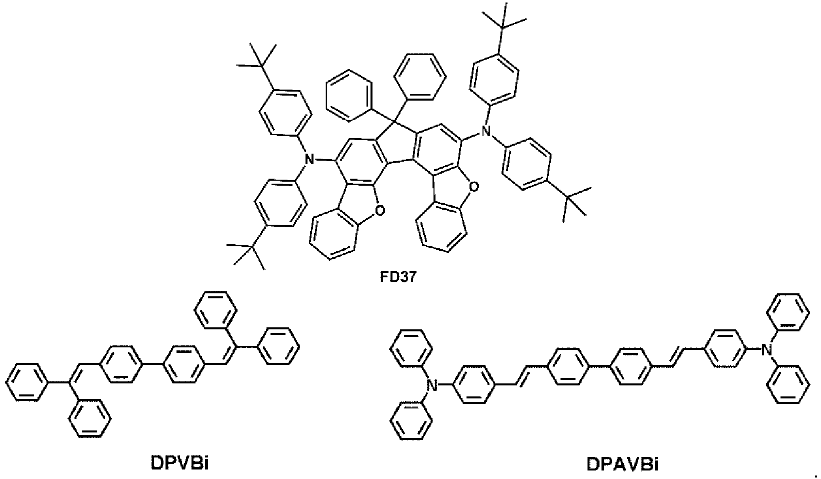

### Delayed fluorescence material

In one or more embodiments, the emission layer 130 may comprise a delayed fluorescence material.

Herein, the delayed fluorescence material may be selected from among compounds capable of emitting delayed fluorescence based on a delayed fluorescence emission mechanism.

The delayed fluorescence material included in the emission layer 130 may act as a host or a dopant, depending on the type (kind) of other materials included in the emission layer 130.

In one or more embodiments, a difference (e.g., an absolute value of the difference) between a triplet energy level (eV) of the delayed fluorescence material and a singlet energy level (eV) of the delayed fluorescence material may be in a range of about 0 eV to about 0.5 eV. When the difference between the triplet energy level (eV) of the delayed fluorescence material and the singlet energy level (eV) of the delayed fluorescence material is satisfied within the range above, up-conversion from the triplet state to the singlet state of the delayed fluorescence materials may effectively occur, and thus, the light-emitting device 10 may have improved luminescence efficiency.

In one or more embodiments, the delayed fluorescence material may comprise i) a material comprising at least one electron donor (for example, a π electron-rich C₃-C₆₀ cyclic group, such as a carbazole group, and/or the like) and at least one electron acceptor (for example, a sulfoxide group, a cyano group, a π electron-deficient nitrogen-containing C₁-C₆₀ cyclic group, and/or the like), ii) a material comprising a C₈-C₆₀ polycyclic group in which two or more cyclic groups are condensed while sharing boron (B), and/or iii) the like.

Non-limiting examples of the delayed fluorescence material may comprise at least one of (e.g., selected from among) Compounds DF1 to DF14:

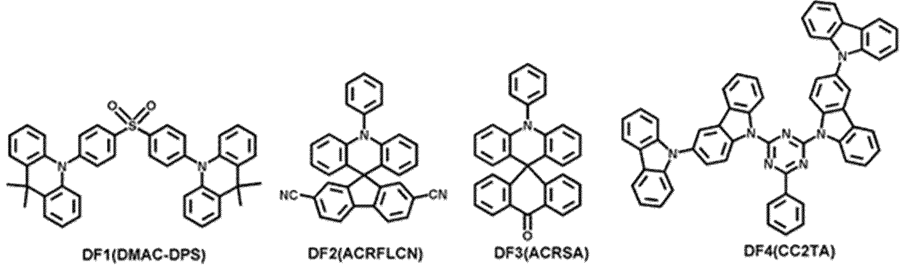

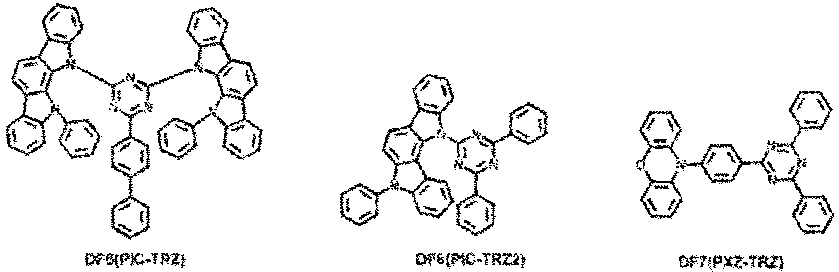

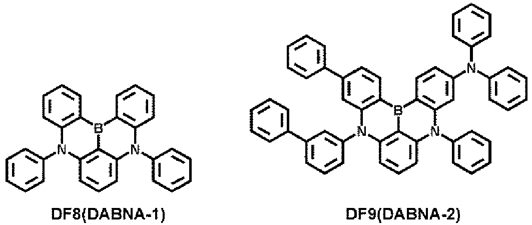

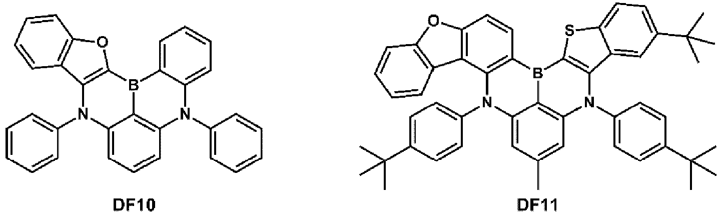

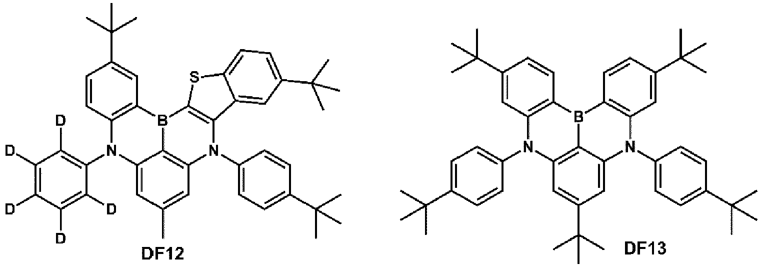

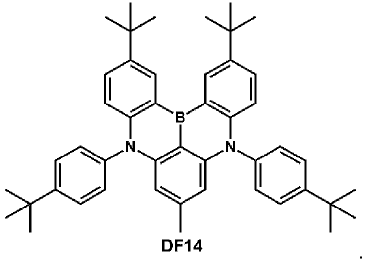

### Quantum dots

In one or more embodiments, the emission layer 130 may comprise quantum dots.

The term "quantum dot" as used herein refers to a crystal of a semiconductor compound. Quantum dots may be to emit light of one or more suitable emission wavelengths depending on the size of crystals. Quantum dots may be to emit light of one or more suitable emission wavelengths by further adjusting the ratio of elements constituting the quantum dots.

A diameter of the quantum dots may be, for example, in a range of about 1 nanometer (nm) to about 10 nm. In the present disclosure, when quantum dots or quantum dot particles are spherical, "diameter" indicates a particle diameter or an average particle diameter, and when the particles are non-spherical, the "diameter" indicates a major axis length or an average major axis length. The diameter of the particles may be measured utilizing a scanning electron microscope or a particle size analyzer. As the particle size analyzer, for example, HORIBA, LA-950 laser particle size analyzer, may be utilized. When the size of the particles is measured utilizing a particle size analyzer, the average particle diameter is referred to as D50. D50 refers to the average diameter of particles whose cumulative volume corresponds to 50 vol% in the particle size distribution (e.g., cumulative distribution), and refers to the value of the particle size corresponding to 50% from the smallest particle when the total number of particles is 100% in the distribution curve accumulated in the order of the smallest particle size to the largest particle size.

The quantum dots may be synthesized by a wet chemical process, a metal organic chemical vapor deposition process, a molecular beam epitaxy process, or any process similar thereto.

The wet chemical process is a method comprising mixing a precursor material of a quantum dot with an organic solvent and then growing quantum dot particle crystals. When the crystals grow, the organic solvent naturally acts as a dispersant coordinated on the surface of the quantum dot crystals and controls the growth of the crystals so that the growth of quantum dot particles may be controlled or selected through a process which costs lower and is easier than vapor deposition methods, such as metal organic chemical vapor deposition (MOCVD) or molecular beam epitaxy (MBE).

The quantum dots may comprise: a Group II-VI semiconductor compound; a Group III-V semiconductor compound; a Group III-VI semiconductor compound; a Group I-III-VI semiconductor compound; a Group IV-VI semiconductor compound; a Group IV element or compound; or any combination thereof.

Non-limiting examples of the Group II-VI semiconductor compound may comprise: a binary compound, such as CdS, CdSe, CdTe, ZnS, ZnSe, ZnTe, ZnO, HgS, HgSe, HgTe, MgSe, and/or MgS; a ternary compound, such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, and/or MgZnS; a quaternary compound, such as CdZnSeS, CdZnSeTe, CdZnSTe, CdHgSeS, CdHgSeTe, CdHgSTe, HgZnSeS, HgZnSeTe, and/or HgZnSTe; or any combination thereof.

Non-limiting examples of the Group III-V semiconductor compound may comprise: a binary compound, such as GaN, GaP, GaAs, GaSb, AIN, AlP, AlAs, AlSb, InN, InP, InAs, and/or InSb; a ternary compound, such as GaNP, GaNAs, GaNSb, GaPAs, GaPSb, AINP, AlNAs, AlNSb, AlPAs, AlPSb, InGaP, InNP, InAlP, InNAs, InNSb, InPAs, and/or InPSb; a quaternary compound, such as GaAlNP, GaAINAs, GaAINSb, GaAlPAs, GaAlPSb, GalnNP, GalnNAs, GaInNSb, GalnPAs, GalnPSb, InAlNP, InAlNAs, InAlNSb, InAlPAs, and/or InAlPSb; or any combination thereof. In one or more embodiments, the Group III-V semiconductor compound may further comprise a Group II element. Non-limiting examples of the Group III-V semiconductor compound further comprising a Group II element may comprise InZnP, InGaZnP, InAlZnP, and/or the like.

Non-limiting examples of the Group III-VI semiconductor compound may comprise: a binary compound, such as GaS, Ga₂S₃, GaSe, Ga₂Se₃, GaTe, InS, InSe, In₂S₃, In₂Se₃, and/or InTe; a ternary compound, such as InGaS₃ and/or InGaSe₃; or any combination thereof.

Non-limiting examples of the Group I-III-VI semiconductor compound may comprise: a ternary compound, such as AgInS, AgInS₂, AgInSe₂, AgGaS, AgGaS₂, AgGaSe₂, CulnS, CuInS₂, CuInSe₂, CuGaS₂, CuGaSe₂, CuGaO₂, AgGaO₂, and/or AgAlO₂; a quaternary compound, such as CulnGaS, CuInGaS₂, AgInGaS, AgInGaS₂, AgInGaSe, and/or AgInGaSe₂; or any combination thereof.

Non-limiting examples of the Group IV-VI semiconductor compound may comprise: a binary compound, such as SnS, SnSe, SnTe, PbS, PbSe, and/or PbTe; a ternary compound, such as SnSeS, SnSeTe, SnSTe, PbSeS, PbSeTe, PbSTe, SnPbS, SnPbSe, and/or SnPbTe; a quaternary compound, such as SnPbSSe, SnPbSeTe, and/or SnPbSTe; or any combination thereof.

Non-limiting examples of the Group IV element or compound may comprise: a single element, such as Si and/or Ge; a binary compound, such as SiC and/or SiGe; or any combination thereof.

Each element included in a multi-component compound, such as the binary compound, the ternary compound, and the quaternary compound, may be present at a substantially uniform concentration or non-uniform concentration in a particle. For example, the formulae above refer to the types (kinds) of elements included in a compound, wherein the element ratios in the compound may vary. For example, AgInGaS₂ may indicate AgInₓGa₁₋ₓS₂ (wherein x is a real number between 0 and 1).

In one or more embodiments, the quantum dots may each have a single structure in which the concentration of each element in the quantum dot is substantially uniform, or a core-shell dual structure. For example, a material comprised in the core and a material comprised in the shell may be different from each other.

The shell of the quantum dot may act as a protective layer that prevents chemical degeneration of the core to maintain semiconductor characteristics, and/or as a charging layer that imparts electrophoretic characteristics to the quantum dots. The shell may be a single layer or a multi-layer. In one or more embodiments, the interface between the core and the shell may have a concentration gradient in which the concentration of an element existing in the shell decreases toward the center of the core.

Examples of the shell of the quantum dots may comprise an oxide of metal or non-metal, a semiconductor compound, or any combination thereof. Non-limiting examples of the oxide of metal or non-metal may comprise: a binary compound, such as SiO₂, Al₂O₃, TiO₂, ZnO, MnO, Mn₂O₃, Mn₃O₄, CuO, FeO, Fe₂O₃, Fe₃O₄, CoO, Co₃O₄, and/or NiO; a ternary compound, such as MgAl₂O₄, CoFe₂O₄, NiFe₂O₄, and/or CoMn₂O₄; or any combination thereof. Examples of the semiconductor compound may comprise: a Group III-VI semiconductor compound; a Group II-VI semiconductor compound; a Group III-V semiconductor compound; a Group I-III-VI semiconductor compound; a Group IV-VI semiconductor compound; or any combination thereof, as described herein. For example, the semiconductor compound suitable as a shell may comprise CdS, CdSe, CdTe, ZnS, ZnSe, ZnTe, ZnSeS, ZnTeS, GaAs, GaP, GaS, GaSe, AgGaS, AgGaS₂, GaSb, HgS, HgSe, HgTe, InAs, InP, InGaP, InSb, AlAs, AlP, AlSb, or any combination thereof.

The quantum dots may have a full width at half maximum (FWHM) of the emission spectrum of less than or equal to about 45 nm, less than or equal to about 40 nm, or, for example, less than or equal to about 30 nm. When the FWHM of the quantum dots is within these ranges, the quantum dots may have improved color purity or improved color reproducibility. In addition, because light emitted through the quantum dots is emitted in all directions, the wide viewing angle may be improved.

In addition, the quantum dots may be in the form of a spherical particle, a pyramidal particle, a multi-arm particle, a cubic nanoparticle, a nanotube particle, a nanowire particle, a nanofiber particle, a nanoplate particle, and/or the like.

Because the energy band gap of the quantum dots may be controlled or selected by adjusting the size of the quantum dots and/or the ratio of elements in the quantum dot compound, light of one or more suitable wavelengths may be obtained from a quantum dot-containing emission layer. Accordingly, by using the quantum dots described above (by using quantum dots of different sizes or by varying the ratio of elements in the quantum dot compound), a light-emitting device that emits light of one or more suitable wavelengths may be implemented. In one or more embodiments, the size of the quantum dots and/or the ratio of elements in the quantum dot compound may be controlled and selected to enable the quantum dots to emit red light, green light, and/or blue light. In addition, the quantum dots with suitable sizes may be configured to emit white light by combination of light of one or more suitable colors.

### Electron transport region 140

The electron transport region 140 may have i) a single-layer structure comprising (e.g., consisting of) a single layer comprising (e.g., consisting of) a single material, ii) a single-layer structure comprising (e.g., consisting of) a single layer comprising multiple materials that are different from each other, or iii) a multi-layer structure comprising multiple layers comprising multiple materials that are different from each other.

The electron transport region 140 may comprise a buffer layer, a hole-blocking layer, an electron control layer, an electron transport layer, an electron injection layer, or any combination thereof.

In one or more embodiments, the electron transport region 140 may have an electron transport layer/electron injection layer structure, a hole-blocking layer/electron transport layer/electron injection layer structure, an electron control layer/electron transport layer/electron injection layer structure, or a buffer layer/electron transport layer/electron injection layer structure, wherein constituent layers of each structure are stacked sequentially from the emission layer 130 in the stated order.

The electron transport region 140 (for example, the buffer layer, the hole-blocking layer, the electron control layer, or the electron transport layer in the electron transport region) may comprise a metal-free compound comprising at least one π electron-deficient nitrogen-containing C₁-C₆₀ cyclic group.

In one or more embodiments, the electron transport region 140 may comprise a compound represented by Formula 601.

Formula 601 [Ar₆₀₁]ₓₑ₁₁-[(L₆₀₁)ₓₑ₁-R₆₀₁]ₓₑ₂₁,

wherein, in Formula 601,
Ar₆₀₁ and L₆₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xe11 may be 1, 2, or 3,
xe1 may be 0, 1, 2, 3, 4, or 5,
R₆₀₁ may be a C₃-C₆₀ carbocyclic group unsubstituted or substituted with at least one R₁₀ₐ, a C₁-C₆₀ heterocyclic group unsubstituted or substituted with at least one R₁₀ₐ, - Si(Q₆₀₁)(Q₆₀₂)(Q₆₀₃), -C(=O)(Q₆₀₁), -S(=O)₂(Q₆₀₁), or -P(=O)(Q₆₀₁)(Q₆₀₂),
Q₆₀₁ to Q₆₀₃ may each be the same as described with respect to Q₁,
xe21 may be 1, 2, 3, 4, or 5, and
at least one selected from among Ar₆₀₁, L₆₀₁, and R₆₀₁ may each independently be a π electron-deficient nitrogen-containing C₁-C₆₀ cyclic group unsubstituted or substituted with at least one R₁₀ₐ.

In one or more embodiments, if (e.g., when) xe11 in Formula 601 is 2 or greater, two or more of Ar₆₀₁(s) may be linked to each other via a single bond.

In one or more embodiments, Ar₆₀₁ in Formula 601 may be an anthracene group unsubstituted or substituted with at least one R₁₀ₐ.

In one or more embodiments, the electron transport region 140 may comprise a compound represented by Formula 601-1: 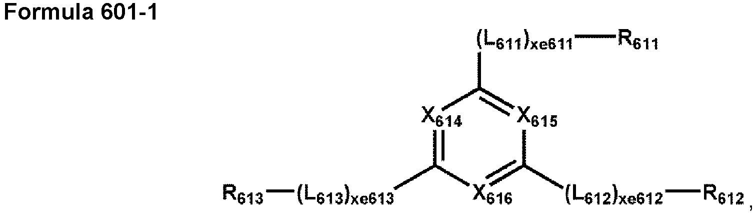
wherein, in Formula 601-1,
X₆₁₄ may be N or C(R₆₁₄), X₆₁₅ may be N or C(R₆₁₅), X₆₁₆ may be N or C(R₆₁₆), and at least one selected from among X₆₁₄ to X₆₁₆ may be N,
L₆₁₁ to L₆₁₃ may each be the same as described with respect to L₆₀₁,
xe611 to xe613 may each be the same as described with respect to xe1,
R₆₁₁ to R₆₁₃ may each be the same as described with respect to R₆₀₁, and
R₆₁₄ to R₆₁₆ may each independently be hydrogen, deuterium, -F, -Cl, -Br, -I, a hydroxyl group, a cyano group, a nitro group, a C₁-C₂₀ alkyl group, a C₁-C₂₀ alkoxy group, a C₃-C₆₀ carbocyclic group unsubstituted or substituted with at least one R₁₀ₐ, or a C₁-C₆₀ heterocyclic group unsubstituted or substituted with at least one R₁₀ₐ.

In one or more embodiments, xe1 and xe611 to xe613 in Formulae 601 and 601-1 may each independently be 0, 1, or 2.

In one or more embodiments, the electron transport region 140 may comprise: one of (e.g., comprise at least one or be any one selected from among) Compounds ET1 to ET46; 2,9-dimethyl-4,7-diphenyl-1,10-phenanthroline (BCP); 4,7-diphenyl-1,10-phenanthroline (Bphen); tris(8-hydroxyquinolinato)aluminum (Alq₃); bis(2-methyl-8-quinolinolato-N1,O8)-(1,1'-biphenyl-4-olato)aluminum (BAlq); 3-(4-biphenyl)-4-phenyl-5-tert-butylphenyl-1,2,4-triazole (TAZ); 4-(naphthalen-1-yl)-3,5-diphenyl-4H-1,2,4-triazole (NTAZ); or any combination thereof:

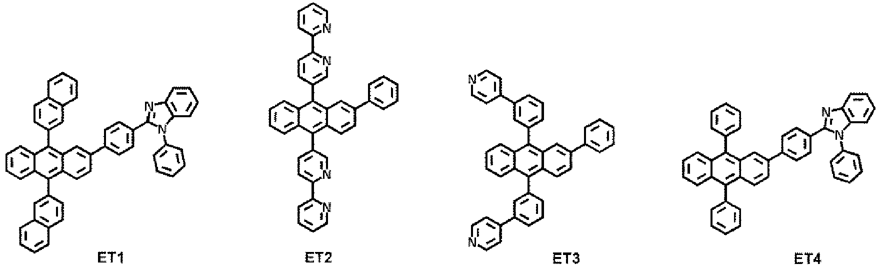

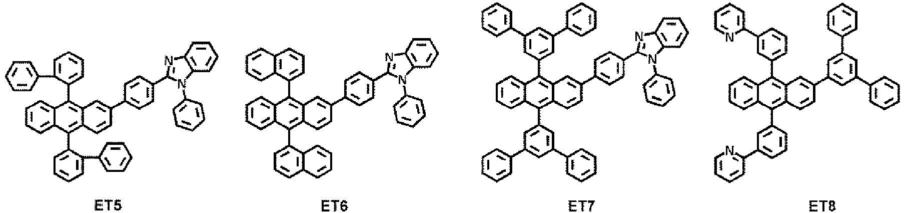

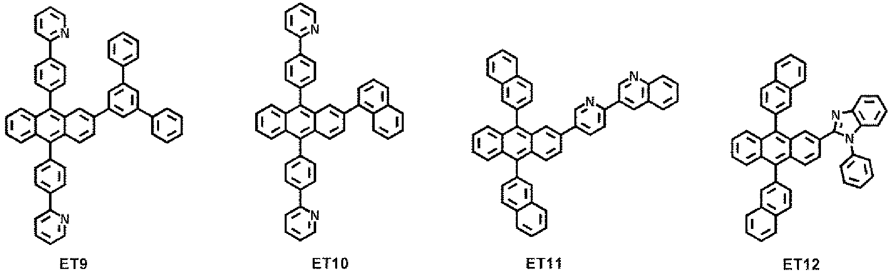

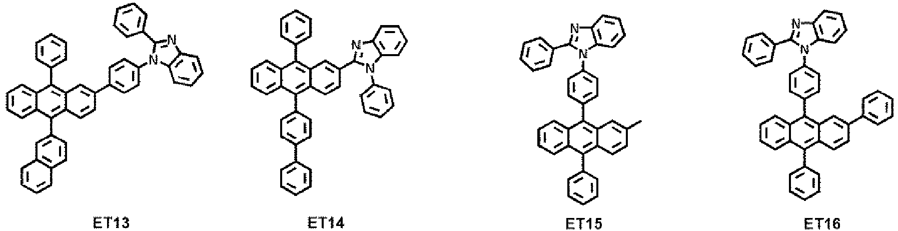

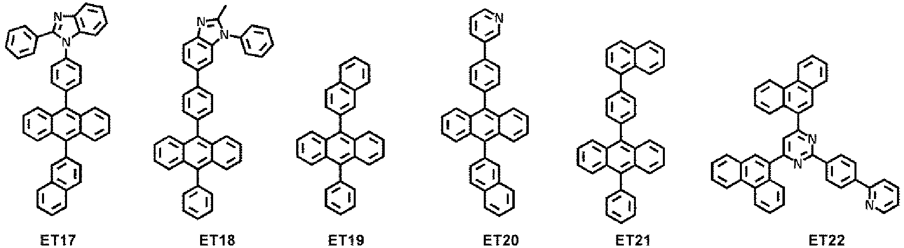

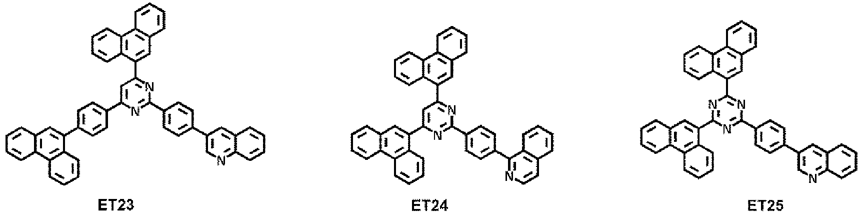

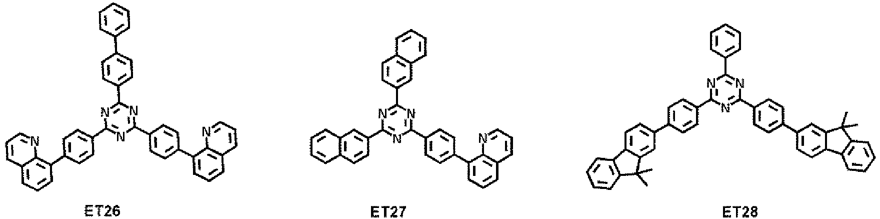

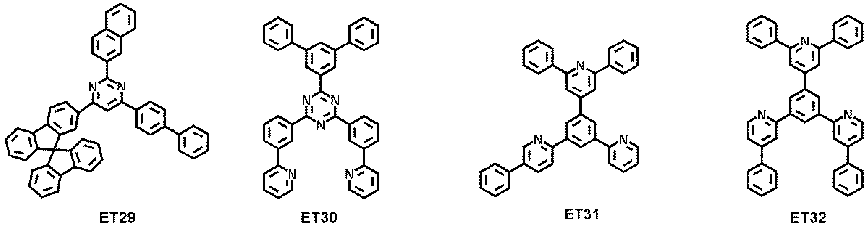

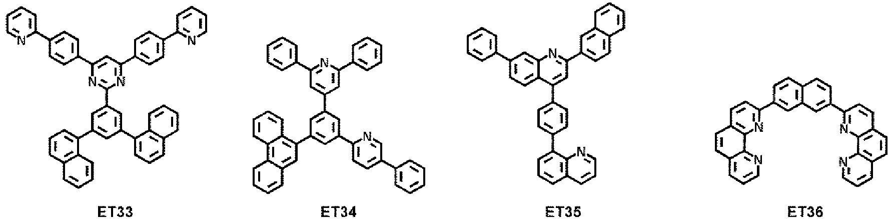

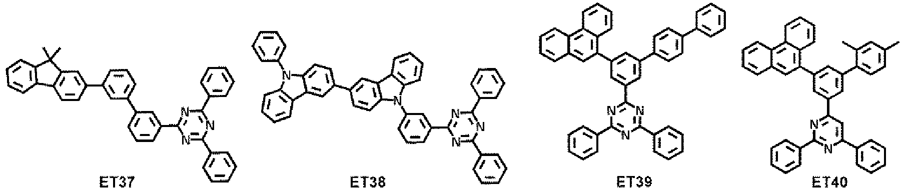

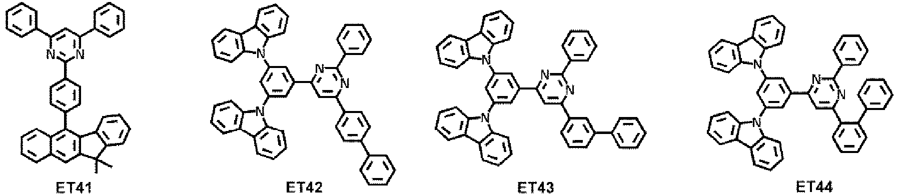

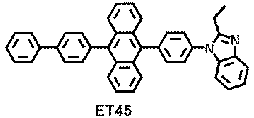

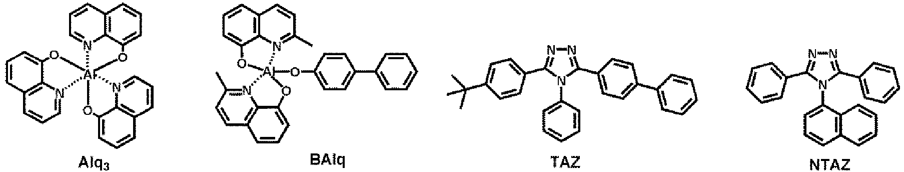

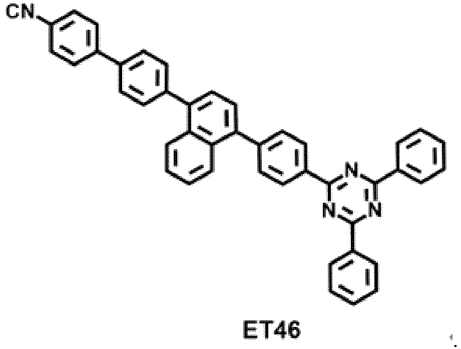

A thickness of the electron transport region 140 may be in a range of about 100 Å to about 5,000 Å, for example, about 160 Å to about 4,000 Å. When the electron transport region 140 comprises a buffer layer, a hole-blocking layer, an electron control layer, an electron transport layer, or any combination thereof, a thickness of the buffer layer, the hole-blocking layer, or the electron control layer may each independently be in a range of about 20 Å to about 1,000 Å, for example, about 30 Å to about 300 Å, and a thickness of the electron transport layer may be in a range of about 100 Å to about 1,000 Å, for example, about 150 Å to about 500 Å. When the thicknesses of the buffer layer, the hole-blocking layer, the electron control layer, the electron transport layer, and/or the electron transport region 140 are within the respective ranges described above, satisfactory electron-transporting characteristics may be obtained without a substantial increase in driving voltage.

In one or more embodiments, the electron transport region 140 (for example, the electron transport layer in the electron transport region 140) may further comprise, in addition to one or more of the materials described above, a metal-containing material.

The metal-containing material may comprise an alkali metal complex, an alkaline earth metal complex, or any combination thereof. A metal ion of the alkali metal complex may be a Li ion, a Na ion, a K ion, a Rb ion, or a Cs ion, and a metal ion of the alkaline earth metal complex may be a Be ion, a Mg ion, a Ca ion, a Sr ion, or a Ba ion. A ligand coordinated with the metal ion of the alkali metal complex or the metal ion of the alkaline earth-metal complex may comprise hydroxyquinoline, hydroxyisoquinoline, hydroxybenzoquinoline, hydroxyacridine, hydroxyphenanthridine, hydroxyphenyloxazole, hydroxyphenylthiazole, hydroxyphenyloxadiazole, hydroxyphenylthiadiazole, hydroxyphenylpyridine, hydroxyphenylbenzimidazole, hydroxyphenylbenzothiazole, bipyridine, phenanthroline, cyclopentadiene, or any combination thereof.

In one or more embodiments, the metal-containing material may comprise a Li complex. The Li complex may comprise, for example, Compound ET-D1 (LiQ) or ET-D2:

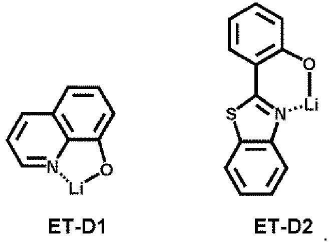

In one or more embodiments, the electron transport region 140 may comprise an electron injection layer that facilitates the injection of electrons from the second electrode 150. The electron injection layer may directly contact the second electrode 150.

The electron injection layer may have i) a single-layer structure comprising (e.g., consisting of) a single layer comprising (e.g., consisting of) a single material, ii) a single-layer structure comprising (e.g., consisting of) a single layer comprising multiple materials that are different from each other, or iii) a multi-layer structure comprising multiple layers comprising multiple materials that are different from each other.

The electron injection layer may comprise an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof.

The alkali metal may comprise Li, Na, K, Rb, Cs, or any combination thereof. The alkaline earth metal may comprise Mg, Ca, Sr, Ba, or any combination thereof. The rare earth metal may comprise Sc, Y, Ce, Tb, Yb, Gd, or any combination thereof.

The alkali metal-containing compound, the alkaline earth metal-containing compound, and the rare earth metal-containing compound may comprise oxides, halides (for example, fluorides, chlorides, bromides, iodides, and/or the like), or tellurides of the alkali metal, the alkaline earth metal, and the rare earth metal, respectively, or any combination thereof.

The alkali metal-containing compound may comprise: an alkali metal oxide, such as Li₂O, Cs₂O, and/or K₂O; an alkali metal halide, such as LiF, NaF, CsF, KF, Lil, Nal, Csl, and/or Kl; or any combination thereof. The alkaline earth metal-containing compound may comprise an alkaline earth metal oxide, such as BaO, SrO, CaO, BaₓSr₁₋ₓO (wherein x is a real number satisfying 0<x<1), and/or BaₓCa₁₋ₓO (wherein x is a real number satisfying 0<x<1). The rare earth metal-containing compound may comprise YbF₃, ScF₃, Sc₂O₃, Y₂O₃, Ce₂O₃, GdF₃, TbF₃, YbI₃, ScI₃, TbI₃, or any combination thereof. In one or more embodiments, the rare earth metal-containing compound may comprise a lanthanide metal telluride. Non-limiting examples of the lanthanide metal telluride may comprise LaTe, CeTe, PrTe, NdTe, PmTe, SmTe, EuTe, GdTe, TbTe, DyTe, HoTe, ErTe, TmTe, YbTe, LuTe, La₂Te₃, Ce₂Te₃, Pr₂Te₃, Nd₂Te₃, Pm₂Te₃, Sm₂Te₃, Eu₂Te₃, Gd₂Te₃, Tb₂Te₃, Dy₂Te₃, Ho₂Te₃, Er₂Te₃, Tm₂Te₃, Yb₂Te₃, Lu₂Te₃, and/or the like.

The alkali metal complex, the alkaline earth-metal complex, and the rare earth metal complex may comprise i) one of metal ions of the alkali metal, one of metal ions of the alkaline earth metal, and one of metal ions of the rare earth metal, respectively, and ii) a ligand bonded to the respective metal ion, for example, hydroxyquinoline, hydroxyisoquinoline, hydroxybenzoquinoline, hydroxyacridine, hydroxyphenanthridine, hydroxyphenyloxazole, hydroxyphenylthiazole, hydroxyphenyloxadiazole, hydroxyphenylthiadiazole, hydroxyphenylpyridine, hydroxyphenyl benzimidazole, hydroxyphenylbenzothiazole, bipyridine, phenanthroline, cyclopentadiene, or any combination thereof.

In one or more embodiments, the electron injection layer may comprise (e.g., consist of) an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof, as described above. In one or more embodiments, the electron injection layer may further comprise an organic material (for example, the compound represented by Formula 601).

In one or more embodiments, the electron injection layer may comprise (e.g., consist of) i) an alkali metal-containing compound (for example, an alkali metal halide), or ii) a) an alkali metal-containing compound (for example, an alkali metal halide), and b) an alkali metal, an alkaline earth metal, a rare earth metal, or any combination thereof. In one or more embodiments, the electron injection layer may be a Kl:Yb co-deposited layer, an Rbl:Yb co-deposited layer, a LiF:Yb co-deposited layer, and/or the like.

When the electron injection layer further comprises an organic material, the alkali metal, the alkaline earth metal, the rare earth metal, the alkali metal-containing compound, the alkaline earth metal-containing compound, the rare earth metal-containing compound, the alkali metal complex, the alkaline earth-metal complex, the rare earth metal complex, or any combination thereof may be uniformly (e.g., substantially uniformly) or non-uniformly dispersed in a matrix comprising the organic material.

A thickness of the electron injection layer may be in a range of about 1 Å to about 100 Å, for example, about 3 Å to about 90 Å. When the thickness of the electron injection layer is within these ranges, satisfactory electron injection characteristics may be obtained without a substantial increase in driving voltage.

### Second electrode 150

The second electrode 150 may be arranged on the electron transport region 140. The second electrode 150 may be a cathode, which is an electron injection electrode, and as a material for forming the second electrode 150, a metal, an alloy, an electrically conductive compound, or any combination thereof, each having a low-work function, may be used.

The second electrode 150 may comprise lithium (Li), silver (Ag), magnesium (Mg), aluminum (Al), aluminum-lithium (Al-Li), calcium (Ca), magnesium-indium (Mg-In), magnesium-silver (Mg-Ag), ytterbium (Yb), silver-ytterbium (Ag-Yb), ITO, IZO, or any combination thereof. The second electrode 150 may be a transmissive electrode, a transflective electrode, or a reflective electrode.

The second electrode 150 may have a single-layer structure or a multi-layer structure comprising multiple layers.

### Capping layer

In one or more embodiments, the light-emitting device 10 may further comprise a capping layer arranged outside (e.g., on) the first electrode 110 and/or the second electrode 150.

In one or more embodiments, the capping layer may comprise the organic compound described above.

In one or more embodiments, the light-emitting device 10 may further comprise a first capping layer arranged outside (e.g., on) the first electrode 110. The first capping layer may comprise the organic compound described above.

In one or more embodiments, the light-emitting device 10 may further comprise a second capping layer arranged outside (e.g., on) the second electrode 150. The second capping layer may comprise the organic compound described above.

In one or more embodiments, the light-emitting device 10 may further comprise a first capping layer arranged outside (e.g., on) the first electrode 110 and a second capping layer arranged outside (e.g., on) the second electrode 150. At least one of the first capping layer and/or the second capping layer may (e.g., the first capping layer and the second capping layer may each independently) comprise the organic compound described above.

In one or more embodiments, light generated in the emission layer 130 of the light-emitting device 10 may be extracted toward the outside through the first electrode 110, which is a transflective electrode or a transmissive electrode, and the first capping layer. In one or more embodiments, light generated in the emission layer 130 of the light-emitting device 10 may be extracted toward the outside through the second electrode 150, which is a transflective electrode or a transmissive electrode, and the second capping layer.

The first capping layer and the second capping layer may increase external emission efficiency according to the principle of constructive interference. Accordingly, the light extraction efficiency of the light-emitting device 10 may be increased, and thus, the luminescence efficiency of the light-emitting device 10 may be improved.

Each of the first capping layer and the second capping layer may comprise a material having a refractive index of about 1.2 or more (at 460 nm).

The first capping layer and the second capping layer may each independently be an organic capping layer comprising an organic material, an inorganic capping layer comprising an inorganic material, or an organic-inorganic composite capping layer comprising an organic material and an inorganic material.

At least one of the first capping layer and/or the second capping layer may (e.g., the first capping layer and the second capping layer may each independently) comprise a carbocyclic compound, a heterocyclic compound, an amine group-containing compound, a porphine derivative, a phthalocyanine derivative, a naphthalocyanine derivative, an alkali metal complex, an alkaline earth metal complex, or any combination thereof. The carbocyclic compound, the heterocyclic compound, and the amine group-containing compound may each optionally be substituted with a substituent comprising O, N, S, Se, Si, F, Cl, Br, I, or any combination thereof. In one or more embodiments, at least one of the first capping layer and/or the second capping layer may (e.g., the first capping layer and the second capping layer may each independently) comprise an amine group-containing compound.

In one or more embodiments, at least one of the first capping layer and/or the second capping layer may (e.g., the first capping layer and the second capping layer may each independently) comprise a compound represented by Formula 201, a compound represented by Formula 202, or any combination thereof.

In one or more embodiments, at least one of the first capping layer and/or the second capping layer may (e.g., the first capping layer and the second capping layer may each independently) comprise: one of (e.g., at least one or any one selected from among) Compounds HT28 to HT33; one of (e.g., at least one or any one selected from among) Compounds CP1 to CP6; β-NPB; or any combination thereof:

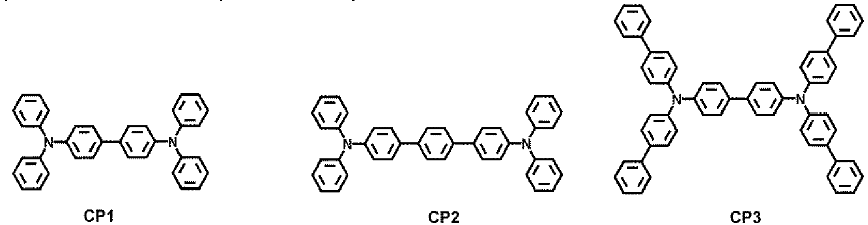

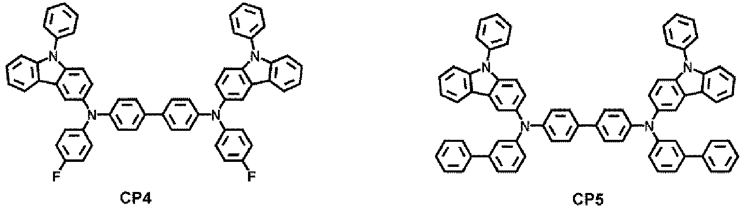

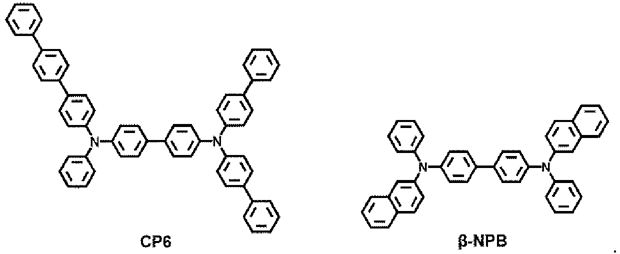

### Film

In one or more embodiments, the electronic apparatus may further comprise a film. The film may be, for example, an optical member (or a light control element) (for example, a color filter, a color conversion layer, a capping layer, a light extraction efficiency enhancement layer, a selective light-absorbing layer, a polarizing layer, a quantum dot-containing layer, and/or the like), a light-blocking member (for example, a light-reflective layer, a light-absorbing layer, and/or the like), a protective member (for example, an insulating layer, a dielectric layer, and/or the like), and/or the like.

### Electronic apparatus

The light-emitting device 10 may be comprised in one or more suitable electronic apparatuses. For example, an electronic apparatus comprising the light-emitting device 10 may be a display apparatus, an authentication apparatus, and/or the like.

In one or more embodiments, the electronic apparatus (for example, a display apparatus) may further comprise, in addition to the light-emitting device 10, i) a color filter, ii) a color conversion layer, or iii) a color filter and a color conversion layer. The color filter and/or the color conversion layer may be arranged in at least one direction in which light emitted from the light-emitting device 10 travels. For example, in one or more embodiments, light emitted from the light-emitting device 10 may be blue light or white light (e.g., combined white light). Details on the light-emitting device 10 may refer to the descriptions above.

The electronic apparatus may comprise a first substrate. The first substrate may comprise a plurality of subpixel areas, the color filter may comprise a plurality of color filter areas respectively corresponding to the subpixel areas, and the color conversion layer may comprise a plurality of color conversion areas respectively corresponding to the subpixel areas.

A pixel-defining film may be arranged among the subpixel areas to define each of the subpixel areas.

The color filter may further comprise a plurality of color filter areas and light-shielding patterns arranged among the color filter areas, and the color conversion layer may further comprise a plurality of color conversion areas and light-shielding patterns arranged among the color conversion areas.

The plurality of color filter areas (or the plurality of color conversion areas) may comprise a first area configured to emit first color light, a second area configured to emit second color light, and/or a third area configured to emit third color light, wherein the first color light, the second color light, and/or the third color light may have different maximum emission wavelengths. For example, in one or more embodiments, the first color light may be red light, the second color light may be green light, and the third color light may be blue light. For example, in one or more embodiments, the plurality of color filter areas (or the plurality of color conversion areas) may comprise quantum dots. For example, the first area may comprise red quantum dots to emit red light, the second area may comprise green quantum dots to emit green light, and the third area may not comprise (e.g., may exclude any of the) quantum dots. Details on the quantum dots may refer to the descriptions provided herein. The first area, the second area, and/or the third area may each further comprise a scatterer.

In one or more embodiments, the light-emitting device 10 may be to emit first light, the first area may be to absorb the first light to emit first-first color light, the second area may be to absorb the first light to emit second-first color light, and the third area may be to absorb the first light to emit third-first color light. In this regard, the first-first color light, the second-first color light, and the third-first color light may have different maximum emission wavelengths. For example, the first light may be blue light, the first-first color light may be red light, the second-first color light may be green light, and the third-first color light may be blue light.

In one or more embodiments, the electronic apparatus may further comprise a thin-film transistor, in addition to the light-emitting device 10. The thin-film transistor may comprise a source electrode, a drain electrode, and an active layer, and one selected from among the source electrode and the drain electrode may be electrically connected to the first electrode or the second electrode of the light-emitting device 10.

The thin-film transistor may further comprise a gate electrode, a gate insulating film, and/or the like.

The active layer may comprise crystalline silicon, amorphous silicon, an organic semiconductor, an oxide semiconductor, and/or the like.

In one or more embodiments, the electronic apparatus may further comprise a sealing portion for sealing the light-emitting device 10. The sealing portion may be arranged between the color filter and/or the color conversion layer and the light-emitting device 10. The sealing portion may allow light from the light-emitting device 10 to be extracted to the outside, and may concurrently (e.g., simultaneously) prevent or reduce ambient air and moisture from penetrating into the light-emitting device 10. The sealing portion may be a sealing substrate comprising a transparent glass substrate or a plastic substrate. The sealing portion may be a thin-film encapsulation layer comprising at least one layer of an organic layer and/or an inorganic layer. When the sealing portion is a thin-film encapsulation layer, the electronic apparatus may be flexible.

In one or more embodiments, various functional layers may be additionally arranged on the sealing portion, in addition to the color filter and/or the color conversion layer, according to the use of the electronic apparatus. Non-limiting examples of the functional layers may comprise a touch screen layer, a polarizing layer, and/or the like. The touch screen layer may be a pressure-sensitive touch screen layer, a capacitive touch screen layer, or an infrared touch screen layer.

The authentication apparatus may further comprise, in addition to the light-emitting device described above, a biometric information collector. The authentication apparatus may be, for example, a biometric authentication apparatus that authenticates an individual by using biometric information of a living body (for example, fingertips, pupils, and/or the like).

The electronic apparatus may be applied to one or more of displays, light sources, lighting, personal computers (for example, mobile personal computers), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram displays, ultrasonic diagnostic devices, or endoscope displays), fish finders, one or more suitable measuring instruments, meters (for example, meters for a vehicle, an aircraft, and a vessel), projectors, and/or the like.

### Electronic equipment

The light-emitting device 10 may be comprised in one or more suitable electronic equipment. For example, the electronic apparatus comprising the light-emitting device 10 may be comprised in one or more suitable electronic equipment.

In one or more embodiments, the electronic equipment comprising the light-emitting device 10 may be at least one of a flat panel display, a curved display, a computer monitor, a medical monitor, a television, a billboard, an indoor light, an outdoor light, a signal light, a head-up display, a fully transparent display, a partially transparent display, a flexible display, a rollable display, a foldable display, a stretchable display, a laser printer, a telephone, a mobile phone, a tablet, a phablet, a personal digital assistant (PDA), a wearable device, a laptop computer, a digital camera, a camcorder, a viewfinder, a micro display, a 3-dimension (3D) display, a virtual reality display, an augmented reality display, a vehicle, a video wall comprising multiple displays tiled together, a theater screen, a stadium screen, a phototherapy device, or a signboard.

### Descriptions of FIG. 2 and FIG. 3

FIG. 2 is a cross-sectional view of an electronic apparatus according to one or more embodiments of the present disclosure.

The electronic apparatus of FIG. 2 may comprise a substrate 100, a thin-film transistor (TFT), a light-emitting device, and an encapsulation portion 300 that seals the light-emitting device.

The substrate 100 may be a flexible substrate, a glass substrate, or a metal substrate. A buffer layer 210 may be on (e.g., arranged on) the substrate 100. The buffer layer 210 may prevent or reduce penetration of impurities through the substrate 100 and may provide a flat surface on the substrate 100.

The TFT may be on (e.g., arranged on) the buffer layer 210. The TFT may comprise an active layer 220, a gate electrode 240, a source electrode 260, and a drain electrode 270.

The active layer 220 may comprise an inorganic semiconductor, such as silicon or polysilicon, an organic semiconductor, or an oxide semiconductor, and may comprise a source region, a drain region, and a channel region.

A gate insulating film 230 for insulating the active layer 220 from the gate electrode 240 may be on (e.g., arranged on) the active layer 220, and the gate electrode 240 may be on (e.g., arranged on) the gate insulating film 230.

An interlayer insulating film 250 may be on (e.g., arranged on) the gate electrode 240. The interlayer insulating film 250 may be arranged between the gate electrode 240 and the source electrode 260 to insulate the gate electrode 240 from the source electrode 260 and between the gate electrode 240 and the drain electrode 270 to insulate the gate electrode 240 from the drain electrode 270.

The source electrode 260 and the drain electrode 270 may be on (e.g., arranged on) the interlayer insulating film 250. The interlayer insulating film 250 and the gate insulating film 230 may be formed to expose the source region and the drain region of the active layer 220, and the source electrode 260 and the drain electrode 270 may be arranged in contact with the exposed portions of the source region and the drain region of the active layer 220, respectively.

The TFT may be electrically connected to the light-emitting device to drive the light-emitting device, and may be covered and protected by a passivation layer 280. The passivation layer 280 may comprise an inorganic insulating film, an organic insulating film, or any combination thereof. The light-emitting device may be provided on the passivation layer 280. The light-emitting device may comprise a first electrode 110, an interlayer comprising an emission layer 130, and a second electrode 150.

The first electrode 110 may be on (e.g., arranged on) the passivation layer 280. The passivation layer 280 may be arranged to expose a portion of the drain electrode 270 without fully covering the drain electrode 270, and the first electrode 110 may be arranged to be connected to the exposed portion of the drain electrode 270.

A pixel-defining film 290 comprising an insulating material may be on (e.g., arranged on) the first electrode 110. The pixel-defining film 290 may expose a certain region of the first electrode 110, and the interlayer may be formed in the exposed region of the first electrode 110. The pixel-defining film 290 may be a polyimide-based organic film or a polyacrylic organic film. In one or more embodiments, at least some layers of the interlayer may extend beyond the upper portion of the pixel-defining film 290 to be arranged in the form of a common layer.

The second electrode 150 may be on (e.g., arranged on) the interlayer, and a capping layer 170 may be further formed on the second electrode 150. The capping layer 170 may be formed to cover the second electrode 150.

The encapsulation portion 300 may be on (e.g., arranged on) the capping layer 170. The encapsulation portion 300 may be arranged on the light-emitting device to protect the light-emitting device from moisture and/or oxygen. The encapsulation portion 300 may comprise: an inorganic film comprising silicon nitride (SiNₓ), silicon oxide (SiOₓ), indium tin oxide, indium zinc oxide, or any combination thereof; an organic film comprising polyethylene terephthalate, polyethylene naphthalate, polycarbonate, polyimide, polyethylene sulfonate, polyoxymethylene, polyarylate, hexamethyldisiloxane, an acrylic-based resin (for example, polymethyl methacrylate, polyacrylic acid, and/or the like), an epoxy-based resin (for example, aliphatic glycidyl ether (AGE), and/or the like), or any combination thereof; or any combination of the inorganic film and the organic film.

FIG. 3 is a cross-sectional view of an electronic apparatus according to one or more embodiments of the present disclosure.

The electronic apparatus of FIG. 3 is substantially the same as the electronic apparatus of FIG. 2, except that a light-shielding pattern 500 and a functional region 400 are additionally arranged on the encapsulation portion 300. The functional region 400 may be i) a color filter area, ii) a color conversion area, or iii) a combination of a color filter area and a color conversion area. In one or more embodiments, the light-emitting device comprised in the electronic apparatus of FIG. 3 may be a tandem light-emitting device.

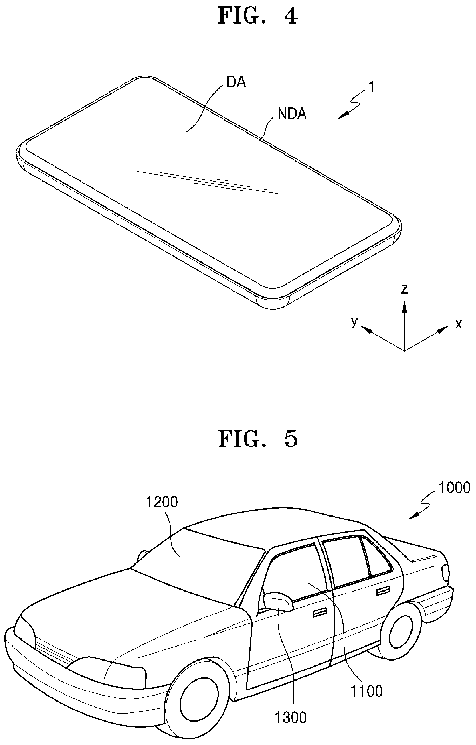

### Description of FIG. 4

FIG. 4 is a schematic perspective view of electronic equipment 1 comprising a light-emitting device according to one or more embodiments of the present disclosure. The electronic equipment 1 may be, as an electronic apparatus that displays a moving image or a still image, a portable electronic equipment, such as a mobile phone, a smartphone, a tablet personal computer (PC), a mobile communication terminal, an electronic notebook, an electronic book, a portable multimedia player (PMP), a navigation, or an ultra-mobile PC (UMPC), as well as one or more suitable products, such as a television, a laptop, a monitor, a billboard, or an Internet of things (IoT) device. The electronic equipment 1 may be such a product above or a part thereof. In addition, the electronic equipment 1 may be a wearable device, such as a smart watch, a watch phone, a glasses-type (kind) display, or a head mounted display (HMD), or a part of the wearable device. However, embodiments are not limited thereto. For example, in one or more embodiments, the electronic equipment 1 may be a center information display (CID) arranged on an instrument panel and a center fascia or dashboard of a vehicle, a room mirror display instead of a side mirror of a vehicle, an entertainment display for a rear seat of a vehicle, a display arranged on the back of a front seat thereof, a head up display (HUD) installed in the front of a vehicle or projected on a front window glass thereof, or a computer generated hologram augmented reality head up display (CGH AR HUD). FIG. 4 illustrates one or more embodiments in which the electronic equipment 1 is a smartphone for convenience of explanation.

The electronic equipment 1 may comprise a display area DA and a non-display area NDA outside (e.g., around) the display area DA. The electronic equipment 1 may implement an image through an array of a plurality of pixels that are two-dimensionally arranged in the display area DA.

The non-display area NDA may be an area that does not display an image, and may entirely be around (e.g., surround) the display area DA. On the non-display area NDA, a driver for providing electrical signals or power to display devices arranged on the display area DA may be arranged. On the non-display area NDA, a pad, which is an area to which an electronic element or a printed circuit board may be electrically connected, may be arranged.

In the electronic equipment 1, a length in an x-axis direction and a length (e.g., a width) in a y-axis direction may be different from each other. For example, in one or more embodiments, as shown in FIG. 4, the length in the x-axis direction may be less than the length (e.g., the width) in the y-axis direction. In one or more embodiments, the length in the x-axis direction may be the same as the length (e.g., the width) in the y-axis direction. In one or more embodiments, the length in the x-axis direction may be greater than the length (e.g., the width) in the y-axis direction.

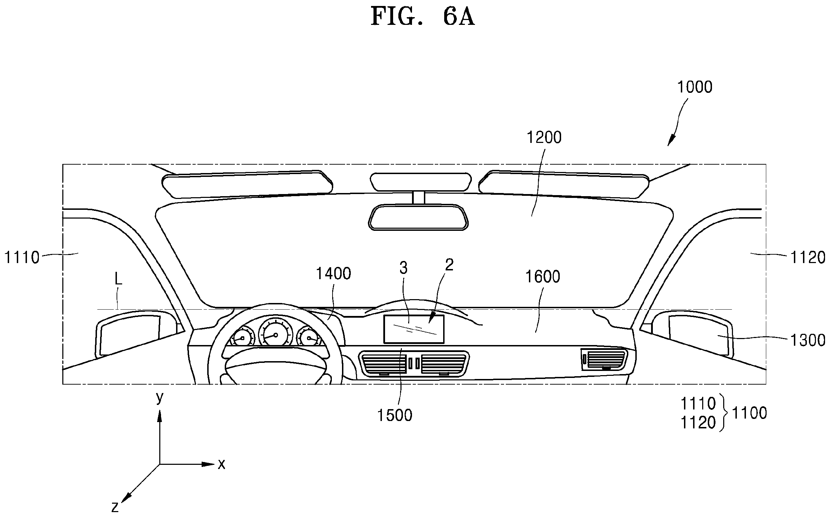

### Descriptions of FIGS. 5 and 6A to 6C

FIG. 5 is a schematic view of an exterior of a vehicle 1000 as electronic equipment comprising a light-emitting device according to one or more embodiments of the present disclosure. FIGS. 6A to 6C are each a schematic view of an interior of the vehicle 1000 according to one or more embodiments.

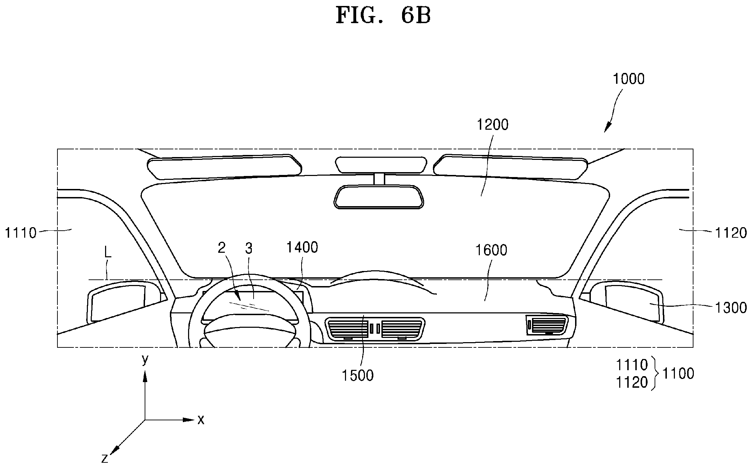

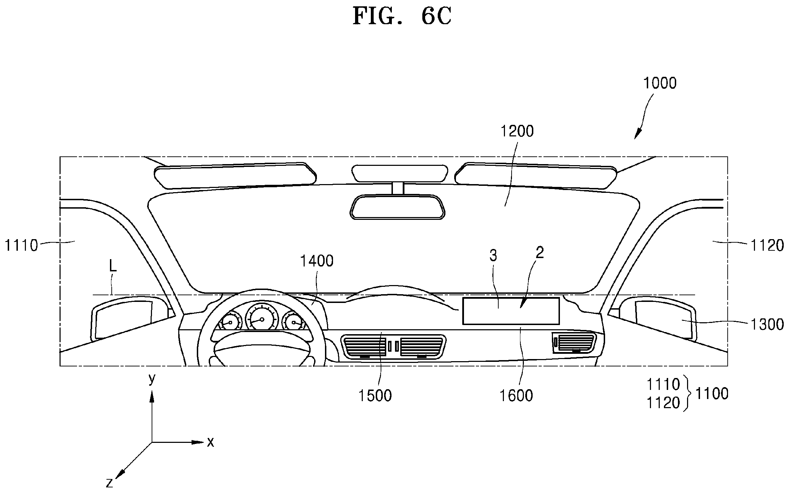

Referring to FIGS. 5, 6A, 6B, and 6C, the vehicle 1000 may refer to one or more suitable apparatuses for moving an object to be transported, such as a human, an object, or an animal, from a departure point to a destination point. The vehicle 1000 may comprise a vehicle traveling on a road or a track, a vessel moving over the sea or a river, an airplane flying in the sky using the action of air, and/or the like.

In one or more embodiments, the vehicle 1000 may travel on a road or a track. The vehicle 1000 may move in a certain direction according to rotation of at least one wheel thereof. For example, the vehicle 1000 may comprise a three-wheeled or four-wheeled vehicle, a construction machine, a two-wheeled vehicle, a prime mover device, a bicycle, or a train running on a track.

The vehicle 1000 may comprise a body having an interior and an exterior, and a chassis in which mechanical apparatuses necessary for driving are installed as other parts except for the body of the vehicle 1000. The exterior of the body of the vehicle 1000 may comprise a front panel, a bonnet, a roof panel, a rear panel, a trunk, a pillar provided at a boundary between doors, and/or the like. The chassis of the vehicle 1000 may comprise a power generating device, a power transmitting device, a driving device, a steering device, a braking device, a suspension device, a transmission device, a fuel device, front and rear left and right wheels, and/or the like.

The vehicle 1000 may comprise a side window glass 1100, a front window glass 1200, a side mirror 1300, a cluster 1400, a center fascia 1500, a passenger seat dashboard 1600, and a display apparatus 2.

The side window glass 1100 and the front window glass 1200 may be partitioned by a pillar arranged between the side window glass 1100 and the front window glass 1200.

The side window glass 1100 may be installed on a side of the vehicle 1000. In one or more embodiments, the side window glass 1100 may be installed on a door of the vehicle 1000. A plurality of side window glasses 1100 may be provided and may face each other. In one or more embodiments, the side window glass 1100 may comprise a first side window glass 1110 and a second side window glass 1120. In one or more embodiments, the first side window glass 1110 may be arranged adjacent to the cluster 1400. The second side window glass 1120 may be arranged adjacent to the passenger seat dashboard 1600.

In one or more embodiments, the side window glasses 1100 may be spaced and/or apart (e.g., spaced apart or separated) from each other in an x-direction or a -x-direction (the direction opposite the x-direction). For example, the first side window glass 1110 and the second side window glass 1120 may be spaced and/or apart (e.g., spaced apart or separated) from each other in the x-direction or the -x-direction. For example, an imaginary straight line L connecting the side window glasses 1100 may extend in the x-direction or the -x-direction. For example, an imaginary straight line L connecting the first side window glass 1110 and the second side window glass 1120 to each other may extend in the x-direction or the -x-direction.

The front window glass 1200 may be installed in the front of the vehicle 1000. The front window glass 1200 may be arranged between the side window glasses 1100 opposite to (e.g., facing) each other.

The side mirror 1300 may provide a rear view of the vehicle 1000. The side mirror 1300 may be installed on the exterior of the body of the vehicle 1000. In one or more embodiments, a plurality of side mirrors 1300 may be provided. Any one of the plurality of side mirrors 1300 may be arranged outside the first side window glass 1110. Another of the plurality of side mirrors 1300 may be arranged outside the second side window glass 1120.

The cluster 1400 may be arranged in front of the steering wheel. The cluster 1400 may comprise a tachometer, a speedometer, a coolant thermometer, a fuel gauge, a turn signal indicator, a high beam indicator, a warning light, a seat belt warning light, an odometer, a tachograph, an automatic shift selector indicator, a door open warning light, an engine oil warning light, and/or a low fuel warning light.

The center fascia 1500 may comprise a control panel on which a plurality of buttons for adjusting an audio device, an air conditioning device, and a seat heater are arranged. The center fascia 1500 may be arranged on one side of the cluster 1400.

The passenger seat dashboard 1600 may be spaced and/or apart (e.g., spaced apart or separated) from the cluster 1400, and the center fascia 1500 may be arranged between the cluster 1400 and the passenger seat dashboard 1600. In one or more embodiments, the cluster 1400 may be arranged to correspond to a driver seat, and the passenger seat dashboard 1600 may be arranged to correspond to a passenger seat. In one or more embodiments, the cluster 1400 may be adjacent to the first side window glass 1110, and the passenger seat dashboard 1600 may be adjacent to the second side window glass 1120.

In one or more embodiments, the display apparatus 2 may comprise a display panel 3, and the display panel 3 may display an image. The display apparatus 2 may be arranged inside the vehicle 1000. In one or more embodiments, the display apparatus 2 may be arranged between the side window glasses 1100 opposite to (e.g., facing) each other. The display apparatus 2 may be arranged on at least one of the cluster 1400, the center fascia 1500, or the passenger seat dashboard 1600.

The display apparatus 2 may comprise an organic light-emitting display apparatus, an inorganic light-emitting display apparatus, a quantum dot display apparatus, and/or the like. Hereinafter, as the display apparatus 2 according to one or more embodiments, an organic light-emitting display apparatus comprising the light-emitting device according to the disclosure will be described as an example, but one or more suitable types (kinds) of display apparatuses as described above may be used in embodiments.

Referring to FIG. 6A, in one or more embodiments, the display apparatus 2 may be arranged on the center fascia 1500. In one or more embodiments, the display apparatus 2 may display navigation information. In one or more embodiments, the display apparatus 2 may display information regarding audio settings, video setting, or vehicle settings.

Referring to FIG. 6B, in one or more embodiments, the display apparatus 2 may be arranged on the cluster 1400. In these embodiments, the cluster 1400 may display driving information and/or the like through the display apparatus 2. For example, the cluster 1400 may digitally implement driving information and/or the like. The cluster 1400 may digitally display vehicle information and driving information as images. For example, in one or more embodiments, a needle and a gauge of a tachometer and one or more suitable warning light icons may be displayed by digital signals.

Referring to FIG. 6C, in one or more embodiments, the display apparatus 2 may be arranged on the passenger seat dashboard 1600. The display apparatus 2 may be embedded in the passenger seat dashboard 1600 or arranged on the passenger seat dashboard 1600. In one or more embodiments, the display apparatus 2 arranged on the passenger seat dashboard 1600 may display an image related to information displayed on the cluster 1400 and/or information displayed on the center fascia 1500. In one or more embodiments, the display apparatus 2 arranged on the passenger seat dashboard 1600 may display information different from information displayed on the cluster 1400 and/or information displayed on the center fascia 1500.

### Manufacturing method

Respective layers comprised in the hole transport region 120, the emission layer 130, and respective layers comprised in the electron transport region 140 may each be formed in a certain region by using one or more suitable methods, such as vacuum deposition, spin coating, casting, a Langmuir-Blodgett (LB) method, ink-jet printing, laser-printing, and/or laser-induced thermal imaging (LITI).

When respective layers comprised in the hole transport region 120, the emission layer 130, and respective layers comprised in the electron transport region 140 are each formed by vacuum deposition, the deposition may be performed at a deposition temperature of about 100 °C to about 500 °C, a vacuum degree of about 10⁻⁸ torr to about 10⁻³ torr, and a deposition speed of about 0.01 Å/sec to about 100 Å/sec, depending on a material to be comprised in a layer to be formed and the structure of a layer to be formed.

### Definition of Terms

The term "C₃-C₆₀ carbocyclic group" as used herein refers to a cyclic group comprising (e.g., consisting of) carbon atoms as the only ring-forming atoms and having 3 to 60 carbon atoms.

The term "C₁-C₆₀ heterocyclic group" as used herein refers to a cyclic group that has 1 to 60 ring-forming carbon atoms and further has, in addition to carbon atoms, a heteroatom as a ring-forming atom.

The C₃-C₆₀ carbocyclic group and the C₁-C₆₀ heterocyclic group may each be a monocyclic group comprising (e.g., consisting of) one (e.g., exactly one) ring or a polycyclic group in which two or more rings are condensed with each other. For example, the number of ring-forming atoms of the C₁-C₆₀ heterocyclic group may be from 3 to 61.

The term "cyclic group" as used herein may comprise both (e.g., simultaneously) the C₃-C₆₀ carbocyclic group and the C₁-C₆₀ heterocyclic group.

The term "π electron-rich C₃-C₆₀ cyclic group" as used herein refers to a cyclic group that has 3 to 60 carbon atoms and does not comprise *-N=*' as a ring-forming moiety.

The term "π electron-deficient nitrogen-containing C₁-C₆₀ cyclic group" as used herein refers to a heterocyclic group that has 1 to 60 carbon atoms and comprises *-N=*' as a ring-forming moiety.

For example,
the C₃-C₆₀ carbocyclic group may be i) Group T1 or ii) a condensed cyclic group in which two or more of Group T1 are condensed with each other (for example, a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, or an indenoanthracene group), and
the C₁-C₆₀ heterocyclic group may be i) Group T2, ii) a condensed cyclic group in which two or more of Group T2 are condensed with each other, or iii) a condensed cyclic group in which at least one Group T2 and at least one Group T1 are condensed with each other (for example, a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, a xanthene group, and/or the like).

The π electron-rich C₃-C₆₀ cyclic group may be i) Group T1, ii) a condensed cyclic group in which two or more of Group T1 are condensed with each other, iii) Group T3, iv) a condensed cyclic group in which two or more of Group T3 are condensed with each other, or v) a condensed cyclic group in which at least one Group T3 and at least one Group T1 are condensed with each other (for example, the C₃-C₆₀ carbocyclic group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, and/or the like).

The π electron-deficient nitrogen-containing C₁-C₆₀ cyclic group may be i) Group T4, ii) a condensed cyclic group in which two or more of Group T4 are condensed with each other, iii) a condensed cyclic group in which at least one Group T4 and at least one Group T1 are condensed with each other, iv) a condensed cyclic group in which at least one Group T4 and at least one Group T3 are condensed with each other, or v) a condensed cyclic group in which at least one Group T4, at least one Group T1, and at least one Group T3 are condensed with one another (for example, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, and/or the like).

Group T1 may be a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane (or bicyclo[2.2.1]heptane) group, a norbornene group, a bicyclo[1.1.1]pentane group, a bicyclo[2.1.1]hexane group, a bicyclo[2.2.2]octane group, or a benzene group.

Group T2 may be a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group.

Group T3 may be a furan group, a thiophene group, a 1H-pyrrole group, a silole group, or a borole group.

Group T4 may be a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, or a tetrazine group.

The terms "cyclic group," "C₃-C₆₀ carbocyclic group," "C₁-C₆₀ heterocyclic group," "π electron-rich C₃-C₆₀ cyclic group," and "π electron-deficient nitrogen-containing C₁-C₆₀ cyclic group" as used herein each refer to a group condensed to any cyclic group, a monovalent group, or a polyvalent group (for example, a divalent group, a trivalent group, a tetravalent group, and/or the like) according to the structure of a formula for which the corresponding term is used.

For example, the "benzene group" may be a benzo group, a phenyl group, a phenylene group, and/or the like, which may be easily understood by one of ordinary skill in the art according to the structure of a formula comprising the "benzene group."

Non-limiting examples of the monovalent C₃-C₆₀ carbocyclic group and the monovalent C₁-C₆₀ heterocyclic group may comprise a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group.

Non-limiting examples of the divalent C₃-C₆₀ carbocyclic group and the divalent C₁-C₆₀ heterocyclic group may comprise a C₃-C₁₀ cycloalkylene group, a C₁-C₁₀ heterocycloalkylene group, a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a substituted or unsubstituted divalent non-aromatic condensed heteropolycyclic group.

The term "C₁-C₆₀ alkyl group" as used herein refers to a linear or branched aliphatic hydrocarbon monovalent group that has 1 to 60 carbon atoms, and non-limiting examples thereof may comprise a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, and/or the like.

The term "C₁-C₆₀ alkylene group" as used herein refers to a divalent group having substantially the same structure as the C₁-C₆₀ alkyl group.

The term "C₂-C₆₀ alkenyl group" as used herein refers to a monovalent hydrocarbon group having at least one carbon-carbon double bond in the middle or at the terminus of a C₂-C₆₀ alkyl group, and non-limiting examples thereof may comprise an ethenyl group, a propenyl group, a butenyl group, and/or the like.

The term "C₂-C₆₀ alkenylene group" as used herein refers to a divalent group having substantially the same structure as the C₂-C₆₀ alkenyl group.

The term "C₂-C₆₀ alkynyl group" as used herein refers to a monovalent hydrocarbon group having at least one carbon-carbon triple bond in the middle or at the terminus of a C₂-C₆₀ alkyl group, and unlimiting examples thereof may comprise an ethynyl group, a propynyl group, and/or the like.

The term "C₂-C₆₀ alkynylene group" as used herein refers to a divalent group having substantially the same structure as the C₂-C₆₀ alkynyl group.

The term "C₁-C₆₀ alkoxy group" as used herein refers to a monovalent group represented by -OA₁₀₁ (wherein A₁₀₁ is a C₁-C₆₀ alkyl group), and non-limiting examples thereof may comprise a methoxy group, an ethoxy group, an isopropyloxy group, and/or the like.

The term "C₃-C₁₀ cycloalkyl group" as used herein refers to a monovalent saturated hydrocarbon cyclic group having 3 to 10 carbon atoms, and non-limiting examples thereof may comprise a cyclopropyl group, a cyclobutyl group, a cyclopentyl group, a cyclohexyl group, a cycloheptyl group, a cyclooctyl group, an adamantanyl (i.e., adamantyl) group, a norbornanyl (i.e., norbornyl) group (or bicyclo[2.2.1]heptyl group), a bicyclo[1.1.1]pentyl group, a bicyclo[2.1.1]hexyl group, a bicyclo[2.2.2]octyl group, and/or the like.

The term "C₃-C₁₀ cycloalkylene group" as used herein refers to a divalent group having substantially the same structure as the C₃-C₁₀ cycloalkyl group.

The term "C₁-C₁₀ heterocycloalkyl group" as used herein refers to a monovalent cyclic group of 1 to 10 carbon atoms, further comprising, in addition to carbon atoms, at least one heteroatom as ring-forming atoms, and non-limiting examples thereof may comprise a 1,2,3,4-oxatriazolidinyl group, a tetrahydrofuranyl group, a tetrahydrothiophenyl group, and/or the like.

The term "C₁-C₁₀ heterocycloalkylene group" as used herein refers to a divalent group having substantially the same structure as the C₁-C₁₀ heterocycloalkyl group.

The term "C₃-C₁₀ cycloalkenyl group" as used herein refers to a monovalent cyclic group that has 3 to 10 carbon atoms, at least one carbon-carbon double bond in the ring thereof, and no aromaticity, and non-limiting examples thereof may comprise a cyclopentenyl group, a cyclohexenyl group, a cycloheptenyl group, and/or the like.

The term "C₃-C₁₀ cycloalkenylene group" as used herein refers to a divalent group having substantially the same structure as the C₃-C₁₀ cycloalkenyl group.

The term "C₁-C₁₀ heterocycloalkenyl group" as used herein refers to a monovalent cyclic group of 1 to 10 carbon atoms, further comprising, in addition to carbon atoms, at least one heteroatom as ring-forming atoms and at least one double bond in the ring thereof. Non-limiting examples of the C₁-C₁₀ heterocycloalkenyl group may comprise a 4,5-dihydro-1,2,3,4-oxatriazolyl group, a 2,3-dihydrofuranyl group, a 2,3-dihydrothiophenyl group, and/or the like.

The term "C₁-C₁₀ heterocycloalkenylene group" as used herein refers to a divalent group having substantially the same structure as the C₁-C₁₀ heterocycloalkenyl group.

The term "C₆-C₆₀ aryl group" as used herein refers to a monovalent group having a carbocyclic aromatic system of 6 to 60 carbon atoms.

The term "C₆-C₆₀ arylene group" as used herein refers to a divalent group having a carbocyclic aromatic system of 6 to 60 carbon atoms.

Non-limiting examples of the C₆-C₆₀ aryl group may comprise a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, an ovalenyl group, and/or the like.

When the C₆-C₆₀ aryl group and the C₆-C₆₀ arylene group each comprise two or more rings, the two or more rings may be condensed with each other.

The term "C₁-C₆₀ heteroaryl group" as used herein refers to a monovalent group having a heterocyclic aromatic system of 1 to 60 carbon atoms, further comprising, in addition to carbon atoms, at least one heteroatom, as ring-forming atoms.

The term "C₁-C₆₀ heteroarylene group" as used herein refers to a divalent group having a heterocyclic aromatic system of 1 to 60 carbon atoms, further comprising, in addition to carbon atoms, at least one heteroatom, as ring-forming atoms.

Non-limiting examples of the C₁-C₆₀ heteroaryl group may comprise a pyridinyl group, a pyrimidinyl group, a pyrazinyl group, a pyridazinyl group, a triazinyl group, a quinolinyl group, a benzoquinolinyl group, an isoquinolinyl group, a benzoisoquinolinyl group, a quinoxalinyl group, a benzoquinoxalinyl group, a quinazolinyl group, a benzoquinazolinyl group, a cinnolinyl group, a phenanthrolinyl group, a phthalazinyl group, a naphthyridinyl group, and/or the like.

When the C₁-C₆₀ heteroaryl group and the C₁-C₆₀ heteroarylene group each comprise two or more rings, the two or more rings may be condensed with each other.

The term "monovalent non-aromatic condensed polycyclic group" as used herein refers to a monovalent group (for example, having 8 to 60 carbon atoms) having two or more rings condensed to each other, only carbon atoms as ring-forming atoms, and no aromaticity in its entire molecular structure when considered as a whole. Non-limiting examples of the monovalent non-aromatic condensed polycyclic group may comprise an indenyl group, a fluorenyl group, a spiro-bifluorenyl group, a benzofluorenyl group, an indenophenanthrenyl group, an indenoanthracenyl group, and/or the like.

The term "divalent non-aromatic condensed polycyclic group" as used herein refers to a divalent group having substantially the same structure as the monovalent non-aromatic condensed polycyclic group.

The term "monovalent non-aromatic condensed heteropolycyclic group" as used herein refers to a monovalent group (for example, having 1 to 60 carbon atoms) having two or more rings condensed to each other, further comprising, in addition to carbon atoms, at least one heteroatom, as ring-forming atoms, and having no aromaticity in its entire molecular structure when considered as a whole. Non-limiting examples of the monovalent non-aromatic condensed heteropolycyclic group may comprise a pyrrolyl group, a thiophenyl group, a furanyl group, an indolyl group, a benzoindolyl group, a naphthoindolyl group, an isoindolyl group, a benzoisoindolyl group, a naphthoisoindolyl group, a benzosilolyl group, a benzothiophenyl group, a benzofuranyl group, a carbazolyl group, a dibenzosilolyl group, a dibenzothiophenyl group, a dibenzofuranyl group, an azacarbazolyl group, an azafluorenyl group, an azadibenzosilolyl group, an azadibenzothiophenyl group, an azadibenzofuranyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, a benzoxazolyl group, a benzothiazolyl group, a benzoxadiazolyl group, a benzothiadiazolyl group, an imidazopyridinyl group, an imidazopyrimidinyl group, an imidazotriazinyl group, an imidazopyrazinyl group, an imidazopyridazinyl group, an indenocarbazolyl group, an indolocarbazolyl group, a benzofurocarbazolyl group, a benzothienocarbazolyl group, a benzosilolocarbazolyl group, a benzoindolocarbazolyl group, a benzocarbazolyl group, a benzonaphthofuranyl group, a benzonaphthothiophenyl group, a benzonaphthosilolyl group, a benzofurodibenzofuranyl group, a benzofurodibenzothiophenyl group, a benzothienodibenzothiophenyl group, and/or the like.

The term "divalent non-aromatic condensed heteropolycyclic group" as used herein refers to a divalent group having substantially the same structure as the monovalent non-aromatic condensed heteropolycyclic group.

The term "C₆-C₆₀ aryloxy group" as used herein refers to -OA₁₀₂ (wherein A₁₀₂ is a C₆-C₆₀ aryl group).

The term "C₆-C₆₀ arylthio group" as used herein refers to -SA₁₀₃ (wherein A₁₀₃ is a C₆-C₆₀ aryl group).

The term "C₇-C₆₀ arylalkyl group" as used herein refers to -A₁₀₄A₁₀₅ (wherein A₁₀₄ is a C₁-C₅₄ alkylene group, and A₁₀₅ is a C₆-C₅₉ aryl group).

The term "C₂-C₆₀ heteroarylalkyl group" as used herein refers to -A₁₀₆A₁₀₇ (wherein A₁₀₆ is a C₁-C₅₉ alkylene group, and A₁₀₇ is a C₁-C₅₉ heteroaryl group).

The term "R₁₀ₐ" as used herein may be:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), - P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, - Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)2(Q₃₁), or - P(=O)(Q₃₁)(Q₃₂).

Q₁ to Q₃, Q₁₁ to Q₁₃, Q₂₁ to Q₂₃, and Q₃₁ to Q₃₃ as used herein may each independently be: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

The term "heteroatom" as used herein refers to any atom other than a carbon atom or a hydrogen atom. Non-limiting examples of the heteroatom may comprise O, S, N, P, Si, B, Ge, Se, or any combination thereof.

The term "transition metal" as used herein may comprise hafnium (Hf), tantalum (Ta), tungsten (W), rhenium (Re), osmium (Os), iridium (Ir), platinum (Pt), gold (Au), and/or the like.

As used herein, the term "D" may refer to deuterium, the term "Ph" may refer to a phenyl group, the term "Me" may refer to a methyl group, the term "Et" may refer to an ethyl group, the term "tert-Bu," "^{t}Bu," or "Bu^{t}" may refer to a tert-butyl group, and the term "OMe" may refer to a methoxy group.

The term "biphenyl group" as used herein refers to "a phenyl group substituted with a phenyl group." For example, the "biphenyl group" may be a substituted phenyl group having a C₆-C₆₀ aryl group as a substituent.

The term "terphenyl group" as used herein refers to "a phenyl group substituted with a biphenyl group." The "terphenyl group" may also refer to i) "a substituted phenyl group" which is "a C₆-C₆₀ aryl group in which a substituent is substituted with a C₆-C₆₀ aryl group", or ii) "a substituted phenyl group" having two substituents, each of which is "a C₆-C₆₀ aryl group."

* and *' as used herein, unless defined otherwise, each refer to a bonding site to a neighboring atom in a corresponding formula or moiety.

The terms "x-axis," "y-axis," and "z-axis" as used herein are not limited to three axes in an orthogonal coordinate system, and may be interpreted in a broader sense than the aforementioned three axes in an orthogonal coordinate system. For example, the x-axis, y-axis, and z-axis may describe axes that are orthogonal to each other, or may describe axes that are in different directions that are not orthogonal to each other.

Hereinafter, organic compounds according to one or more embodiments and light-emitting devices comprising the organic compounds will be described in more detail with reference to the following synthesis examples and examples.

### Synthesis Example 1 (Synthesis of Compound 2)

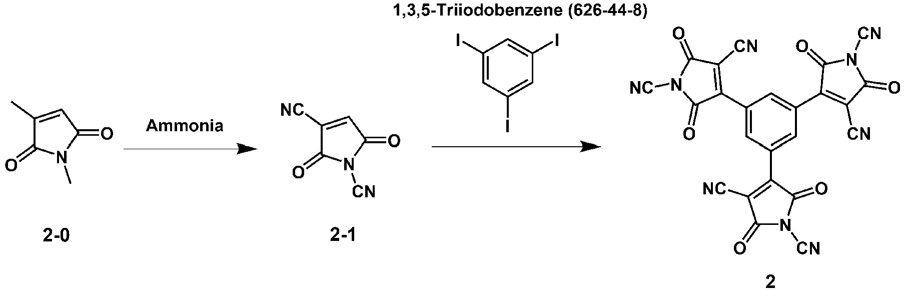

### Synthesis of Intermediate 2-1

1,3-dimethyl-1H-pyrrole-2,5-dione (Compound 2-0, 10 g, 0.08 mol), ammonia (4.08 g, 0.24 mol), and air at a molar ratio of 1:3:6 were allowed for a reaction at a temperature in a range of 375 °C to 450 °C for 2 hours. Afterwards, the resulting crude product was collected at 0 °C and separated by column chromatography, to thereby obtain 10.3 g of Intermediate 2-1 (yield: 88 %).

### Synthesis of Compound 2

Di-µ-chlorobis[(1,2,5,6-η)-1,5-cyclooctadiene]diiridium (0.41 g, 0.0006 mol) and bis(pinacolato)diborane (7.78 g, 0.031 mol) as catalysts were dissolved in a pure alkene solvent (10 mL) and allowed for a reaction with Intermediate 2-1 (10 g, 0.068 mol) and 1,3,5-triiodobenzene (10.33 g, 0.023 mol) at 60 °C for 16 hours. Afterwards, the reaction product was cooled to room temperature and diluted with a mixed solution (25 mL) containing tetrahydrofuran (THF) and water at a ratio of 4:1. Barium hydroxide (10.49 g, 0.061 mol), Pd(OAc)₂ (0.34 g, 0.0015 mol), PPh₃ (0.8 g, 0.0031 mol), and Cs₂CO₃ (19.95 g, 0.06 mol) were added to the mixture, followed by stirring at 60 °C for 18 hours. Afterwards, the resulting crude reaction mixture was evaporated, and the residue was purified by silica gel column chromatography, to thereby obtain 9.3 g of Compound 2 (yield: 79 %).

### Synthesis Example 2 (Synthesis of Compound 3)

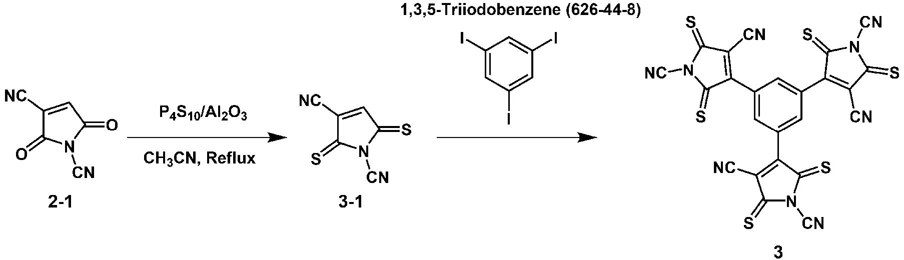

### Synthesis of Intermediate 3-1

Intermediate 2-1 (10 g, 0.068 mol), P₄S₁₀ (20.56 g, 0.046 mol), and Al₂O₃ (4.72 g, 0.046 mol) were added to acetonitrile (CH₃CN) and allowed for a reaction at room temperature for 2 hours, to thereby obtain 5.1 g of Intermediate 3-1 (yield: 65 %).

### Synthesis of Compound 3

11.9 g of Compound 3 was obtained in substantially the same manner as in the synthesis of Compound 2, except that Intermediate 3-1 was used instead of Intermediate 2-1 (yield: 80 %).

### Synthesis Example 3 (Synthesis of Compound 9)

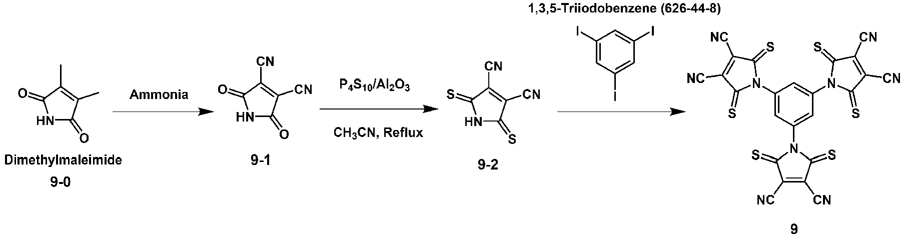

### Synthesis of Intermediate 9-1

Intermediate 9-1 was obtained in substantially the same manner as in the synthesis of Intermediate 2-1, except that Compound 9-0 (dimethylmaleimide) was used instead of Compound 2-0.

### Synthesis of Intermediate 9-2

Intermediate 9-2 was obtained in substantially the same manner as in the synthesis of Intermediate 3-1, except that Intermediate 9-1 was used instead of Intermediate 2-1.

### Synthesis of Compound 9

9.3 g of Compound 9 was obtained in substantially the same manner as in the synthesis of Compound 2, except that Intermediate 9-2 was used instead of Intermediate 2-1 (yield: 82 %).

### Synthesis Example 4 (Synthesis of Compound 23)

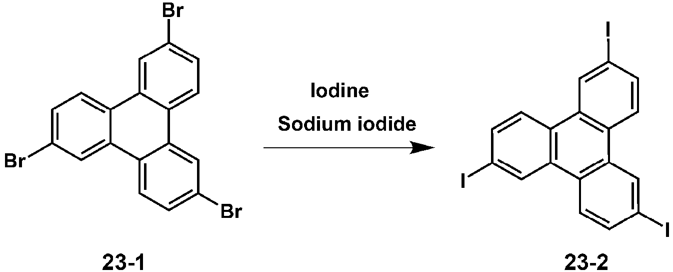

### Synthesis of Intermediate 23-2

Compound 23-1 (2,6,10-tribromotriphenylene, CAS No.: 1384858-36-9) (10 g, 0.022 mol), sodium iodide (57.36 g, 0.129 mol), and iodine (1.64 g, 0.006 mol) were added to 300 mL of acetonitrile. A freeze-pump-thaw cycle was repeated four times in a sealed environment, and ultra-pure argon was filled thereinto, followed by stirring at 20 °C under UV irradiation for 72 hours at an intensity of 4.0 mWcm⁻², to thereby obtain 12.1 g of Intermediate 23-2 (yield: 93 %).

### Synthesis of Compound 23

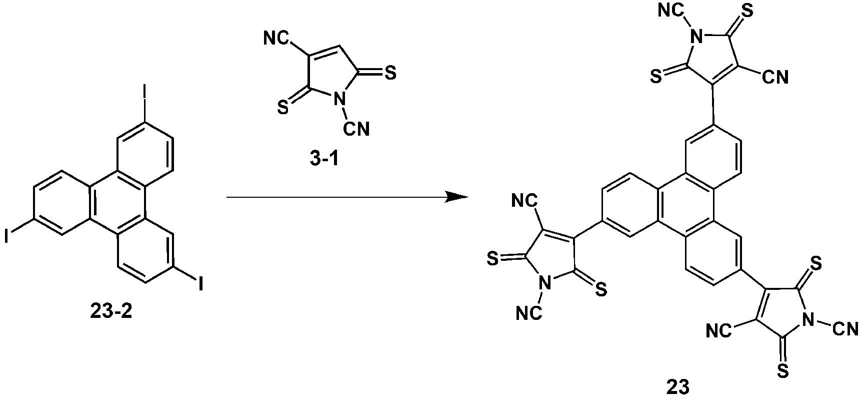

Intermediate 3-1 (10 g, 0.087 mol) and Intermediate 23-2 (17.55 g, 0.029 mol), together with di-µ-chlorobis[(1,2,5,6-η)-1,5-cyclooctadiene]diiridium (0.52 g, 0.0008 mol) and bis(pinacolato)diborane (9.94 g, 0.039 mol) as catalysts, were dissolved in a pure alkene solvent (15 mL) and allowed for a reaction at 60 °C for 16 hours. Afterwards, the reaction product was cooled to room temperature and diluted with a mixed solution (25 mL) containing THF and water at a ratio of 4:1. Barium hydroxide (13.40 g, 0.078 mol), Pd(OAc)₂ (0.44 g, 0.00195 mol), PPh₃ (1.02 g, 0.0039 mol), and Cs₂CO₃ (25.48 g, 0.078 mol) were added to the mixture, followed by stirring at 60 °C for 18 hours. Afterwards, the resulting crude reaction mixture was evaporated, and the residue was purified by silica gel column chromatography, to thereby obtain 17.8 g of Compound 23 (yield: 81 %).

### Synthesis Example 5 (Synthesis of Compound 24)

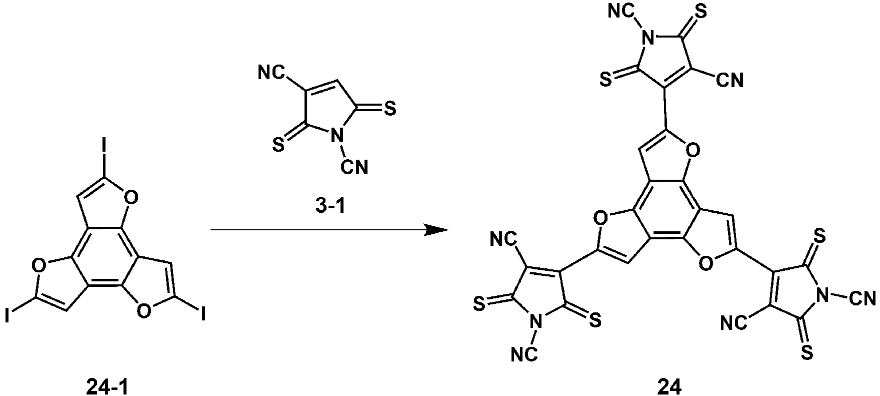

16.9 g of Compound 24 was obtained in substantially the same manner as in the synthesis of Compound 23, except that Compound 24-1 (2,5,8-triiodobenzo[1,2-b:3,4-b':5,6-b"]trifuran (CAS No.: 2187370-87-0)) was used instead of Intermediate 23-2 (yield: 80 %).

Proton nuclear magnetic resonance spectroscopy (¹H NMR), carbon-13 nuclear magnetic resonance spectroscopy (¹³C NMR), and liquid chromatography-mass spectroscopy (LC-MS) of the compounds synthesized according to Synthesis Examples 1 to 5 are shown in Table 1. Synthesis methods for other compounds than the compounds shown in Table 1 may be easily recognized by those skilled in the technical field by referring to the synthesis paths and source material materials described above.

**Table 1**

| Compound | ¹H NMR (CDCl₃, 500 MHz) | LC-MS | |
|---|---|---|---|
| | ¹³C-NMR (125 MHz, CDCl₃) | Found [M+1] | Calcd. |
| 2 | ¹H NMR (CDCl₃, 500 MHz): 6.55 (s, 3H) | 512.58 | 513.02 |
| | ¹³C-NMR (125 MHz, CDCl3): 172.0, 166.3, 139.2, 108.5, 132.3, 122.0, 117.8, 115.8 | | |
| 3 | ¹H NMR (CDCl₃, 500 MHz): 6.55 (s, 3H) | 608.12 | 608.88 |
| | ¹³C-NMR (125 MHz, CDCl₃): 195.8, 194.9, 173.0, 118.2, 132.3, 122.0, 117.8, 115.8, | | |
| 9 | ¹H NMR (CDCl₃, 500 MHz): 7.41 (s, 3H) | 608.55 | 608.88 |
| | ¹³C-NMR (125 MHz, CDCl₃): 189.1, 188.2, 143.4, 134.5, 119.1, 115.8 | | |
| 23 | ¹H NMR (CDCl₃, 500 MHz): 7.88 (m, 3H), 8.33 (m, 3H), 8.70 (m, 3H) | 758.52 | 758.93 |
| | ¹³C-NMR (125 MHz, CDCl₃): 195.8, 194.9, 173.0, 118.2, 132.7, 128.9, 128.5, 120.5, 125.6, 132.4, 117.8, 115.8 | | |
| 24 | ¹H NMR (CDCl₃, 500 MHz): 7.14 (s, 3H) | 728.22 | 728.87 |
| | ¹³C-NMR (125 MHz, CDCl₃): 195.8, 194.9, 130.7, 159.6, 121.9, 173.0, 124.2, 106.8, 117.8, 115.8 | | |

### Evaluation Example 1

The HOMO energy level, LUMO energy level, hole mobility, electron mobility, and glass transition temperature of each of the compounds synthesized in Synthesis Examples 1 to 5 were measured by the methods shown in Table 2, and the results are shown in Table 3.

**Table 2**

| | |
|---|---|
| HOMO energy level evaluation method | A potential (V)-current (A) graph of each compound was obtained by using cyclic voltammetry (CV) (electrolyte: 0.1 M Bu₄NPF₆/ solvent: dimethyl formamide (DMF)/ electrode: 3 electrode system (working electrode: glass carbon (GC), reference electrode: Ag/AgCl, auxiliary electrode: Pt)), and then, from oxidation onset of the graph, the HOMO energy level of the compound was calculated. |
| LUMO energy level evaluation method | A potential (V)-current (A) graph of each compound was obtained by using cyclic voltammetry (CV) (electrolyte: 0.1 M Bu₄NPF₆/ solvent: dimethyl formamide (DMF)/ electrode: 3 electrode system (working electrode: GC, reference electrode: Ag/AgCl, auxiliary electrode: Pt)), and then, from reduction onset of the graph, from LUMO energy level of the compound was calculated. |
| Hole mobility and electron mobility evaluation method | The hole mobility and electron mobility of each compound were evaluated by using the space-charge-limited current (SCLC) method described in the document "Hole mobility of N,N'-bis(naphtanlen-1-yl)-N,N'-bis(phenyl)benzidine investigated by using space-charge-limited currents,' Appl. Phys. Lett. 90, 203512 (2007)," the entire content of which is incorporated herein by reference. |
| Glass transition temperature evaluation method | Each compound was analyzed by using differential scanning calorimetry (DSC) under the following analysis conditions: a sample having a weight of 5 mg was heated from room temperature to 300 °C at a scan rate of 10 °C/min, cooled from 300 °C to 25 °C at a scan rate of 10 °C/min, and then heated again to 300 °C at a scan rate of 10 °C/min. In this regard, the glass transition temperature of the compound was measured during the second heating and was obtained from an inflection point on a graph obtained by the analysis. |

**Table 3**

| Compound No. | HOMO (eV) | LUMO (eV) | Hole mobility (cm²/(Vs)) | Electron mobility (cm²/(Vs)) | Glass transition temperature (°C) |
|---|---|---|---|---|---|
| 2 | -8.85 | -5.17 | 9.08×10⁻⁵ | 2.52×10⁻⁴ | 123.0 |
| 3 | -7.71 | -5.32 | 2.01×10⁻⁴ | 1.60×10⁻³ | 117.9 |
| 9 | -7.74 | -5.37 | 2.16×10⁻⁵ | 1.74×10⁻⁴ | 121.3 |
| 23 | -7.40 | -5.08 | 2.37×10⁻³ | 2.25×10⁻⁴ | 133.6 |
| 24 | -7.49 | -5.22 | 1.06×10⁻⁴ | 1.30×10⁻⁴ | 141.2 |

### Example 1

As an anode, a glass substrate (product of Corning Inc.) with a 15 Ω/cm² (1,300 Å) ITO formed thereon was cut to a size of 50 mm x 50 mm x 0.7 mm, sonicated with isopropyl alcohol and then with pure water each for 5 minutes, cleaned by irradiation of ultraviolet rays and exposure of ozone thereto for 30 minutes, and then mounted on a vacuum deposition apparatus.

Compound 2 and Compound HT3 were vacuum-deposited at a weight ratio of 3:97 on the anode to form a hole injection layer having a thickness of 100 Å. Compound HT40 was vacuum-deposited on the hole injection layer to form a hole transport layer having a thickness of 1,250 Å.

Compound H129, Compound H130, and Compound D1 were vacuum-deposited at a weight ratio of 45:45:10 on the hole transport layer to form an emission layer having a thickness of 300 Å.

Compound ET37 was vacuum-deposited on the emission layer to form a buffer layer having a thickness of 50 Å. Compound ET46 and LiQ were vacuum-deposited at a weight ratio of 5:5 on the buffer layer to form an electron transport layer having a thickness of 310 Å. Yb was vacuum-deposited on the electron transport layer to form an electron injection layer having a thickness of 15 Å, and Ag and Mg were vacuum-deposited at a weight ratio of 5:5 on the electron injection layer to form a cathode having a thickness of 1,000 Å, thereby completing the manufacture of a light-emitting device.

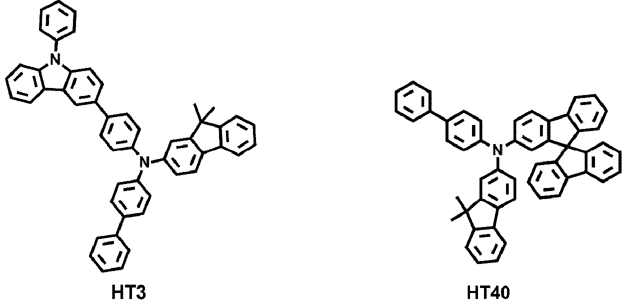

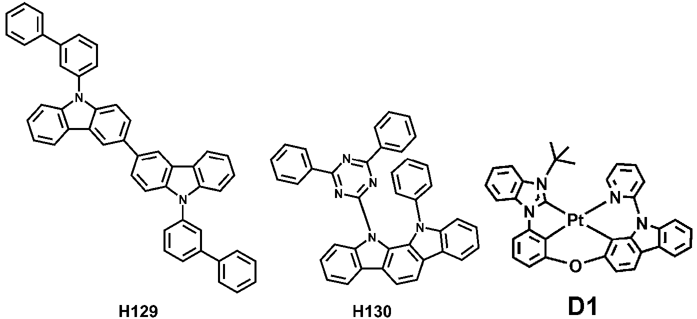

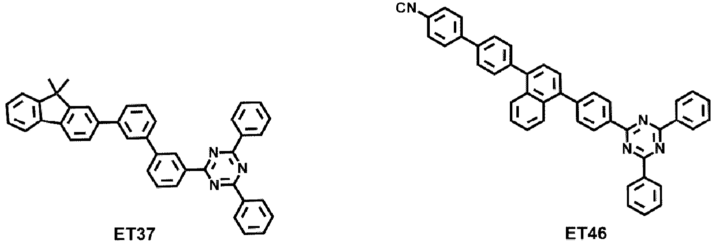

### Examples 2 to 5 and Comparative Examples 1 to 4

Light-emitting devices were each manufactured in substantially the same manner as in Example 1, except that the compounds shown in Table 4 were each respectively used instead of Compound 2 in forming a hole injection layer.

### Evaluation Example 2

To evaluate the characteristics of each of the light-emitting devices manufactured according to Examples 1 to 5 and Comparative Examples 1 to 4, the driving voltage, current efficiency, and lifespan thereof at a current density of 10 mA/cm² were measured, and the results are shown in Table 4. The driving voltage was measured in the unit of V by using a source meter (Keithley Instrument Inc., 2400 series). The current efficiency was measured in the unit of cd/A by using a luminance meter CS-2000 (Konica Minolta Inc.). The lifespan was measured as the time (hr) taken for the luminance to reach 95 % of the initial luminance. The driving voltage, current efficiency, and lifespan are expressed as relative values with respect to Comparative Example 1.

**Table 4**

| No. | Hole injection layer | Driving voltage | Current efficiency | Lifespan |
|---|---|---|---|---|
| Example 1 | Compound 2: Compound HT3 | 61.4 % | 100.7 % | 120.5 % |
| Example 2 | Compound 3 : Compound HT3 | 59.9 % | 101.8 % | 121.1 % |
| Example 3 | Compound 9 : Compound HT3 | 58.6 % | 102.0 % | 122.4 % |
| Example 4 | Compound 23 : Compound HT3 | 59.2 % | 102.0 % | 122.2 % |
| Example 5 | Compound 24 : Compound HT3 | 60.5 % | 101.2 % | 120.9 % |
| Comparative Example 1 | HAT-CN : Compound HT3 | 100.0 % | 100.0 % | 100.0 % |
| Comparative Example 2 | Compound CE1 : Compound HT3 | 102.5 % | 90.1 % | 92.7 % |
| Comparative Example 3 | Compound CE2 : Compound HT3 | 82.6 % | 102.1 % | 105.8 % |
| Comparative Example 4 | Compound CE3 : Compound HT3 | 101.5 % | 95.4 % | 96.7 % |

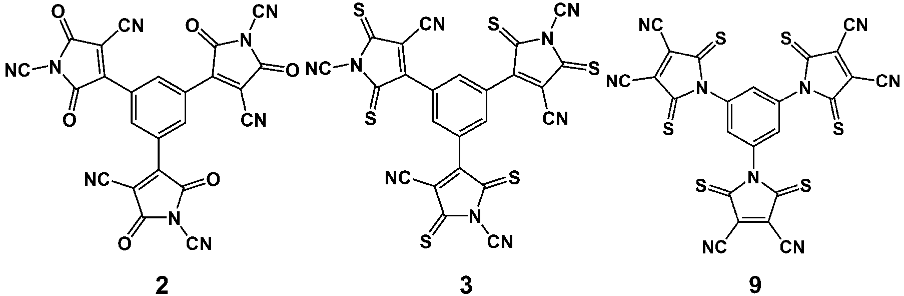

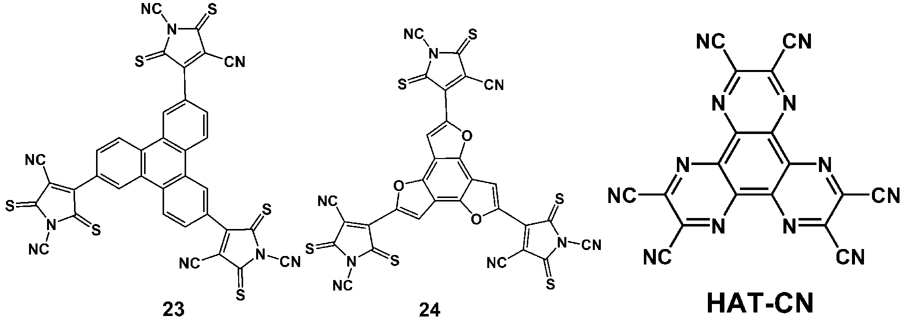

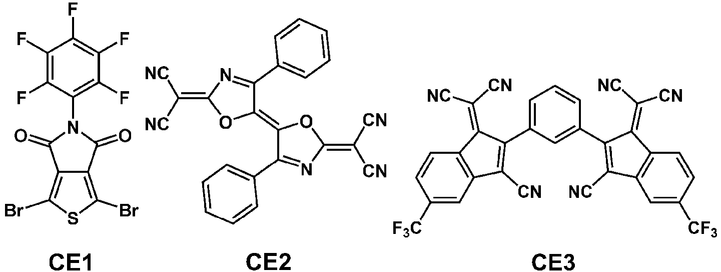

Referring to Table 4, it was confirmed that the light-emitting devices according to Examples 1 to 5 each had a lower driving voltage, higher current efficiency, and/or a longer lifespan than the light-emitting devices according to Comparative Examples 1 to 4.

According to the one or more embodiments, an organic compound may comprise a first moiety that is represented by Formula 1 and is not a condensed ring, and a second moiety that is represented by Formula 2 and is not a condensed ring, wherein each carbon atom adjacent to a nitrogen atom in a nitrogen-containing 5-membered ring comprised in each of the first moiety and the second moiety may have a double bond. Thus, the organic compound may have a HOMO energy level and a LUMO energy level that are suitable for use with a hole transport material, may have high hole mobility, and may concurrently (e.g., simultaneously) have a high glass transition temperature. For example, the organic compound may have excellent or suitable hole-transporting characteristics and thermal stability and morphological stability.

For example, the synthesis examples provided in the present disclosure illustrate the step-by-step (e.g., act-by-act or task-by-task) procedures for creating various organic compounds, which are integral to the development of high-performance light-emitting devices. Each synthesis example outlines the specific reactants, conditions, and methods used to obtain the desired intermediates and final compounds. These detailed procedures ensure reproducibility and provide a clear understanding of the chemical processes involved. These synthesis examples are for demonstrating the practical feasibility of producing the organic compounds described in the disclosure. They provide a foundation for further evaluation of the compounds' properties, such as HOMO and LUMO energy levels, hole and electron mobility, and glass transition temperature, which are, e.g., critical for their application in light-emitting devices. By comprising specific examples and detailed procedures, the disclosure ensures that the inventive concept is well-supported and clearly communicated.

In the present disclosure, it will be understood that the term "comprise(s)/comprising," "include(s)/including," or "have/has/having" specifies the presence of stated features, integers, steps, operations, elements, and/or components, but do not preclude the presence or addition of one or more other features, integers, steps, operations, elements, components, and/or groups thereof. Additionally, the terms "comprise(s)/comprising," "include(s)/including," "have/has/having", or other similar terms include or support the terms "consisting of" and "consisting essentially of," indicating the presence of stated features, integers, steps, operations, elements, and/or components, without or essentially without the presence of other features, integers, steps, operations, elements, components, and/or groups thereof.

In the context of the present application and unless otherwise defined, the terms "use," "using," and "used" may be considered synonymous with the terms "utilize," "utilizing," and "utilized," respectively.

Throughout the present disclosure, when a component such as a layer, a film, a region, or a plate is mentioned to be placed "on" another component, it will be understood that it may be directly on another component or that another component may be interposed therebetween. In some embodiments, "directly on" may refer to that there are no additional layers, films, regions, plates, etc., between a layer, a film, a region, a plate, etc. and the other part. For example, "directly on" may refer to two layers or two members are disposed without utilizing an additional member such as an adhesive member therebetween.

In the present disclosure, although the terms "first," "second," etc., may be utilized herein to describe one or more elements, components, regions, and/or layers, these elements, components, regions, and/or layers should not be limited by these terms. These terms are only utilized to distinguish one component from another component.

As utilized herein, the singular forms "a," "an," "one," and "the" are intended to include the plural forms as well, unless the context clearly indicates otherwise. Further, the use of "may" when describing embodiments of the present disclosure refers to "one or more embodiments of the present disclosure".

As utilized herein, the terms "substantially," "about," or similar terms are used as terms of approximation and not as terms of degree, and are intended to account for the inherent deviations in measured or calculated values that would be recognized by those of ordinary skill in the art. "About" as used herein, is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, or 5% of the stated value.

Any numerical range recited herein is intended to comprise all sub-ranges of the same numerical precision subsumed within the recited range. For example, a range of "1.0 to 10.0" is intended to comprise all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to comprise all lower numerical limitations subsumed therein and any minimum numerical limitation recited in the present disclosure is intended to comprise all higher numerical limitations subsumed therein. Accordingly, Applicant reserves the right to amend the disclosure, including the claims, to expressly recite any sub-range subsumed within the ranges expressly recited herein.

The light-emitting device, the light-emitting apparatus, the display device, the electronic apparatus, the electronic device/equipment, the manufacturing apparatus thereof, or any other relevant devices or components according to embodiments of the present disclosure described herein may be implemented utilizing any suitable hardware, firmware (e.g., an application-specific integrated circuit), software, or a combination of software, firmware, and hardware. For example, the various components of the device may be formed on one integrated circuit (IC) chip or on separate IC chips. Further, the various components of the device may be implemented on a flexible printed circuit film, a tape carrier package (TCP), a printed circuit board (PCB), or formed on one substrate. Further, the various components of the device may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random access memory (RAM). The computer program instructions may also be stored in other non-transitory computer readable media such as, for example, a CD-ROM, flash drive, or the like. Also, a person of skill in the art should recognize that the functionality of various computing devices may be combined or integrated into a single computing device, or the functionality of a particular computing device may be distributed across one or more other computing devices without departing from the scope of the embodiments of the present disclosure.

It should be understood that embodiments described herein should be considered in a descriptive sense only and not for purposes of limitation. Descriptions of features or aspects within each embodiment should typically be considered as available for other similar features or aspects in one or more embodiments. While one or more embodiments have been described with reference to the drawings, it will be understood by those of ordinary skill in the art that one or more suitable changes in form and details may be made therein without departing from the scope as defined by the appended claims and equivalents thereof.

## Claims

1. A light-emitting device comprising:
a first electrode;
a second electrode opposite to the first electrode; and
an interlayer between the first electrode and the second electrode and comprising an emission layer,
wherein the interlayer comprises an organic compound comprising a first moiety represented by Formula 1 and a second moiety represented by Formula 2: 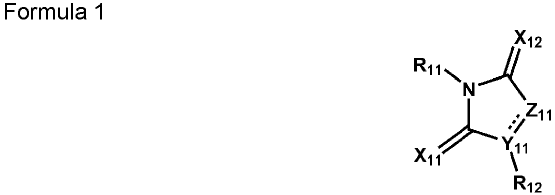 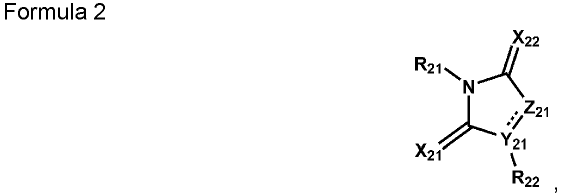
wherein, in Formulae 1 and 2,
a moiety represented by 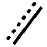 is a single bond or a double bond,
X₁₁, X₁₂, X₂₁, and X₂₂ are each independently O or S,
Y₁₁ is C, C(R₁₃), or N,
Z₁₁ is C(R₁₄), N, N(R₁₄), O, or S,
Y₂₁ is C, C(R₂₃), or N,
Z₂₁ is C(R₂₄), N, N(R₂₄), O, or S,
R₁₁ to R₁₄ and R₂₁ to R₂₄ are each independently hydrogen, deuterium, -F, -Cl, -Br, -I, a hydroxyl group, a cyano group, a nitro group, a C₁-C₆₀ alkyl group unsubstituted or substituted with at least one R₁₀, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂),
R₁₁ or R₁₂ is a linking site to the second moiety,
R₂₁ or R₂₂ is a linking site to the first moiety, and
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, - Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
wherein Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, or a nitro group; or
a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

2. The light-emitting device of claim 1, wherein the interlayer comprises a hole transport region between the first electrode and the emission layer, and
the hole transport region comprises the organic compound.

3. The light-emitting device of claim 2, wherein the hole transport region comprises a hole injection layer and a hole transport layer between the hole injection layer and the emission layer, and
the hole injection layer comprises the organic compound.

4. The light-emitting device of claim 3, wherein the hole injection layer is in contact with the first electrode.

5. The light-emitting device of any one of claims 1 to 4, wherein the emission layer comprises a phosphorescent dopant containing a transition metal; AND/OR
wherein the emission layer is to emit blue light.

6. An electronic apparatus comprising:
a light-emitting device; and
a thin-film transistor electrically connected to the light-emitting device,
wherein the light-emitting device comprises:
a first electrode;
a second electrode opposite to the first electrode; and
an interlayer between the first electrode and the second electrode and comprising an emission layer,
wherein the interlayer comprises an organic compound comprising a first moiety represented by Formula 1 and a second moiety represented by Formula 2: 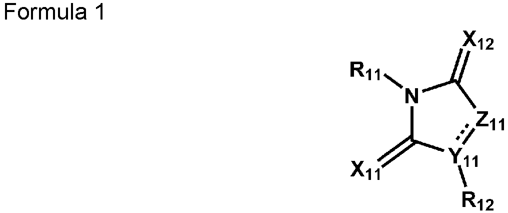 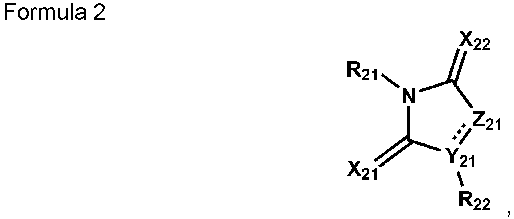
wherein, in Formulae 1 and 2,
a moiety represented by 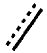 is a single bond or a double bond,
X₁₁, X₁₂, X₂₁, and X₂₂ are each independently O or S,
Y₁₁ is C, C(R₁₃), or N,
Z₁₁ is C(R₁₄), N, N(R₁₄), O, or S,
Y₂₁ is C, C(R₂₃), or N,
Z₂₁ is C(R₂₄), N, N(R₂₄), O, or S,
R₁₁ to R₁₄ and R₂₁ to R₂₄ are each independently hydrogen, deuterium, -F, -Cl, -Br, - I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂),
R₁₁ or R₁₂ is a linking site to the second moiety,
R₂₁ or R₂₂ is a linking site to the first moiety, and
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), -C(=O)(Q₁₁), -S(=O)2(Q11), - P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, - Si(Q₂₁)(Q₂₂)(Q₂₃), -N(Q₂₁)(Q₂₂), -B(Q₂₁)(Q₂₂), -C(=O)(Q₂₁)**,** -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
wherein Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, or a nitro group; or
a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

7. An electronic equipment comprising the electronic apparatus of claim 6, wherein the electronic equipment is at least one of a flat panel display, a curved display, a computer monitor, a medical monitor, a television, a billboard, an indoor light, an outdoor light, a signal light, a head-up display, a fully transparent display, a partially transparent display, a flexible display, a rollable display, a foldable display, a stretchable display, a laser printer, a telephone, a mobile phone, a tablet, a phablet, a personal digital assistant, a wearable device, a laptop computer, a digital camera, a camcorder, a viewfinder, a micro display, a 3D display, a virtual reality display, an augmented reality display, a vehicle, a video wall comprising multiple displays tiled together, a theater screen, a stadium screen, a phototherapy device, or a signboard.

8. An organic compound comprising:
a first moiety represented by Formula 1; and
a second moiety represented by Formula 2: 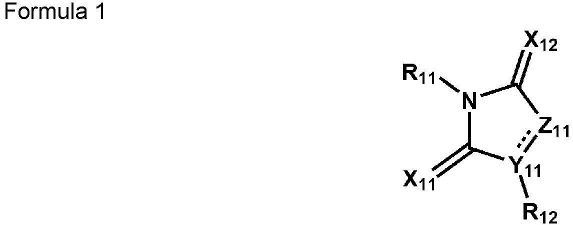 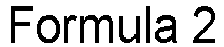 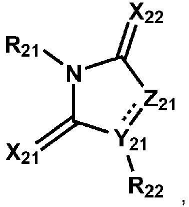
wherein, in Formulae 1 and 2,
a moiety represented by 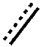 is a single bond or a double bond,
X₁₁, X₁₂, X₂₁, and X₂₂ are each independently O or S,
Y₁₁ is C, C(R₁₃), or N,
Z₁₁ is C(R₁₄), N, N(R₁₄), O, or S,
Y₂₁ is C, C(R₂₃), or N,
Z₂₁ is C(R₂₄), N, N(R₂₄), O, or S,
R₁₁ to R₁₄ and R₂₁ to R₂₄ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂),
R₁₁ or R₁₂ is a linking site to the second moiety,
R₂₁ or R₂₂ is a linking site to the first moiety, and
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, - Si(Q₂₁)(Q₂₂)(Q₂₃), -N(Q₂₁)(Q₂₂), -B(Q₂₁)(Q₂₂), -C(=O)(Q₂₁)**,** -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
wherein Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, or a nitro group; or
a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

9. The organic compound of claim 8, wherein each of the first moiety and the second moiety does not comprise a condensed ring; AND/OR
wherein the first moiety and the second moiety are identical to each other.

10. The organic compound of claim 8 or claim 9, wherein at least one of the first moiety or the second moiety comprises at least one of deuterium, -F, or a cyano group;
AND/OR
wherein the first moiety and the second moiety are each independently represented by any one selected from among Formulae M1 to M63: 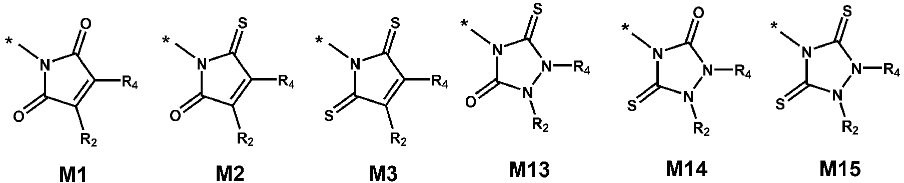 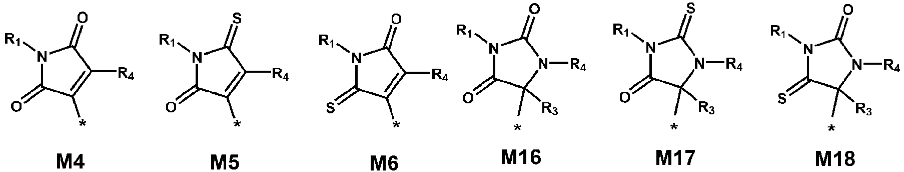 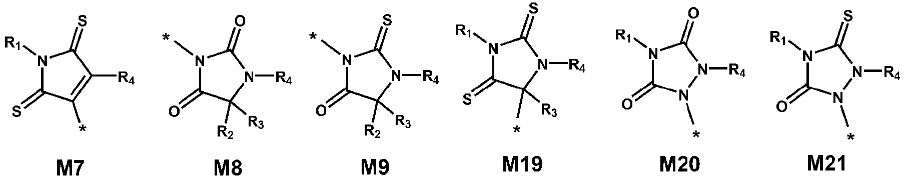 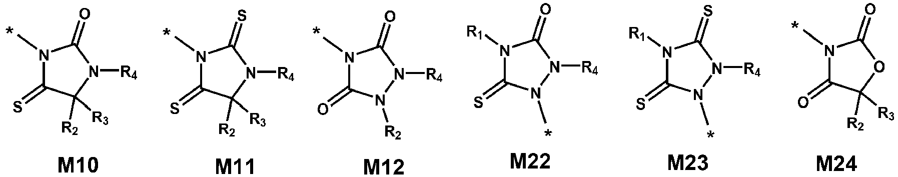 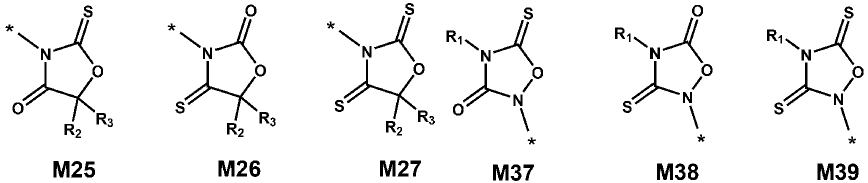 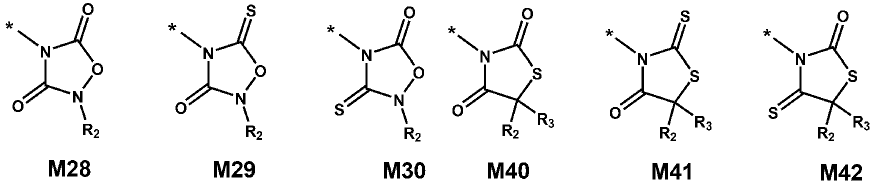 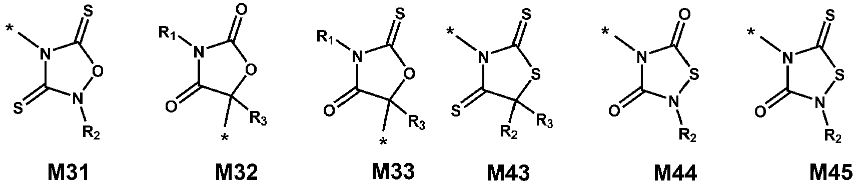 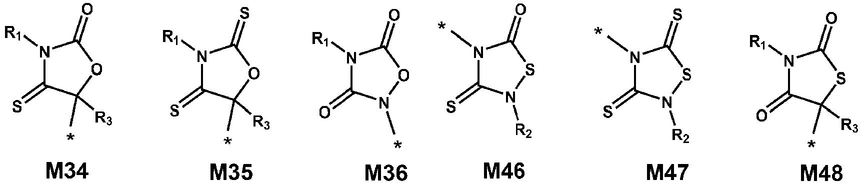 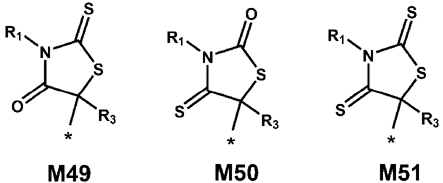 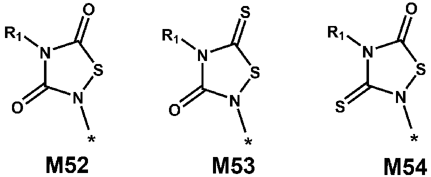 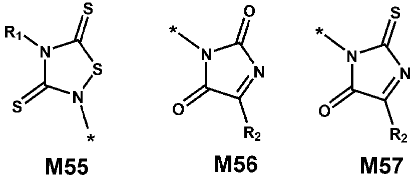 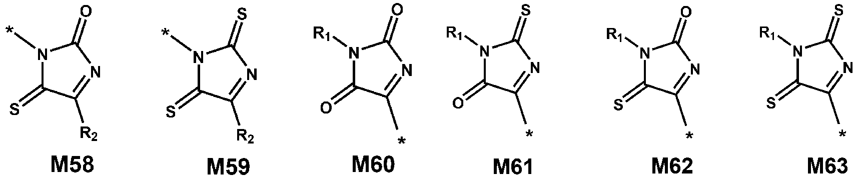
in Formulae M1 to M63,
R₁ being the same as defined with respect to R₁₁ and R₂₁,
R₂ being the same as defined with respect to R₁₂ and R₂₂,
R₃ being the same as defined with respect to R₁₃ and R₂₃,
R₄ being the same as defined with respect to R₁₄ and R₂₄, and
* indicating a bonding site to a neighboring atom.

11. The organic compound of any one of claims 8 to 10, further comprising a third moiety for linking the first moiety and the second moiety to each other.

12. The organic compound of claim 11, wherein the third moiety is *-(L₁)ₙ₁-*', *-(L₁)ₙ₂=(L₂)ₙ₃-*', or *-C(R₁)=(L₁)ₙ₄=C(R₂)-*',
L₁ and L₂ are each independently a C₃-C₆₀ carbocyclic group unsubstituted or substituted with at least one R_{10b} or a C₁-C₆₀ heterocyclic group unsubstituted or substituted with at least one R_{10b},
n1 to n4 are each independently an integer from 0 to 5,
* indicates a bonding site to the first moiety,
*' indicates a bonding site to the second moiety,
R₁ and R₂ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), -S(=O)₂(Q₁), or - P(=O)(Q₁)(Q₂),
R₁₀ₐ and Q₁ to Q₃ are each the same as defined in Formula 1 and Formula 2, and
R_{10b} is the same as defined with respect to R₁₀ₐ.

13. The organic compound of claim 12, wherein L₁ and L₂ are each independently a C₆-C₆₀ arylene group unsubstituted or substituted with at least one R_{10b} or a C₁-C₆₀ heteroarylene group unsubstituted or substituted with at least one R_{10b}; OR
wherein L₁ and L₂ are each independently a cyclopentene group, a cyclopentadiene group, a cyclohexene group, a cyclohexadiene group, a cyclooctatetraene group, a benzene group, a naphthalene group, an anthracene group, a phenanthrene group, a perylene group, a phenalene group, a pyrene group, a tetracene group, a triphenylene group, a pyridine group, a pyrimidine group, a triazine group, a pyrrole group, an imidazole group, a thiazole group, an oxazole group, a furan group, a thiophene group, a carbazole group, a dibenzofuran group, a dibenzothiophene group, a pyridoquinolizine group, a dihydropyridine group, a dihydropyrazine group, or a condensed cyclic group fused by any combination thereof.

14. The organic compound of claim 12 or 13, wherein R_{10b} comprises a group represented by Formula 3: 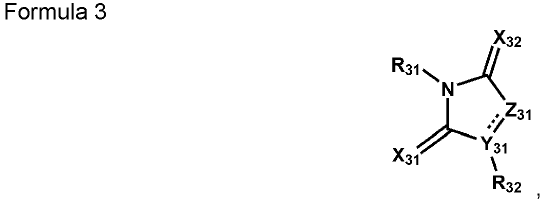 and
wherein, in Formula 3,
a moiety represented by 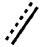 is a single bond or a double bond,
X₃₁ and X₃₂ are each independently O or S,
Y₃₁ is C, C(R₃₃), or N,
Z₃₁ is C(R₃₄), N, N(R₃₄), O, or S,
R₃₁ to R₃₄ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), -S(=O)₂(Q₁), or - P(=O)(Q₁)(Q₂),
R₃₁ or R₃₂ is a bonding site to a neighboring atom, and
R₁₀ₐ and Q₁ to Q₃ are each the same as defined in Formula 1 and Formula 2;
AND/OR
wherein n1 is 0, 1, 2, or 3,
n2 is 1 or 2,
n3 is 1 or 2, and
n4 is 1 or 2.

15. The organic compound of any one of claims 8 to 14, wherein the organic compound is any one selected from among Compounds 1 to 39:
